(19) Europäisches Patentamt
European Patent Office
Office européen des brevets

(11) EP 2 102 222 B1

(12) **FASCICULE DE BREVET EUROPEEN**

(45) Date de publication et mention de la délivrance du brevet:
**19.01.2011 Bulletin 2011/03**

(21) Numéro de dépôt: **07872005.9**

(22) Date de dépôt: **20.12.2007**

(51) Int Cl.:
*C07H 3/06* (2006.01) *C07H 3/08* (2006.01)
*A61K 31/702* (2006.01) *C08B 37/18* (2006.01)

(86) Numéro de dépôt international:
**PCT/FR2007/052595**

(87) Numéro de publication internationale:
**WO 2008/087340 (24.07.2008 Gazette 2008/30)**

(54) **Oligo-beta-(1,3)-glucane-(1,3)-mannose, oligo-beta-(1.3)-glucane-(1,3)-mannitol et leurs derives pour le traitement de maladies du systeme immunitaire, leurs procedes de preparation et medicaments les contenant**

Oligo-beta-(1,3)-glucan-(1,3)-mannose, oligo-beta-(1,3)-glucan-(1,3)- mannitol und seine Derivate zur Behandlung von Erkrankungen des Immunsystems, Herstellungsverfahren dafür und Wirkstoffe damit

Oligo-beta-(1,3)-glucane-(1,3)-mannose, oligo-beta-(1,3)-glucane-(1,3)-mannitol and derivatives thereof for treating diseases of the immune system, methods for preparing the same and drugs containing them

(84) Etats contractants désignés:
**AT BE BG CH CY CZ DE DK EE ES FI FR GB GR HU IE IS IT LI LT LU LV MC MT NL PL PT RO SE SI SK TR**

(30) Priorité: **22.12.2006 FR 0611333**

(43) Date de publication de la demande:
**23.09.2009 Bulletin 2009/39**

(73) Titulaire: **Laboratoires Goëmar**
**35400 Saint-Malo (FR)**

(72) Inventeurs:
- **YVIN, Jean-Claude**
  **F-35400 Saint Malo (FR)**
- **DESCROIX, Karine**
  **F-44310 Saint-colomban (FR)**
- **FERRIERES, Vincent**
  **F-35490 Gahard (FR)**
- **JAMOIS, Frank**
  **F-35520 La Chapelle Des Fouegeretz (FR)**
- **LAURENT, Isabelle**
  **F-35000 Rennes (FR)**
- **VETVICKA, Vaclav**
  **F-40220 Louisville Ky (US)**

(74) Mandataire: **Cabinet Plasseraud**
**52, rue de la Victoire**
**75440 Paris Cedex 09 (FR)**

(56) Documents cités:
**EP-A- 0 089 940** **WO-A-03/045414**
**WO-A-2005/049044** **JP-A- 59 036 691**

- **GOLDSTEIN, IRWIN J. ET AL: "Carbohydrate binding properties of banana (Musa acuminata) lectin. II. Binding of laminaribiose oligosaccharides and .beta.-glucans containing .beta.1,6-glucosyl end groups" EUROPEAN JOURNAL OF BIOCHEMISTRY , 268 (9), 2616-2619 CODEN: EJBCAI; ISSN: 0014-2956, 2001, XP002429236**

EP 2 102 222 B1

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

## Description

La présente invention décrit l'utilisation d'oligo-β-(1,3)-glucanes modifiés de formule (I) ou (II) ci-dessous pour la préparation de médicaments utiles dans les traitements basés sur la stimulation du système immunitaire.

Les glucanes, qui sont des produits naturels, ont été longuement étudiés et les propriétés immunostimulantes de certains glucanes sont connues. Rependant tous les glucanes naturels ne sont pas actifs. Des exemples de glucanes actifs sont décrits dans WO 2005/049 044 et dans JP 59 036 691.

La Société Demanderesse a montré, notamment dans WO03/045414 que la Laminarine, qui est un polysaccharide extrait de l'algue brune *Laminaria digitata* présentait des activités d'immunostimulation. La Laminarine est un polysaccharide de faible poids moléculaire constitué d'une chaîne principale linéaire β-(1,3)-glucane, de 20 à 30 unités glucose faiblement ramifié en position 6, qui présente à l'extrémité terminale ou bien une entité glucose (chaîne G) ou bien une entité mannitol (chaîne M).

Bien que l'activité immunostimulante de la Laminarine ait été reconnue, un inconvénient majeur de son utilisation à des fins thérapeutiques est son origine naturelle. En effet, la composition de la Laminarine n'est pas constante et dépend notamment de son lieu de ramassage, de l'époque de ramassage, etc.

Il existe donc un réel besoin en des produits de synthèse présentant les propriétés thérapeutiques de la Laminarine.

Les présents inventeurs se sont plus particulièrement tournés vers les oligosaccharides du groupe M présents dans la Laminarine. Dans la littérature, il est mentionné que ces oligosaccharides présentent une liaison de configuration β entre le carbone 1 de l'entité terminale du glucane et un carbone primaire du résidu mannitol, et peuvent être représentés de la façon suivante :

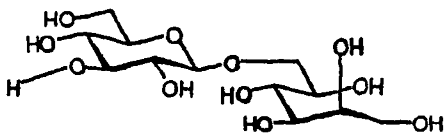

Ils ont cherché à synthétiser ces molécules de façon à mettre à la disposition notamment de l'industrie pharmaceutiques, des molécules dont la caractérisation est complète et la structure tout à fait élucider, évitant ainsi les difficultés inhérentes à l'utilisation de produits naturels en tant que produits pharmaceutiques.

De façon inattendue et surprenante, les présents inventeurs ont trouvé que les oligosaccharides de formule (I) ou (II) ci-dessous, qui eux présentent une liaison β entre le carbone 1 de l'entité terminale glucose et un carbone secondaire de l'entité terminale mannose ou mannitol, présentaient des activités immunostimulantes.

L'invention décrit donc l'utilisation d'au moins un oligo-β-(1,3)-glucane modifié de formule (I) ou (II)

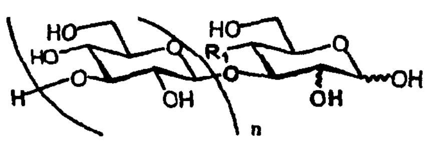

(I)

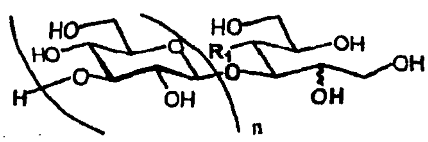

(II)

dans lesquelles $R_1$ représente H ou OH et n est un entier de 2 à 10, de préférence de 2 à 7, et plus préférentiellement encore égal à 3, 4 ou 5,

pour la préparation d'une composition destinée au traitement de maladies choisies dans le groupe consistant en tumeur, cancer, maladie virale, maladie bactérienne, maladie fongique, maladie du système immunitaire, maladie auto-immune ou maladie liée à une déficience de l'immunostimulation, chez des êtres humains et animaux à sang chaud. L'action des composés de formule (I) ou (II) est liée à la présence de récepteurs spécifiques de ces composés à la surface des

macrophages et plus généralement des différentes populations de leucocytes. Cette activité est attribuée à la stimulation de cellules immunocompétentes de l'organisme et est mesurée à travers :

- l'activation des cellules NK, des lymphocytes T, et du facteur nucléaire $\kappa$B(NF-$\kappa$B);
- l'activité phagocytaire ;
- la sécrétion de cytokines telles que les interleukines (IL), le TNF-$\alpha$, ou encore l'interféron $\gamma$ ;
- la production d'espèces oxydantes réactives comme l'anion superoxyde ou le peroxyde d'hydrogène.

Avantageusement, lorsque dans le composé de formule (II), $R_1$ représente OH, le groupe terminal est un mannitol.

Les mécanismes d'interaction de nombreux glucanes ont été largement décrits, mais ont montré des résultats souvent contradictoires, empêchant de prédire quel glucane est le meilleur immunomodulateur. Les effets liés à des modifications fonctionnelles ou structurelles sont quant à eux totalement imprévisibles.

La présente invention porte sur certains des dérivés de formule (I) et (II) ci-dessus qui sont des composés nouveaux. Ces nouveaux composés sont des oligo-β-(1,3) -glucane-(1,3)-mannose et oligo-β-(1,3) -glucane-(1,3)-mannitol et leurs dérivés désoxy en position 4, qui présentent respectivement la formule générale (I') ou la formule générale (II'):

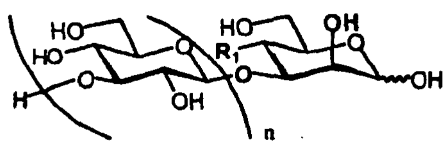

(I')

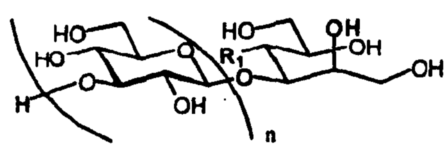

(II')

dans lesquelles $R_1$ représente H ou OH et n est un entier de 2 à 10, de préférence de 2 à 7, et plus préférentiellement encore égal à 3, 4 ou 5.
Plus particulièrement, de tels composés sont les composés suivants :

β-D-glucopyranosyl-(1→3)-β-D-glucopyranosyl-(1→3)-(α,β)-D-mannopyranose ;
β-D-glucopyranosyl-(1→3)-β-D-glucopyranosyl-(1→3)-β-D-glucopyranosyl-(1→3)-β-D-glucopyranosyl-(1→3)-β-D-glucopyranosyl-(1→3)-β-D-mannopyranose ;
β-D-glucopyranosyl-(1→3)-β-D-glucopyranosyl-(1→3)-4-désoxy-(α,β)-D-désoxy-mannopyranose ;
β-D-glucopyranosyl-(1→3)-β-D-glucopyranosyl-(1→3)-mannitol ;
β-D-glucopyranosyl-(1→3)-β-D-glucopyranosyl-(1→3)-4-désoxy-mannitol ;
β-D-glucopyranosyl-(1→3)-β-D-glucopyranosyl-(1→3)-β-D-glucopyranosyl-(1→3)-β-D-glucopyranosyl-(1→3)-β-D-glucopyranosyl-(1→3)-mannitol ;
β-D-glucopyranosyl-(1→3)-β-D-glucopyranosyl-(1→3)-β-D-glucopyranosyl-(1→3)-β-D-glucopyranosyl-(1→3)-β-D-glucopyranosyl-(1→3)-4-désoxy-(α,β)-D-mannopyranose ;
β-D-glucopyranosyl-(1→3)-β-D-glucopyranosyl-(1→3)-β-D-glucopyranosyl-(1→3)-β-D-glucopyranosyl-(1→3)-β-D-glucopyranosyl-(1→3)-4-désoxy-mannitol.

Ces nouveaux composés sont utiles en tant que composés pharmaceutiques.

L'invention a donc également pour objet des médicaments comprenant ces composés nouveaux avec au moins un excipient choisi en fonction du mode d'administration retenu et du dosage.

Ces médicaments peuvent être des médicaments destinés à une administration par voie orale, par voir intraveineuse ou par voie intrapéritonéale.

Les doses de composés de formule (I'), (II') sont fonction de la pathologie à traiter et du mode d'administration retenu. Elles sont de façon générale de 25 à 70 UI/kg de poids corporel par jour, plus préférentiellement de 35 à 45

UI/kg de poids corporel par jour.

Des médicaments utiles par voie orale sont des comprimés, granules, sirop, gélules, gel.

Ces médicaments peuvent également comprendre au moins un agent chimiothérapeutique choisi dans le groupe comprenant le cisplatine, vinblastine, paclitaxel, le taxol et ses dérivés, les anticorps monoclonaux tels que notamment Rituximab et Cituximab.

En particulier, ces composés sont utiles en tant qu'agents immunomodulateurs.

Un autre objet de l'invention est relatif à l'utilisation d'au moins un composé de formule (I') ou (II') pour le traitement d'une maladie choisie dans le groupe consistant en tumeur, cancer, maladie virale, maladie bactérienne, maladie fongique, maladie du système immunitaire, maladie auto-immune ou maladie liée à une déficience de l'immunostimulation, chez des animaux à sang chaud et des êtres humains.

Ces composés présentent une structure originale puisque la partie glucanique est reliée au résidu mannitol par l'un de ces hydroxyles secondaires par l'intermédiaire d'une liaison de configuration β. La présence des molécules de formule (I') et (II') dans lesquelles $R_1$ représente -OH a été mise en évidence dans la *Laminaria digita.* Aucune méthode de préparation n'a été décrite.

La préparation par voie chimique des nouveaux composés de formule (I') ou (II') repose principalement sur une épimérisation de l'entité réductrice terminale de type D-glucose en résidu de type D-mannose. Ceci implique la singularisation de l'entité réductrice par rapport aux autres unités glucose de l'oligossaccharide et la singularisation de l'hydroxyle 2 de cette entité par rapport aux autres fonctions de l'unité réductrice. Deux voies de synthèse différentes sont envisagées, une synthèse itérative et une synthèse par épimérisation de l'entité terminale.

Selon le premier mode de réalisation, le procédé de préparation des nouveaux composés comprend une réaction entre un donneur de glycosyle de formule (D) ci-dessous et un accepteur de glycosyle de formule (A) ci-dessous :

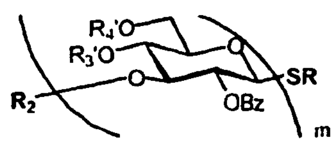

(D)

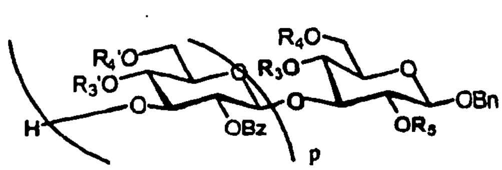

(A)

dans lesquelles

m est un entier de 1 à 9, de préférence de 1 à 6, et plus préférentiellement encore égal à 2, 3 ou 4;
p est un entier de 0 à 9, de préférence de 0 à 6, et plus préférentiellement encore égal à 2, 3 ou 4 ;
R représente alkyle tel que éthyle, méthyle, propyle ou butyle, ou aryle tel que phényle ou tolyle ;
$R_2$ représente allyle, méthylnaphtyle, benzyle, paraméthoxybenzyle, halogénoacétyle (chloroacétyle, bromoacétyle, iodoacétyle) ;
$R_3$ et $R_4$ d'une part et $R_3$' et $R_4$' d'autre part forment ensemble un radical éthylidyle, isopropylidyle, hexafluoroisopropylidyle, cyclopentylidyle, cyclohexylidyle, cycloheptylidyle, butylidyle, 1-tertiobutyléthylidyle, benzylidyle, méthoxybenzylidyle, 1-phénylbenzylidyle ou $R_3$, $R_4$, $R_3$' et $R_4$' représentent chacun indépendamment l'un de l'autre un benzyle, chlorobenzyle, nitrobenzyle, allyle, triarylméthyle, trialkylsilyle tel que triéthylsilyle, tri-isopropylsilyle, tertiobutyldiméthylsilyle, ester tel que acétyle, chloroacétyle, benzoyle, pivaloyle ;
$R_5$ représente H, un groupe lévulinoyle, acétyle, chloroacétyle, fluorénylméthyloxycarbonyle, trialkylsilyle tel que triéthylsilyle, tri-isopropylsilyle, tertiobutyldiméthylsilyle, de préférence un groupe lévulinoyle,
à la condition que aucun de $R_3$, $R_4$, $R_3$', $R_4$' ne soit identique à $R_5$.

Dans la présente demande les abréviations suivantes sont utilisées :

Bn : benzyle, NAP : 2-méthylnaphtyle, Bz : benzoyle, X : halogène, SEt :Ethylthio-,

Ac : acétyle, Lev : levulinoyle.

De façon avantageuse, le composé de formule (A) est

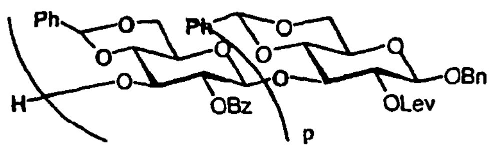

De façon avantageuse, le composé de formule (D) est

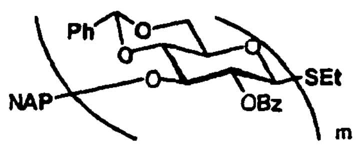

Cette étape du procédé peut s'inclure dans une synthèse itérative qui diffère de celle décrite dans FR2804684 par la nécessaire utilisation d'un premier accepteur comportant en position 2 un groupement protecteur orthogonal de tous les autres groupements présents sur les oligomères synthétisés après élongation de la chaîne, ce qui rend possible l'épimérisation régiosélective.

Cette synthèse itérative peut être décrite selon le schéma réactionnel (schéma réactionnel 1) tel que donné ci-après :

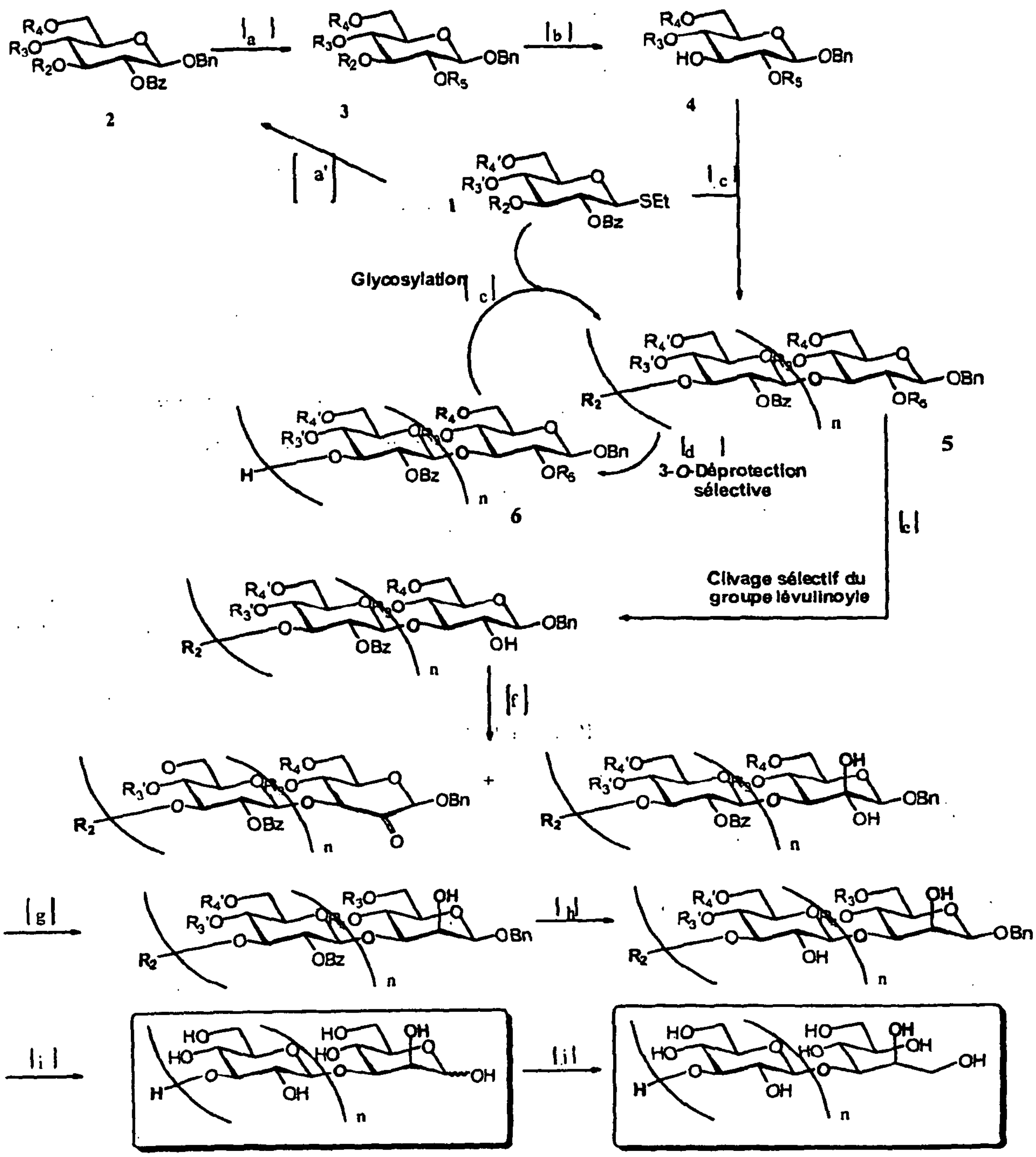


Le schéma réactionnel 1 ci-dessus comprend dans l'ordre suivant :

- la préparation [a'] à partir d'un composé 1 tel qu'obtenu dans FR2804694, d'un composé 2 dont le groupement -SEt a été remplacé par un groupement -OBn ;
- la substitution [a] en position 2 de -OBz par -$OR_5$, $R_5$ étant de préférence lévulinoyle, et la déprotection [b] sélective de l'hydroxyle en position 3 ;
- la réaction entre le donneur 1 et l'accepteur 4 [c], ou glycosylation;
- éventuellement la déprotection [d] sélective en position 3 de l'hydroxyle et la glycosylation entre le composé 1 et le composé 6, ces deux étapes pouvant être réitérées jusqu'à l'obtention du nombre n d'unités glucose souhaité ;
- clivage [e] sélectif du groupe levulinoyle (Lev) ; de façon avantageuse ce clivage se fait avec $NH_2NH_2$ dans AcOH.
- oxydation [f], de préférence avec le periodinane de Dess-Martin;
- réduction épimérisante [g] ; avantageusement avec le L-sélectride ;
- déprotection [h] de tous les groupements ester, avantageuse avec MeONa/MeOH;

- clivage [i] de tous les groupes hydrogénolysables, avantageuse avec $H_2$, $Pd(OAc)_2$ ;
- éventuellement réduction [j] du mannose en mannitol correspondant, avantageusement avec $NaBH_4$ dans MeOH.

Il est également possible d'envisager la synthèse en mettant en oeuvre un schéma réactionnel plus convergent, tel que défini ci-après (schéma réactionnel 2).
Dans ce schéma réactionnel, le donneur D est un disaccharide, ou un oligosaccharide supérieur, que l'on fait réagir avec un accepteur A qui est également un disaccharide ou qui est un oligosaccharide supérieur : tri-, tétra-, penta-, tel qu'obtenu en tant que composé 6 selon le schéma réactionnel 1.

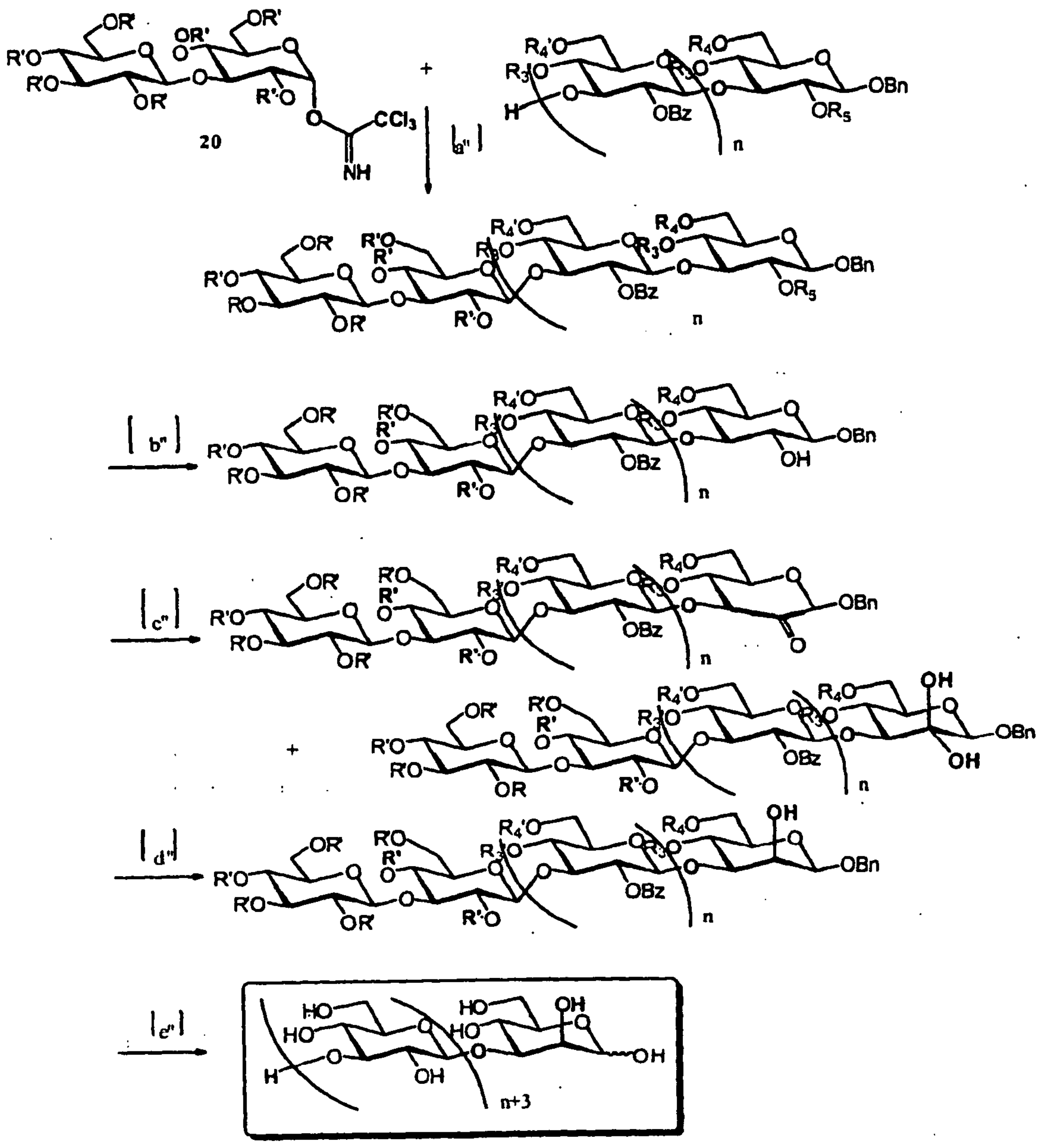


Dans ce second schéma réactionnel, de préférence R' est Ac, Bz ou pivaloyle et $R_5$ est Lev.
Ce second schéma réactionnel comprend dans l'ordre suivant :

- la réaction [a"] entre un disaccharide donneur de glycosyle (20) tel qu'obtenu dans FR2804694, et un accepteur de glycosyle tel que préparé selon le premier schéma réactionnel, avantageusement en présence de TMSOTf (acide trifluorométhanesulfonique);
- clivage [b"] sélectif du groupe levulinoyle (Lev), avantageusement avec $NH_2NH_2$ dans AcOH ;

- Oxydation [c"], avantageusement avec le peridionane de Dess-Martin;
- réduction épimérisante [d"], avantageusement avec le L-selectride ;
- déprotection [e"] complète, avantageusement par MeONa/MeOH, réaction suivie de $H_2$, $Pd(OAc)_2$.

Selon un second mode de réalisation, le procédé de préparation des nouveaux composés comprend une étape d'épimérisation en position 2 du chaînon terminal. Cette voie de synthèse permet également d'obtenir les composés désoxygénés en position 4.

Le schéma réactionnel 3 ci-dessous illustre ce mode de réalisation :

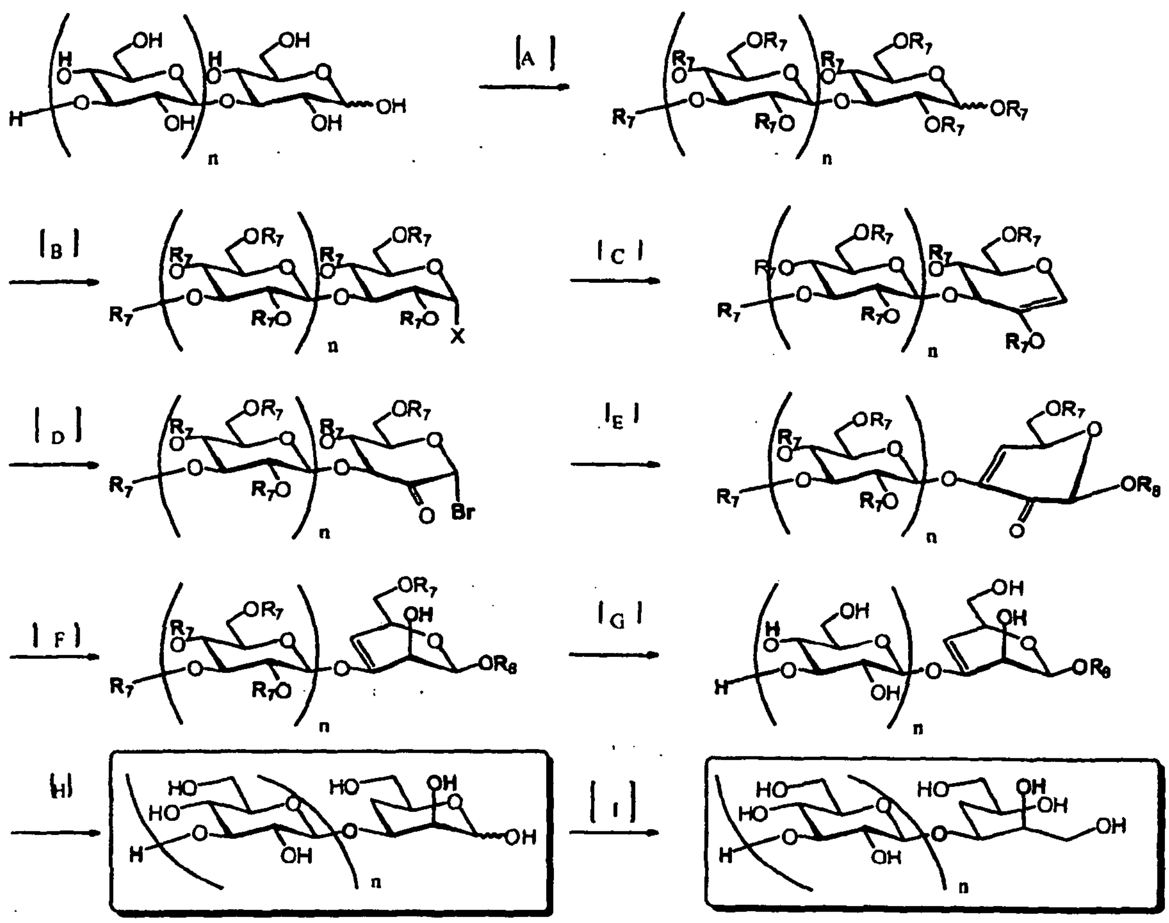

Dans le schéma réactionnel 3, $R_7$ est un ester, notamment acétyle, benzoyle, pivaloyle, de préférence Bz, et $R_8$, un groupement orthogonal à tous les autres groupements protecteurs, peut être Bn, NAP, p-méthoxybenzyle, p-méthoxyphényle, allyle, et de préférence Bn, X est de préférence un brome.

Ce troisième schéma réactionnel comprend dans l'ordre suivant :

- la réaction [A] de protection de tous les groupes OH, de préférence le groupe protecteur est Bz et la réaction est alors conduite avec du BzCl dans la pyridine,
- halogénation sélective [B], avantageusement bromation par HBr dans AcOH;
- élimination [C], avantageusement avec DBU diazabicylcoundécène;
- addition [D], avantageusement avec N-bromosuccinimide dans MeOH
- substitution en position anomérique et élimination du groupe hydroxy protégé en position 4 de l'entité terminale [E], avantageusement avec $Ph_3PO$ ou un acide de Lewis tel que le triflate d'argent dans l'alcool benzylique ou tout autre alcool parmi lesquels le naphthalèneméthanol, l'alcool p-méthoxybenzylique, le p-méthoxyphénol, l'alcool allylique;
- réduction épimérisante [F], avantageusement avec le L-sélectride ;
- déprotection [G] sélective des groupes protecteurs $R_7$, avantageusement dans MeONafi4eOH ;
- déprotection [H] sélective du groupe protecteur $R_8$, avantageusement avec $H_2$ $Pd(OAc)_2$ lorsque le groupement

est hydrogénolysable;

- réduction [I] du désoxy-mannose en désoxy-mannitol, avantageusement avec $NaBH_4$ dans $EtOH/H_2O$.

Les intermédiaires de synthèse utilisés dans les procédés décrits ci-dessus sont des produits nouveaux. L'invention a donc également pour objet, les composés de formules (III), (IV), (V) et (VI) :

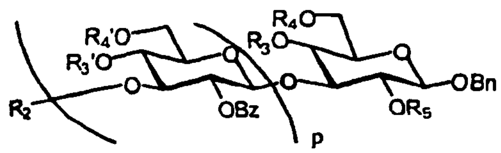

(III)

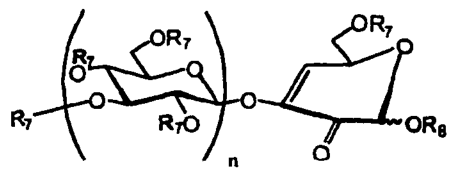

(IV)

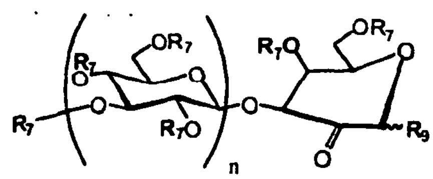

(V)

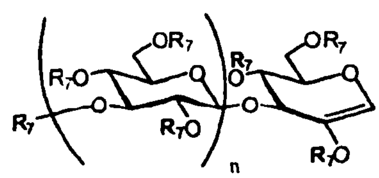

(VI)

dans lesquelles :

p est un entier de 0 à 9, de préférence de 0 à 6, et plus préférentiellement encore égal à 2, 3 ou 4 ;
$R_2$ représente hydrogène, allyle, méthylnaphtyle, benzyle, paraméthoxybenzyle, halogénoacétyle (chloroacétyle, bromoacétyle, iodoacétyle) ;
$R_3$ et $R_4$ d'une part et $R'_3$ et $R'_4$ d'autre part, forment ensemble un radical éthylidyle, isopropylidyle, hexafluoroisopropylidyle, cyclopentylidyle, cyclohexylidyle, cycloheptylidyle, butylidyle, 1-tertiobutyléthylidyle, benzylidyle, méthoxybenzylidyle, 1-phénylbenzylidyle,
ou $R_3$, $R_4$, $R_3$' et $R_4$' représentent chacun indépendamment l'un de l'autre un benzyle, chlorobenzyle, nitrobenzyle, allyle, triarylméthyle, trialkylsilyle tel que triéthylsilyle, tri-isopropylsilyle, tertiobutyldiméthylsilyle, ester tel que acétyle, chloroacétyle, benzoyle, pivaloyle ;
$R_5$ représente H, un groupe lévulinoyle, acétyle, chloroacétyle, fluorénylméthyloxycarbonyle, trialkylsilyle tel que triéthylsilyle, tri-isopropylsilyle, tertiobutyldiméthylsilyle, de préférence un groupe lévulinoyle,
à la condition que aucun de $R_3$, $R_4$, $R_3$', $R_4$' ne soit identique à $R_5$;
$R_7$ représente un ester, notamment acétyle, benzoyle, pivaloyle, de préférence Bz ;
$R_8$, un groupement orthogonal à tous les autres groupements protecteurs, qui peut être choisi parmi Bn, NAP, p-méthoxybenzyle, p-méthoxyphényle, allyle, et de préférence $R_8$ représente Bn,
$R_9$ représente $OR_8$ ou X.

Les composés de formule (III) sont les composés accepteurs A tels que définis précédemment.

L'invention sera encore mieux comprise à l'aide du complément de description qui suit et des exemples qui ne sont nullement limitatifs mais correspondent à des modes de réalisation avantageux.

## EXEMPLES

Les Exemples 1 à 7 illustrent les procédés de synthèse des produits de l'invention, et les exemples 8 à 11 montrent l'activité biologique des composés de l'invention.

### EXEMPLE 1: Préparation de selon le schéma réactionnel 1 du β-D-glucopyranosyl-(1→3)-β-D-glucopyranosyl-(1→3)-(α,β)-D-mannopyranose (produit A1)

Les produits 1 et 2 sont préparés selon le procédé décrits dans FR 2 804 684 et Jamois, F.; Ferrières, V.; Guégan, J.-P.; Yvin, J.-C.; Plusquellec, D.; Vetvicka, V. Glycobiology 2005, 15, 393-407.

#### [a]- Préparation de 4,6-*O*-benzylidène-3-*O*-(2-méthylnaphtyl)-2-*O*-levulinoyl-β-D-glucopyranoside de benzyle (produit 3)

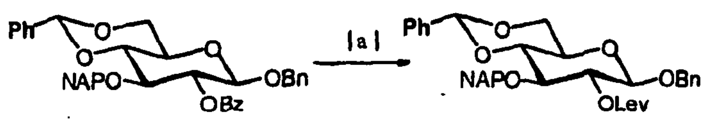


Le composé **2** (1 g, 2,006 mmol) est mis en solution dans 50 mL de dichlorométhane puis additionné de DMAP (50 mg, 4,093 mmol), d'acide levulinoïque (250 μL, 2,441 mmol) et de DCC (500 mg, 2,423 mmol). Après 5 heures d'agitation à température ambiante, la DCU est éliminée par filtration, puis le milieu est repris par 50 mL de dichlorométhane et lavé par une solution aqueuse à 10% d'acide chlorhydrique, par une solution aqueuse saturée d'hydrogénocarbonate de sodium et par une solution aqueuse saturée de chlorure de sodium. Après séchage sur $MgSO_4$ de la phase organique et concentration, le composé est purifié sur gel de silice [éther de pétrole/acétate d'éthyle (3:1 ; v/v)] pour conduire de manière quantitative à 1,2 g du composé recherché 3.

Produit 3 : solide blanc ; **Pf** (°C) 133 ; **Rf** (EP/AcOEt, 3:1) 0,4 ; $[\alpha]^{20}_{D}$ (c=1,0, $CH_2Cl_2$).
**RMN $^1$H ($CDCl_3$, 400 MHz)** δ (ppm) : 7,83-7,74 (m, 4H, H arom.) ; 7,53-7,28 (m, 13H, H arom.) ; 5,61 (s, 1H, H7) ; 5,01 (d, 1H, H9, $J_{H9-H9'}$ = 12,4 Hz) ; 4,87 (d, 1H, H9') ; 4,86 (d, 1H, H8, $J_{H8-H8'}$ = 12,4 Hz) ; 4,60 (d, 1H, H8') ; 2,58 (t, 2H, H12, $J_{H11-H12}$ = 6,6 Hz) ; 2,44 (t, 2H, H11) ; 2,09 (s, 3H, H 14) et tableau 1a.
**RMN $^{13}$C ($CDCl_3$, 100 MHz)** δ (ppm) : 206,2 (C13) ; 171,3 (C10) ; 137,1, 136,9, 135,6, 133,1, 132,9 (5C, C quat. arom.); 129,0, 128,4, 128,3, 128,2, 128,0, 127,9, 127,8, 127,7, 127,6, 126,8, 126,1, 126,0, 125,8 (C arom.) ; 101,3 (C7) ; 74,1 (C9) ; 70,7 (C8) ; 37,7 (C12) ; 29,8 (C14) ; 27,8 (C11) et tableau 1b.
**Analyse élémentaire** ($C_{36}H_{36}O_8$) Théorique : C = 72,47 %, H = 6,08 % ; Mesurée : C = 72,05 %, H = 6,12 %.
**HRMS (ESI$^+$) :** $[M+Na]^+$ $C_{36}H_{36}NaO_8$ : m/z théorique : 619,2308, m/z mesurée : 619,2308 ; $[M+K]^+$ $C_{36}H_{36}KO_8$ : m/z théorique: 635,2047, m/z mesurée: 635,2050.

#### [b] Préparation de 4,6-*O*-benzylidène-2-*O*-levulinoyl-β-D-glucopyranoside de benzyle (Produit 4)

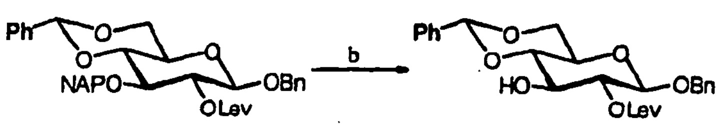


4

Dans 50 mL d'un mélange dichlorométhane/méthanol (4:1 ; v/v) sont introduits le monosaccharide **3** (1,1 g,

1,844 mmol) et la 2,3-dichloro-5,6-dicyano-1,4-benzoquinone (DDQ) (810 mg, 3,568 mmol) à température ambiante. Après 5 heures de réaction, le milieu est dilué avec 50 mL de dichlorométhane et lavé 2 fois avec une solution aqueuse saturée d'hydrogénocarbonate de sodium. Après séchage ($MgSO_4$) et concentration de la phase organique, le résidu est purifié sur gel de silice [éther de pétrole/acétate d'éthyle (3:1 ; v/v)]. On obtient ainsi 690 mg du produit recherché **4** avec un rendement de 82%.

**Produit 4** : solide blanc; **Pf** (°C) 122 ; **Rf** (EP/AcOEt, 3:1) 0,2 ; $[\alpha]_D^{20}$ (c=1,0, $CH_2Cl_2$).

**RMN $^1$H ($CDCl_3$, 400 MHz)** δ (ppm) : 7,50-7,48 (m, 2H, H arom.) ; 7,38-7,28 (m, 8H, H arom.) ; 5,55 (s, 1H, H7) ; 4,89 (d, 1H, H8, $J_{H8-H8'}$ = 12,3Hz) ; 4,62 (d, 1H, H8') ; 3,05 (s, 1H, OH3) ; 2,79-2,73 (m, 2H, H11) ; 2,59-2,51 (m, 2H, H10) ; 2,09 (s, 3H, H14) ; 2,16 (s, 3H, H13) et tableau 1a.
**RMN $^{13}$C ($CDCl_3$, 100 MHz)** δ (ppm) : 207,1 (C12) ; 172,0 (C9) ; 136,9 (2C, C quat. arom.) ; 129,2, 128,4, 128,3, 127,9, 127,7, 126,3 (C arom.); 101,9 (C7); 70,8 (C8) ; 38,1 (C11) ; 29,8 (C13) ; 28,0 (C10) et tableau 1b.
**Analyse élémentaire** ($C_{25}H_{28}O_8$) : Théorique : C = 65,78 %, H = 6,18 % ; Mesurée : C = 66,04 %, H = 6,23 %.
**HRMS (ESI$^+$)** : $[M+Na]^+$ $C_{25}H_{28}NaO_8$ : m/z théorique: 479,1682, m/z mesurée : 479,1682 ; $[M+K]^+$ $C_{25}H_{28}KO_8$ : m/z théorique : 495,1421, m/z mesurée : 495,1395.

**[c] Préparation de 2-*O*-benzoyl-4,6-*O*-benzylidène-3-*O*-(2-méthylnaphtyl)-β-D-glucopyranosyl-(1→3)-4,6-*O*-benzylidène-2-*O*-levulinoyl-β-D-glucopyranoside de benzyle (Produit 5)**

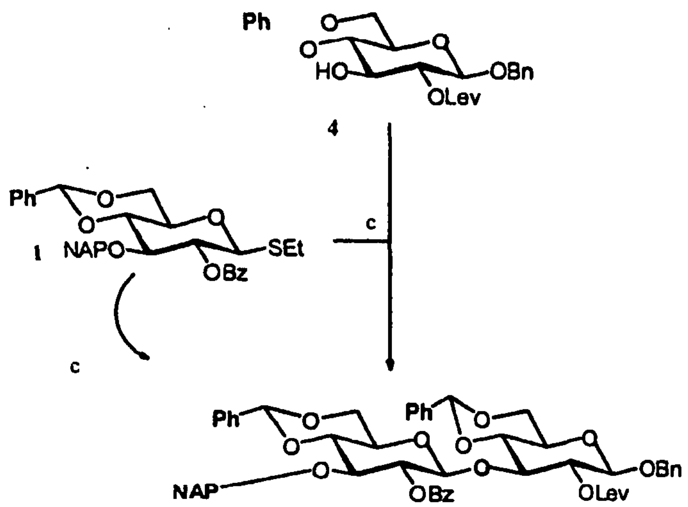


Dans un ballon sont introduits le donneur **1** (1,73 g, 3,108 mmol) et l'accepteur **4** (1,29 g, 2,826 mmol) dans le dichlorométhane anhydre à -60 °C, en présence de tamis moléculaire 4Å. Le milieu est alors additionné de *N*-iodosuccinimide (NIS) (763 mg, 3,390 mmol) et de triflate de triméthylsilyle (50 μL, 0,283 mmol). Après 3 heures de réaction, le milieu est neutralisé par de la triéthylamine, filtré puis concentré. Après purification par chromatographie sur gel de silice [toluène/acétate d'éthyle (9:1 ; v/v)], 2,51 g du produit recherché **5** est obtenu avec un rendement de 94%.

**Produit 5** : solide blanc ; **Pf** (°C) 178-179 ; **Rf** (EP/AcOEt, 3:1) 0,2 ; $[\alpha]_D^{20}$ - 21,0 (c=1,0, $CH_2Cl_2$).

**RMN $^1$H ($CDCl_3$, 400 MHz)** δ (ppm) : 7,90-7,86 (m, 2H, H arom.) ; 7,70-7,67 (m, 1H, H arom.) ; 7,60-7,21 (m, 24H, H arom.) ; 5,53 (s, 1H, H7b) ; 5,28 (s, 1H, H7a) ; 4,94 (d, 1H, H8b, $J_{H8b}$-$H_{8'b}$ = 12,1 Hz) ; 4,84 (d, 1H, H8'b) ; 4,80 (d, 1H, H8a, $J_{H8a-H8'a}$ = 12,2 Hz) ; 4,52 (d, 1H, H8'a) ; 2,66-2,46 (m, 2H, H10a) ; 2,40-2,27 (m, 2H, H11a) ; 2,08 (s, 3H, H13a) et tableau 2a.
**RMN $^{13}$C ($CDCl_3$, 100 MHz)** δ (ppm) : 206,4 (C12a); 171,1 (C9a); 165,0 (OCOPh) ; 137,3, 137,0, 136,7, 135,3, 133,1, 133,0 (7C, C quat. arom.); 132,8, 129,7, 129,4, 128,9, 128,3, 128,2, 127,9, 127,8, 127,7, 127,5, 126,7, 126,1, 126,0, 125,8, 125,6 (C arom.) ; 101,7 (C7b); 100,9 (C7a); 73,4 (C8b); 70,6 (C8a); 37,8 (C11a) ; 29,7 (C13a) ; 27,5 (C10a) et tableau 2b.
**Analyse élémentaire** ($C_{56}H_{54}O_{14}$) : Théorique : C = 70,72%, H = 5,72% ; Mesurée : C = 70,89%, H = 5,72%.
**HRMS (ESI$^+$):** $[M+Na]^+$ $C_{56}H_{54}NaO_{14}$, m/z théorique : 973,3411 ; m/z mesurée : 973,3412.

**[d] Préparation de 2-*O*-benzoyl-4,6-*O*-benzylidène-β-D-glucopyranosyl-(1→3)-4,6-*O*-benzylidène-2-*O*-levulinoyl-β-D-glucopyranoside de benzyle (Produit 6)**

La 2,3-dichloro-5,6-dicyano-1,4-benzoquinone (820 mg, 3,600 mmol) est ajoutée à une solution du composé **5** (1,71 g, 1,800 mmol) dans un mélange dichlorométhane/méthanol (4:1 ; v/v). Après 16 heures de réaction, le milieu est dilué au dichlorométhane, lavé par une solution saturée d'hydrogénocarbonate de sodium et à l'eau. La phase organique est séchée ($MgSO_4$), évaporée et le produit recherché 6 est obtenu après purification sur gel de silice [éther de pétrole/acétate d'éthyle (2:1 ; v/v)] (1,325 g, Rdt=91%).

**Produit 6 :** solide blanc ; **Pf** (˚C) 214 ; **Rf** (EP/AcOEt, 2:1) 0,3 ; $[\alpha]_D^{20}$ - 43,0 (c=1,0, $CH_2Cl_2$).

**RMN $^1$H ($CDCl_3$, 400 MHz)** δ (ppm) : 8,06-8,01 (m, 2H, H arom.) ; 7,63-7,23 (m, 19H, H arom.) ; 5,57 (s, 1H, H7b) ; 5,31 (s, 1H, H7a) ; 4,83 (d, 1H, H8a, $J_{H8a-H8'a}$ = 12,2 Hz) ; 4,55 (d, 1H, H8'a) ; 2,72 (s, 1H, OH3b) ; 2,65-2,49 (m, 2H, H10a) ; 2,37-2,25 (m, 2H, H11a) ; 2,12 (s, 3H, H13a) et tableau 2a.

**RMN $^{13}$C ($CDCl_3$, 100 MHz)** δ (ppm) : 206,4 (C12a) ; 171,1 (C9a) ; 165,7 (OCOPh) ; 137,0, 136,9, 136,7 (3C, C quat. arom.); 133,3, 129,8 (C arom.); 129,5 (C quat. arom.) ; 129,3, 129,1, 128,4, 128,3, 128,2, 128,1, 127.8, 127,6, 126,2, 126,0 (C arom.) ; 101,6 (C7b); 101,5 (C7a) ; 70,6 (C8a) ; 37,7 (C11a) ; 29,7 (C13a) ; 27,5 (C10a) et tableau 2b.

**Analyse élémentaire** ($C_{45}H_{46}O_{14}$) Théorique : C = 66,66%, H = 5,72% ; Mesurée : C = 66,53%, H = 5,80%.

**HRMS (ESI$^+$):** $[M+Na]^+$ $C_{45}H_{46}NaO_{14}$ : m/z théorique : 833,2785 ; m/z mesurée : 833,2785 ; $[M+K]^+$ $C_{45}H_{46}KO_{14}$ : m/z théorique : 849,2525, m/z mesurée : 849,2530.

**[c]- Préparation de 2-*O*-benzoyl-4,6-*O*-benzylidène-3-*O*-(2-méthylnaphtyl)-β-D-glucopyranosyl-(1→3)-2-*O*-benzoyl-4,6-*O*-benzylidène-β-D-glucopyranosyl-(1→3)-4,6-*O*-benzylidène-1-*O*-levulinoyl-β-D-glucopyranoside de benzyle (Produit 7)**

Dans un ballon sont introduits le donneur **1** (226 mg, 0,406 mmol) et l'accepteur **6** (300 mg, 0,370 mmol) dans le dichlorométhane anhydre à -80 ˚C, en présence de tamis moléculaire 4Å. Le milieu est ensuite additionné de *N*-iodosuccinimide (100 mg, 0,444 mmol) et de triflate de triméthylsilyle (6,7 μL, 0,037 mmol). Après 2 heures d'agitation, la réaction est terminée et le milieu est neutralisé par de la triéthylamine, filtré puis concentré. Après purification par chromatographie sur gel de silice [toluène/acétate d'éthyle (9:1 ; v/v)], 328 mg trisaccharide attendu 7 sont obtenus avec un rendement de 68%.

**Produit 7** : solide blanc ; **Pf** (˚C) 115 ; **Rf** (EP/AcOEt, 2:1) 0,5; $[\alpha]_D^{20}$. + 4,7 (c=1,0, $CH_2Cl_2$).

**RMN $^1$H ($CDCl_3$, 400 MHz)** δ (ppm) : 7,95-7,86 (m, 4H, H arom.) ; 7,70-7,13 (m, 33H, H arom.) ; 5,51 (s, 1H, H7) ; 5,43 (s, 1H, H7) ; 4,94 (d, 1H, H8c, $J_{H8c-H8'c}$ = 12,2 Hz) ; 4,83 (d, 1H, H8'c) ; 4,78 (d, 1H, H8a, $J_{H8a-H8'a}$ = 12,4 Hz) ; 4;50 (s, 1H, H7) ; 4,49 (d, 1H, H8'a) ; 2,69-2,47 (m, 2H, H10a) ; 2,40-2,21 (m, 2H, H11a) ; 1,95 (s, 3H, H13a) et tableau 3a.

**RMN $^{13}$C ($CDCl_3$, 100 MHz)** δ (ppm) : 206,6 (C12a) ; 171,1 (C9a) ; 165,0, 164,8 (2C, OCOPh) ; 137,3, 137,1, 137,0, 135,4 (C quat. arom.) ; 133,3, 133,1 (C arom.) ; 133,0, 132,8 (C quat. arom.) ; 129,8, 129,7 (C arom.) ; 129,4 (C quat. arom.) ; 129,4, 128,9, 128,7, 128,6, 128,3, 128,2, 128,1, 127,9, 127,8, 127,7, 127,6, 127,5, 126,6, 126,4, 126, 1, 126,0, 125,7, 125,6 (C arom.); 102,1 (C7); 101,1 (C7) ; 100,9 (C7); 73.,8 (C8c) ; 70,4 (C8a) ; 37,7 (C11a) ; 29,3 (C13a) ; 27,6 (C10a) et tableau 3b.

**Analyse élémentaire** ($C_{76}H_{72}O_{20}$) : Théorique : C = 69,93 %, H = 5,56 % ; Mesurée : C = 69,89 %, H = 5,63 %.

**HRMS (ESI$^+$)** : $[M+Na]^+$ $C_{16}H_{12}NaO_{20}$ m/z théorique : 1327,4515, m/z mesurée : 1327,4522 ; $[M+K]^+$ $C_{76}H_{72}KO_{20}$: m/z théorique : 1343,4254, m/z mesurée : 1343,4206.

**[e]- Préparation de 2-*O*-benzoyl-4,6-*O*-benzylidène-3-*O*-(2-méthylnaphtyl)-β-D-glucopyranosyl-(1-3)-2-*O*-benzoyl-4,6-*O*-benzylidène-β-D-glucopyranosyl-(1→3)-4,6-*O*-benzylidène-β-D-glucopyranoside de benzyle (Produit 12)**

7,6 mL d'une solution 1M d'hydrazine dans un mélange pyridine/acide acétique (3:2, v/v) sont additionnés goutte à goutte à une solution du trisaccharide **7** (500 mg, 0,383 mmol) dans la pyridine (7,6 mL). Après 3 heures d'agitation, la réaction est stoppée par ajout de 2,1 mL de 2,4-pentane-dione et le milieu est concentré sous pression réduite. Le résidu est ensuite repris par 50 mL de dichlorométhane et lavé par une solution d'acide chlorhydrique à 10%, par une solution aqueuse saturée d'hydrogénocarbonate de sodium et par une solution aqueuse saturée de chlorure de sodium. Après séchage ($MgSO_4$) et concentration, le produit est purifié par chromatographie sur gel de silice [éther de pétrole/acétate d'éthyle (2:1 ; v/v)] pour conduire à 377 mg du composé **12** avec un rendement de 82%.

**Produit 12 :** solide blanc ; **Pf** (°C) 136 ; **Rf** (EP/AcOEt, 2:1) 0,5 ; $[\alpha]_D^{20}$ + 13,4 (c=1,0, $CH_2Cl_2$).

**RMN $^1$H ($CDCl_3$, 400 MHz)** δ (ppm) : 7,94-7,90 (m, 4H, H arom.) ; 7,68-7,66 (m, 1H, H arom.) ; 7,58-7,19 (m, 32H, H arom.) ; 5,54 (s, 1H, H7) ; 5,52 (s, 1H, H7) ; 4,95 (d, 1H, H8c, $J_{H8c-H8'c}$ = 12,4 Hz) ; 4,88 (d, 1H, H8a, $J_{H8a-H8'a}$ = 11,6 Hz) ; 4,83 (d, 1H, H8'c) ; 4,77 (s, 1H, H7) ; 4,53 (d, 1H, H8'a) et tableau 3a.

**RMN $^{13}$C ($CDCl_3$, 100 MHz)** δ (ppm) : 165,1, 164,7 (2C, OCOPh) ; 137,3, 137,2, 136,9, 135,3, 133,0, 132,8, 129,2 (8C, C quat. arom.) ; 133,4, 133,0, 129,8, 129,7, 129,3 (C arom.) ; 129,2 (C quat. arom.) ; 129,0, 128,7, 128,5, 128,4, 128,3, 128,2, 128,0, 127,9, 127,8, 127,5, 126,7, 126,4, 126,1, 126,0, 125,7, 125,6 (C arom.); 102,0 (C7) ; 101,2 (C7) ; 100,5 (C7) ; 73,8 (C8c) ; 71,2 (C8a) et tableau 3b.

**Analyse élémentaire** ($C_{71}H_{66}O_{18}$): Théorique : C = 70,64 %, H = 5,51 % ; Mesurée : C = 70,49 %, H = 5,54 %.

**HRMS (ESI$^+$) :** $[M+Na]^+$ $C_{71}H_{66}NaO_{18}$ : m/z théorique : 1229,4147 ; m/z mesurée: 1229,4149; $[M+K]^+$ $C_{71}H_{66}KO_{18}$: m/z théorique : 1245,3886: m/z mesurée : 1245,3944; $[M-H+2Na]^+$ $C_{71}H_{65}Na_2O_{18}$: m/z théorique : 1251,3966 ; m/z mesurée: 1251,4040.

**[f]- Préparation de 2-*O*-benzoyl-4,6-*O*-benzylidène-3-*O*-(2-méthylnaphtyl)-β-D-glucopyranosyl-(1→3)-2-*O*-benzoyl-4,6-*O*-benzylidène-β-D-glucopyranosyl-(1→3)-4,6-*O*-benzylidène-β-D-glucopyranos-2-uloside de benzyle (Produit 15a) 2-*O*-benzoyl-4,6-*O*-benzylidène-3-*O*-(2-méthylnaphtyl)-β-D-glucopyranosyl-(1→3)-2-*O*-benzoyl-4,6-*O*-benzylidène-β-D-glucopyranosyl-(1→3)-4,6-*O*-benzylidène-2-*C*-hydroxyl-β-D-glucopyranoside de benzyle (Produit 15b)**

Le composé déprotégé **12** (330 mg, 0,273 mmol) est additionné de 9 mL d'un mélange DMSO/acide acétique (2:1, v/v). Après 48 heures d'agitation à température ambiante, le milieu est dilué par 50 mL de dichlorométhane et lavé par une solution aqueuse saturée d'hydrogénocarbonate de sodium et par de l'eau. Après concentration et plusieurs coévaporation au toluène, le milieu est purifié par chromatographie sur gel de silice [éther de pétrole/acétate d'éthyle (3:1 ; v/v)], pour conduire à 180 mg du produit oxydé **15a** (Rdt=55%) en mélange 2: avec sa forme hydratée **15b**.

**Produit 15a** : solide blanc ; **Rf** (EP/AcOEt, 2:1) 0,3.

**RMN $^1$H ($CDCl_3$, 400 MHz)** δ (ppm) : 7,95-7,91 (m, 4H, H arom.) ; 7,67-7,60 (m, 1H, H arom.) ; 7,57-7,10 (m, 32H, H arom.) ; 5,53 (s, 1H, H7) ; 5,41 (s, 1H, H7) ; 5,40 (dd, 1H, H2c, $J_{Hlc-H2c}$ = 7,8 Hz, $J_{H2c-H3c}$ = 8,4 Hz) ; 5,32 (d, 1H, H1c) ; 5,27 (dd, 1H, H2b, $J_{H1b-H2b}$ = 2,4 Hz, $J_{H2b-H3b}$ = 1,6 Hz) ; 5,19 (d, 1H, H1b) ; 4,94 (d, 1H, H8c, $J_{H8c-H8'c}$ = 11,7 Hz) ; 4,82 (d, 1H, H8'c) ; 4,82 (d, 1H, H8a, $J_{H8a-H8'a}$ = 12,0 Hz) ; 4,64 (s, 1H, H1a) ; 4,61 (d, 1H, H8'a) ; 4,42 (s, 1H, H7) ; 4,35 (d, 1H, H3a, $J_{H3a-H4a}$ = 10,4 Hz) ; 4,35 (t, 1H, H6a, $J_{H5a-H6a}$ = $J_{H6a-H6'a}$ = 5,5 Hz) ; 4,28 (dd, 1H, H6c, $J_{H5c-H6c}$ = 4,9 Hz, $J_{H6c-H6'c}$ = 10,4 Hz) ; 4,24 (dd, 1H, H4b, $J_{H3b-H4b}$ = 7,5 Hz, $J_{H4b-H5b}$ = 10,6 Hz) ; 4,11 (dd, 1H, H6b, $J_{H5b-H6b}$ = 4,8 Hz, $J_{H6b-H6'b}$ = 10,2 Hz) ; 4,01 (dd, 1H, H3b) ; 3,95 (t, 1H, H3c, $J_{H3c-H4c}$ = 8,4 Hz) ; 3,91 (t, 1H, H4c, $J_{H4c-H5c}$ = 8,4 Hz) ; 3,77 (t, 1H, H6'c, $J_{H5c-H6'c}$ = 10,4 Hz) ; 3,72 (ddd, 1H, H5b, $J_{H5b-H6'b}$ = 10,2 Hz) ; 3,63 (ddd, 1H, H5c) ; 3,56 (t, 1H, H6'a, $J_{H5a-H6'a}$ = 5,5 Hz) ; 3,55 (dt, 1H, H5a, $J_{H4a-H5a}$ = 9,0 Hz) ; 3,41 (t, 1H, H6'b) ; 2,98 (dd, 1H, H4a).

**RMN $^{13}$C ($CDCl_3$, 100 MHz)** δ (ppm) : 165,2, 165,0 (2C, OCOPh) ; 137,3, 137,2, 136,8, 135,9, 135,4 (C quat. arom.); 133,5, 133,3 (C arom.) ; 133,0, 132,8 (C quat. arom.); 129,8, 129,7, 129,3, 129,2 (C arom.); 129,0 (C quat. arom.) ; 129,0, 128,9, 128,7, 128,5, 128,4, 128,3, 128,2, 128,1, 128,0, 127,9, 127,8, 127,5, 126,7, 126,4, 126,2, 126,1, 126,0, 125,7, 125,6 (C arom.) ; 101,6 (C7) ; 101,2 (C7) ; 100,6 (C7) ; 98,6 (C1a) ; 97,6 (C1c) ; 96,1 (C1b) ; 81,6 (C4c) ; 79,9 (C4a) ; 78,1 (C3c) ; 77,4 (C4b) ; 75,9 (C3a) ; 75,8 (C3b) ; 73,9 (C8c) ; 73,1 (C2c) ; 71,8 (C2b) ; 70,3 (C8a) ; 68,9 (C6b) ; 68,7 (C6c) ; 68,3 (C6a) ; 66,4 (C5a) ; 66,2 (C5c) ; 64,9 (C5b).

**HRMS (ESI$^+$)** : $[M+Na]^+$ $C_{71}H_{64}NaO_{18}$ : m/z théorique : 1227,3990 ; m/z mesurée : 1227,3988 ; $[M+Na+CH_3OH]^+$ $C_{72}H_{68}NaO_{19}$ : m/z théorique : 1259,4252, m/z mesurée : 1259,4245.

**Produit 15b :**

**RMN $^1$H ($CDCl_3$, 400 MHz)** δ (ppm) : Signaux caractéristiques à 5,24 (d, 1H, H3a, $J_{H3a-H4a}$ = 2,9 Hz) ; 4,30 (s, 1H, H1a).

**RMN $^{13}$C ($CDCl_3$, 100 MHz)** δ (ppm) : Signaux caractéristiques à 100,4 (C1a) ; 93,4 (C2a).

**[g]- Préparation de 2-*O*-benzoyl-4,6-*O*-benzylidène-3-*O*-(2-méthylnaphtyl)-β-D-glucopyranosyl-(1→3)-2-*O*-benzoyl-4,6-*O*-benzylidène-β-D-glucopyranosyl-(1→3)-4,6-*O*-benzylidène-β-D-mannopyranoside de benzyle (Produit 18)**

Le composé oxydé **15a/15b** (180 mg, 0,149 mmol) est solubilisé dans un mélange dichlorométhane / THF (1: 1, v/v) puis additionné à - 78 °C de L-Sélectride (1M dans THF; 150 µL, 0,150 mmol). Après 45 minutes à - 78 °C, la réaction est supposée terminée et neutralisée par ajout de quelques gouttes d'acide acétique. Une fois le milieu revenu à température ambiante, 30 mL de dichlorométhane sont ajoutés et la phase organique est lavée par une solution

aqueuse d'acide chlorhydrique à 10%, par une solution aqueuse saturée d'hydrogénocarbonate de sodium et pour finir par une solution aqueuse saturée de chlorure de sodium. 180 mg du composé réduit mannoside souhaité **18** sont ainsi obtenus après séchage ($MgSO_4$) et concentration de la phase organique.

Produit **18** : solide blanc ; **Pf** (°C) 180 ; **Rf** (EP/AcOEt, 2:1) 0,2 ; $[\alpha]_D^{20}$ - 4,4 (c=1,0, $CH_2Cl_2$).

**RMN $^1$H ($CDCl_3$, 400 MHz)** δ (ppm) : 7,85-7,83 (m, 2H, H arom.) ; 7,73-7,67 (m, 3H, H arom.) ; 7,62-7,17 (m, 32H, H arom.) ; 5,50 (s, 1H, H7) ; 5,42 (s, 1H, H7) ; 5,21 (s, 1H, H7) ; 4,90 (d, 1H, H8c, $J_{H8c-H8'c}$ = 12,4 Hz) ; 4,87 (d, 1H, H8a, $J_{H8a-H8'a}$ = 12,2 Hz) ; 4,80 (d, 1H, H8'c) ; 4,59 (d, 1H, H8'a) et tableau 3'a.

**RMN $^{13}$C ($CDCl_3$, 100 MHz)** δ (ppm) : 164,8, 164,8 (2C, OCOPh) ; 137,2, 137,1, 136,5, 135,2 (C quat. arom.) ; 133,2 (C arom.) ; 132,9, 132,8 (C quat. arom.) ; 132,8, 129,7, 129,6 (C arom.) ; 129,4, 129,1 (C quat. arom.); 129,0, 128,9, 128,7, 128,5, 128,4, 128,3, 128,2, 128,1, 128,0, 127,8, 127,5, 126,7, 126,4, 126,3, 126,1, 126,0, 125,7, 125,6 (C arom.) ; 101,5 (C7); 101,1 (2 C, C7) ; 73,5 (C8c) ; 70,4 (C8a) et tableau 3'b.

**[h]- Préparation de 4,6-*O*-benzylidène-3-*O*-(2-méthylnaphtyl)-β-D-glucopyranosyl-(1→3)-4,6-*O*-benzylidène-β-D-glucopyranosyl-(1→3)-4,6-*O*-benzylidène-β-D-mannopyranoside de benzyle (Produit 19)**

Le composé **18** (170 mg, 0,141 mmol) est dissous dans 15 mL d'un mélange méthanol/dichlorométhane (2:1, v/v) puis additionné de 2 équivalents de méthylate de sodium (0,1M dans MeOH, 3 mL, 0,300 mmol). Après 6 heures d'agitation à température ambiante, le milieu est neutralisé par ajout de résine Amberlite IR120-$H^+$, filtré puis concentré sous pression réduite. Après chromatographie [dichlorométhane/méthanol (99:1 ; v/v)], le composé **19** est obtenu avec un rendement de 72% (126 mg).

**Produit 19 :** solide blanc ; **Pf** (°C) >230 ; **Rf** ($CH_2Cl_2$/MeOH, 97:3) 0,4 ; $[\alpha]_D^{20}$ - 43.5 (c=1,0, $CH_2Cl_2$).

**RMN $^1$H ($CDCl_3$, 400 MHz)** δ(ppm): 7,70-7,19 (m, 27H, H arom.) ; 5,47 (s, 1H, H7); 5,44 (s, 1H, H7) ; 5,42 (s, 1H, H7) ; 4,96 (d, 1H, H8c, $J_{H8c-H8'c}$ = 12,0 Hz) ; 4,88 (d, 1H, H8'c) ; 4,84 (d, 1H, H8a, $J_{H8a-H8'a}$ = 11,9 Hz) ; 4,56 (d, 1H, H8'a) et tableau 3'a.

**RMN $^{13}$C ($CDCl_3$, 100 MHz)** δ (ppm) : 137,1, 137,0, 136,9, 136,2, 135,8, 133,1, 132,8 (7C, C quat. arom.); 129,0, 128,9, 128,8, 128,5, 128,4, 128,3, 128,2, 128,1, 128,0, 127,9, 127,8, 127,6, 127,5, 127,4, 126,8, 126,5, 126,2, 126,1, 126,0, 125,9, 125,8, 125,7 (C arom.) ; 101,6 (C7) ; 101,2 (C7) ; 100,7 (C7) ; 74,4 (C8c) ; 70,7 (C8a) et tableau 3'b. **HRMS (ESI$^+$):** $[M+Na]^+$ $C_{57}H_{58}NaO_{16}$: m/z théorique : 1021,3623, m/z mesurée: 1021,3624 ; $[M+K]^+$ $C_{57}H_{58}KO_{16}$: m/z théorique: 1037,3362, m/z mesurée : 1037,3370; $[M-H+2Na]^+$ $C_{57}H_{57}Na_2O_{16}$: m/z théorique: 1043,3442, m/z mesurée: 1043,3463.

**[i]- β-D-glucopyranosyl-(1→3)-β-D- glucopyranosyl-(1→3)-(α,β)-D-mannopyranose (Produit A1)**

Dans 10 mL d'un mélange acétate d'éthyle/méthanol/dichlorométhane (2:2:1 ; v/v/v) sont introduits le trisaccharide 18 (90 mg, 0,090 mmol), puis l'acétate de palladium (60 mg, 0,267 mmol). Le milieu est alors agité vigoureusement à température ambiante sous atmosphère d'hydrogène pendant 7 jours. Après filtration sur célite, la phase hydroorganique est extraite au dichlorométhane, puis est mise à sec par coévaporation azéotropique avec de l'éthanol absolu pour conduire de manière quantitative au composé déprotégé (45 mg, 0,090 mmol). 25 mg du composé formé sont ensuite purifiés par chromatographie d'exclusion sur gel Sephadex G-10 (éluant : eau) et les fractions recueillies sont lyophilisées afin d'obtenir le produit recherché qui se présente sous la forme d'une mousse blanche.

**RMN$^1$H ($D_2O$, 400 MHz)** δ (ppm) : 5,13 (d, 1H, H1aα, $J_{H1a-H2a}$ = 1,8 Hz) ; 4,67 (d, 1H, H1c, $J_{H1c-H2c}$ =7,7 Hz) ; 4,53 (d, 1H, H1b, $J_{H1b-H2b}$ = 8,0 Hz) ; 4,03 (dd, 1H, H2a, $J_{H2a-H3a}$ = 3,1 Hz) ; 3,96 (dd, 1H, H3a, $J_{H3a-H4a}$ = 9,5 Hz) ; 3,90-3,80 (m, 3H, H6a, H6b, H6c) ; 3,80-3,73 (m, 1H, H4a) ; 3,72-3,55 (m, 5H, H5a, H6'a, H3b, H6'b, H6'c) ; 3,53-3,36 (m, 5H, H2b, H64b, H5b, H3c, H5c) ; 3,32 (t, 1H, H4c, $J_{H3c-H4c}$ = $J_{H4c-H5c}$ = 9,7 Hz) ; 3,27 (dd, 1H, H2c, $J_{H2c-H3c}$ = 9,3 Hz).

**RMN $^{13}$C ($D_2O$, 100 MHz)** δ (ppm) : 103,1 (C1c) ; 100,5 (C1b); 94,1 (C1aα); 84,5 (C3b); 78,5 (C3a); 76,3 (C5c); 75,9 (2C, C3c, C5b); 75,8 (C2c); 73,1 (C2b); 72,7 (C4a) ; 69,9 (C4c); 68,6 (C2a); 68,4 (C4b); 65,5 (C5a) ; 61,2, 61,0 (3C, C6a, C6b, C6c).

**HRMS (ESI$^+$):** $[M+Na]^+$ $C_{18}H_{32}NaO_{16}$: m/z théorique : 527,1588, m/z mesurée: 527,1587 ; $[M+K]^+$ $C_{18}H_{32}KO_{16}$: m/z théorique : 543,1327, m/z mesurée : 543,1343.

**EXEMPLE 2: Synthèse de différents intermédiaires:**

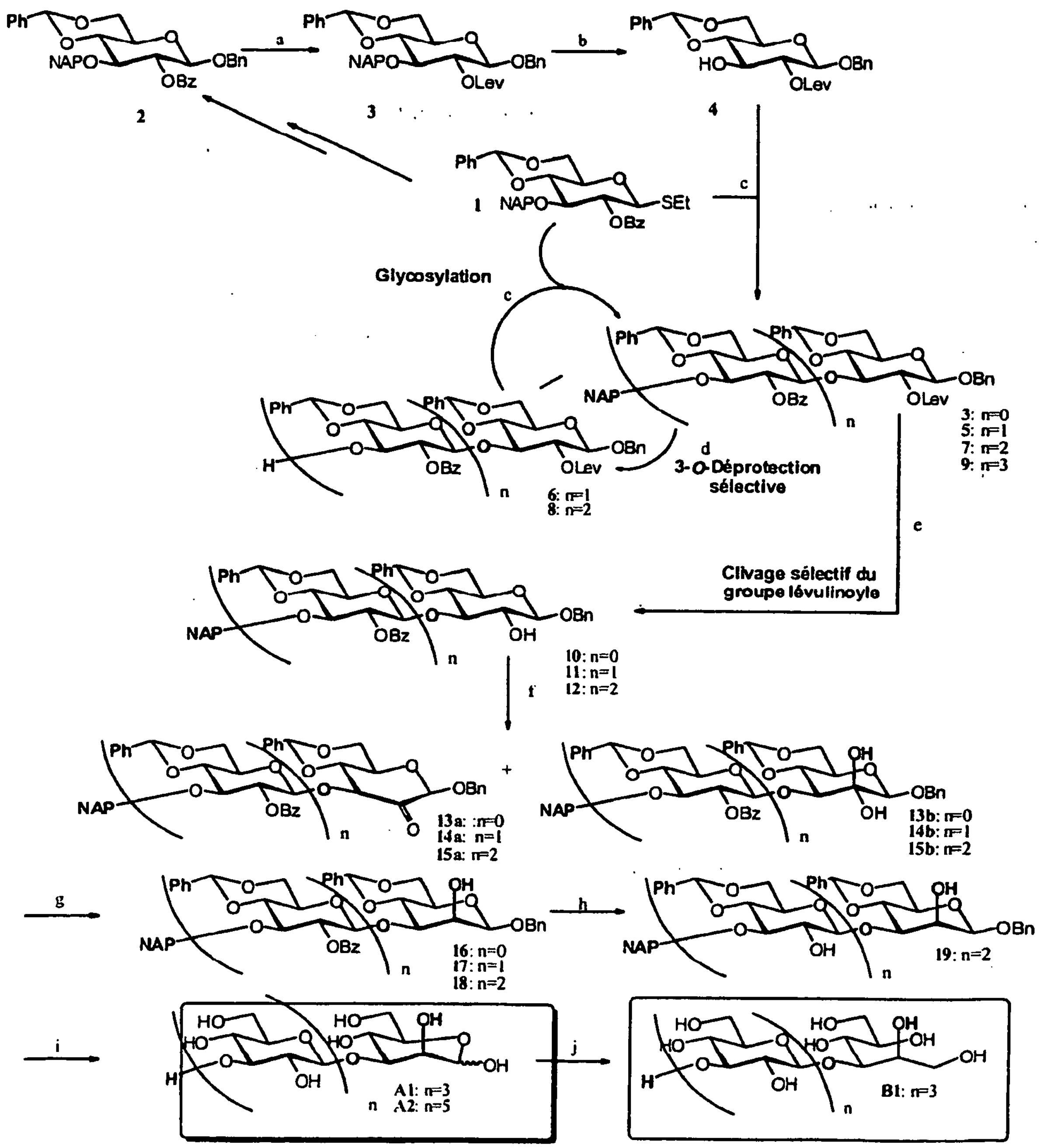


**2.1/ 2-*O*-benzoyl-4,6-*O*-benzylidéne-β-D-glucopyranosyl-(1→3)-2-*O*-benzoyl-4,6-*O*-benzylidène-β-D-glucopyranosyl-(1→3)-4,6-*O*-benzylidène-2-*O*-levulinoyl-β-D-glucopyranoside de benzyle (Produit 8)**

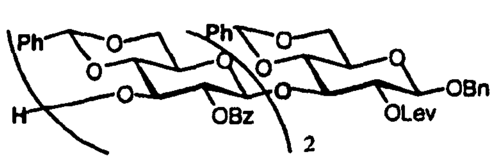

La 2,3-dichloro-5,6-dicyano-1,4-benzoquinone (176 mg, 0,774 mmol) est ajoutée à une solution du composé

trisaccharidique **7** (337 mg, 0,258 mmol) dans un mélange dichlorométhane/méthanol (4:1 ; v/v). Après 16 heures d'agitation à température ambiante, la réaction est terminée et le milieu est dilué au dichlorométhane, lavé par une solution saturée d'hydrogénocarbonate de sodium puis à l'eau. La phase organique est séchée sur $MgSO_4$, concentrée et le produit recherché **8** est obtenu après purification sur gel de silice [éther de pétrole/acétate d'éthyle (2:1 ; v/v)] (185 mg, Rdt = 72%).

**Produit 8 :** solide blanc ; **Rf** (EP/AcOEt, 2:1) 0,2.
**RMN[1]H ($CDCl_3$, 400 MHz)** δ (ppm) : 8,01-7,95 (m, 2H, H arom.) ; 7,90-7,84 (m, 2H, H arom.) ; 7,53-7,18 (m, 24H, H arom.) ; 7,15-7,09 (m, 2H, H arom.) ; 5,39 (s, 1H, H7) ; 5,37 (s, 1H, H7) ; 4,74 (d, 1H, H8a, $J_{H8a-H8'a}$ = 12,2 Hz) ; 4,47 (s, 1H, H7) ; 4,45 (d, 1H, H8'a) ; 2,66 (s, 1H, OH3c); 2,66-2,44 (m, 2H, H10a); 2,36-2,20 (m, 2H, H11a); 1,91 (s, 3H, H13a) et tableau 3a.
**RMN $^{13}$C ($CDCl_3$, 100 MHz)** δ (ppm) : 206,1 (C12a); 171,1 (C9a) ; 165,7, 164,8 (2C, OCOPh) ; 137,3, 137,0, 136,9, 133,5, 133,2 (6C, C quat. arom.) ; 129,9, 129,7, 129,5, 129,4, 129,2, 128,7, 128,4, 128,3, 128,2, 127,9, 127,8, 127,7, 127,6, 126,4, 126,2, 126,0 (C arom.) ; 102,1, 101,7, 100,4 (3C, C7) ; 70,4 (C8a) ; 37,7 (C11a); 29,3 (C13a); 27,7 (C10a) et tableau 3b.

**2.2/ 2-*O*-benzoyl-4,6-*O*-benzylidène-3-*O*-(2-méthylnaphtyl)-β-D-glucopyranosyl-(1→3)-2-*O*-benzoyl-4,6-*O*-benzylidéne-β-D-glucopyranosyl-(1→3)-2-*O*-benzoyl-4,6-*O*-benzyldène-β-D-glucopyranosyl-(1→3)-4,6-*O*-benzylidène-2-*O*-levulinoyl-β-D- glucopyranoside de benzyle (Produit 9)**

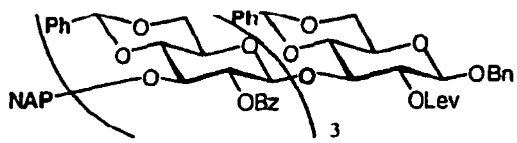

Dans un ballon sont introduits le donneur **1** (26 mg, 0,047 mmol) et l'accepteur trisaccharidique **8** (50 mg, 0,043 mmol) dans le dichlorométhane anhydre à 0 ˚C, en présence de tamis moléculaire 4Å. Après addition de *N*-iodosuccinimide (NIS) (50mg, 0,051 mmol) et de triflate de triméthylsilyle (0,65 μL, 0,004 mmol), le mélange est laissé sous forte agitation pendant 30 minutes. La réaction terminée, le milieu est neutralisé par de la triéthylamine, filtré puis concentré. Après purification par chromatographie sur gel de silice [toluène/acétate d'éthyle (2:1 ; v/v)], 24 mg de tétrasaccharide souhaité **9** sont obtenus avec un rendement de 34%.

**Produit 9 :** solide blanc; **Pf** (˚C) 138; **Rf** (EP/AcOEt, 2:1) 0,3 ; $[\alpha]_D^{20}$ + 8,4 (c=1,0, $CH_2Cl_2$).

**RMN $^1$H ($CDCl_3$, 400 MHz)** δ (ppm) : 7,94-7,74 (m, 6H, H arom.); 7,58-7,17 (m, 41H, H arom.) ; 5,48 (s, 1H, H7); 5,44 (s, 1H, H7); 5,33 (dd, 1H, H2d, $J_{H1d-H2d}$ = 6,9 Hz, $J_{H2d-H3d}$ = 8,2 Hz) ; 5,21 (t, 1H, H2c, $J_{H1c-H2c}$ = $J_{H2c-H3c}$ = 5,8 Hz) ; 5,06 (d, 1H, H1c); 4,97 (d,1H, H1b, $J_{H1b-H2b}$ = 5,8 Hz) ; 4,96 (d, 1H, H1d); 4,96 (s, 1H, H7) ; 4,90 (d, 1H, H8d, $J_{H8d-H8'd}$ = 12,4 Hz) ; 4,82 (t, 1H, H2b, $J_{H2b-H3b}$ = 5,8 Hz) ; 4,80 (d, 1H, H8'd) ; 4,79 (d, 1H, H8a, $J_{H8a-H8'a}$ =12,4 Hz) ; 4,68 (dd, 1H, H2a, $J_{H1a-H2a}$ = 8,0 Hz, $J_{H2a-H3a}$ = 8,6 Hz) ; 4,63 (s,1H, H7) ; 4,51 (d, 1H, H8'a) ; 4,35 (d, 1H, H1a); 4,31 (dd, 1H, H6a, $J_{H5a-H6a}$ = 4,9 Hz, $J_{H6a-H6'a}$ = 10,6 Hz) ; 4,21 (dd, 1H, H6d, $J_{H5d-H6d}$ = 4,9 Hz, $J_{H6d-H6'd}$ = 10,4 Hz) ; 4,16 (dd, 1H, H6c, $J_{H5c-H6c}$ = 5,6 Hz, $J_{H6c-H6'c}$ = 10,4 Hz) ; 4,11 (dd, 1H, H6b, $J_{H5b-H6b}$ = 5,3 Hz, $J_{H6b-H6'b}$ = 9,1 Hz) ; 4,08 (dd, 1H, H3c, $J_{H3c-H4c}$ = 8,8 Hz) ; 4,01 (dd, 1H, H3b, $J_{H3b-H4b}$ = 8,4 Hz) ; 3,98 (t, 1H, H4c, $J_{H4c-H5c}$ = 8,8 Hz) ; 3,90 (dd, 1H, H4d, $J_{H3d-H4d}$ = 8,2 Hz, $J_{H4d-H5d}$ = 9,1 Hz) ; 3,90 (dd, 1H, H3a, $J_{H3a-H4a}$ = 9,1 Hz) ; 3,82 (t, 1H, H3d) ; 3,73 (t, 1H, H6'd, $J_{H5d-H6'd}$ = 10,4 Hz); 3,67 (t, 1H, H6'a, $J_{H5a-H6'a}$ = 10,6 Hz) ; 3,62-3,58 (m, 2H, H5c, H6'c) ; 3,61 (dd, 1H, H4b, $J_{H4b-H5b}$ = 9,1 Hz) ; 3,50 (t, 1H, H6'b, $J_{H5b-H6'b}$ = 9,1 Hz) ; 3,48 (dt, 1H, H5b); 3,44 (ddd, 1H, H5d); 3,34 (ddd, 1H, H5a, $J_{H4a-H5a}$ = 9,1 Hz); 3,16 (t, 1H, H4a); 2,64-2,50 (m, 2H, H10a); 2,36-2,21 (m,2H, H11a); 2,00 (s, 3H, H13a).
**RMN $^{13}$C ($CDCl_3$, 100 MHz)** δ (ppm) : 206,0 (C12a); 171,1 (C9a) ; 164,9, 164,8, 164,5 (3C, OCOPh) ; 137,3, 137,1, 136,9, 135,3 (C quat. arom.); 133,3, 133,2 (C arom.) ; 133,0, 132,8 (C quat. arom.) ; 129,7 (C arom.) ; 129,4 (C quat. arom.) ;129,4, 128,9, 128,8, 128,5, 128,4, 128,3, 128,2, 128,0, 127,9, 127,8, 127,7, 127,5, 126,6, 126,4, 126,2, 126,0, 125,9, 125,7, 125,6 (C arom.) ; 102,0 (C7) ; 101,1 (C7) ; 100,8 (2C, C7) ; 99,7 (C1a); 99,3 (C1d); 97,8 (C1b); 96,8 (C1c); 81,1 (C4d) ; 78,8 (C4a) ; 78,5 (C4c) ; 78,1 (C3d) ; 77,4 (C4b) ; 77,2 (C3c); 74,3 (2C, C3a, C3b) ; 73,6 (2C, C8d, C2a) ; 73,4 (C2d) ; 73,2 (C2b) ; 72,8 (C2c); 70,5 (C8a) ; 68,7 (3C, C6b, C6c, C6d) ; 68,5 (C6a); 66,3 (C5a); 65,9 (C5d) ; 65,7 (C5c): 65,3 (C5b); 37,7 (C11a); 29,5 (C13a); 27,6 (C10a).

**Analyse élémentaire** ($C_{96}H_{90}O_{26}$) : Théorique: C = 69,47 %, H = 5,47 % ; Mesurée : C = 69,57 %, H = 5,58 %.
**HRMS (ESI+):** $[M+Na]^+$ $C_{96}H_{90}NaO_{26}$: m/z théorique: 1681,5618, m/z mesurée : 1681,5619 ; $[M+K]^+$ $C_{96}H_{90}KO_{26}$: m/z théorique: 1697,5357, m/z mesurée: 1697,5398.

**2.3/ 4,6-*O*-benzylidéne-3-*O*-(2-méthylnaphtyl)-β-D- glucopyranoside de benzyle (Produit 10)**

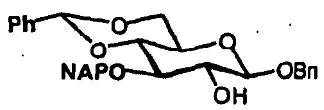

A une solution de **3** (5 g, 8,296 mmol) dans le méthanol anhydre (150 mL) est ajouté le sodium (0,7 g, 30,435 mmol). Le mélange est agité à 50 °C jusqu'à complète consommation du produit de départ. Le milieu est alors refroidi, neutralisé par addition d'une quantité stoechiométrique d'acide acétique et concentré. Le résidu est ensuite dissous dans le dichlorométhane (250 mL), extrait 3 fois par 50 mL d'eau. La phase organique est séchée sur $MgSO_4$ puis concentrée. La purification sur gel de silice [éther de pétrole/acétate d'éthyle (3:1 ; v/v)] permet d'obtenir 3,7 g du composé débenzoylé **10** avec un rendement de 83%.

**Produit 10 :** solide blanc ; **Pf** (°C) 148 ; **Rf** (EP/AcOEt, 3:1) 0,7 ; $[\alpha]_D^{20}$ - 48,1 (c=1,0, $CH_2Cl_2$).
**RMN $^1$H ($CDCl_3$, 400 MHz)** δ (ppm) : 7,96-7,80 (m, 2H, H arom.) ; 7,78-7,72 (m, 1H, H arom.) ; 7,52-7,30 (m, 14H, H arom.); 5,60 (s, 1H, H7) ; 5,10 (d, 1H, H9, $J_{H9-H9'}$ = 12,0 Hz) ; 4,98 (d, 1H, H9') ; 4,94 (d, 1H, H8, $J_{H8-H8'}$ = 11,8 Hz) ; 4,65 (d, 1H, H8') ; 2,46 (s, 1H, OH) et tableau 1a.
**RMN $^{13}$C ($CDCl_3$, 100 MHz)** δ (ppm): 137,2, 136,8, 135,7, 133,2, 133,0 (5C, C quat. arom.); 129,0, 128,5, 128,3, 128,2, 128,1, 128,0, 127,9, 127,6, 126,8, 126,2, 126,1, 126,0, 125,9, 125,8, 125,4, 125,1 (C arom.); 101,4 (C7) ; 74,6 (C9); 71,4 (C8) et tableau 1b.
**Analyse élémentaire** ($C_{31}H_{30}O_6$): Théorique : C = 74,68 %, H = 6,07 % ; Mesurée : C = 74,76 %, H = 6,04 %.
**HRMS (ESI+):** $[M+Na]^+$ $C_{31}H_{30}NaO_6$: m/z théorique : 521,1940, m/z mesurée : 521,1948; $[M+K]^+$ $C_{31}H_{30}KO_6$: m/z théorique: 537,1679, m/z mesurée : 521,1999.

**2.4/ 2-*O*-benzoyl-4,6-*O*-benzylidène-3-*O*-(2-méthylnaphtyl)-β-D-glucopyranosyl-(1→3)-4,6-*O*-benzylidéne-β-D-glucopyranoside de benzyle (Produit 11)**

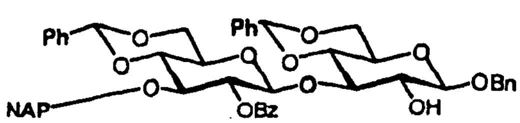

200 μL d'une solution 1M d'hydrazine dans un mélange pyridine/acide acétique (3:2, v/v) sont additionnés goutte à goutte à une solution du disaccharide **5** (11 mg, 0,011 mmol) dans la pyridine (0,2 mL). Après 1 heure d'agitation, la réaction est stoppée par ajout de 50 μL de 2,4-pentane-dione et le milieu est concentré sous pression réduite. Le résidu est ensuite repris par 20 mL de dichlorométhane et lavé par une solution d'acide chlorhydrique à 10%, par une solution aqueuse saturée d'hydrogénocarbonate de sodium et par une solution aqueuse saturée de chlorure de sodium. Après séchage de la phase organique sur $MgSO_4$ et évaporation du solvant, le produit est purifié par chromatographie sur gel de silice [éther de pétrole/acétate d'éthyle (2:1 ; v/v)] pour conduire à 6 mg du composé **11** avec un rendement de 61%.
**Produit 10 :** solide blanc ; **Rf** (EP/AcOEt, 2:1) 0,6.
**RMN $^1$H ($CDCl_3$, 400 MHz)** δ (ppm) : 7,93-7,89 (m, 2H, H arom.) ; 7,70-7,23 (m, 25H, H arom.) ; 5,54 (s, 1H, H7b) ; 5,45 (s, 1H, H7a) ; 4,95 (d, 1H, H8b, $J_{H8b-H8'b}$ = 12,9 Hz) ; 4,86 (d, 1H, H8'b) ; 4,85 (d, 1H, H8a, $J_{H8a-H8'a}$ = 11,7 Hz) ; 4,56 (d, 1H, H8'a) et tableaux 2a et 2b.
**RMN $^{13}$C ($CDCl_3$, 100 MHz)** δ (ppm) : 165,5 (OCOPh) ; 137,2, 137,1, 136,7, 135,2, 133,1, 132,8 (7C, C quat. arom.); 133,0, 129,8, 129,7, 129,1, 129,0, 128,5, 128,4, 128,2, 128,0, 127,9, 127,8, 127,6, 126,9, 126,1, 126,0, 125,8, 125,7

(C arom.) ; 102,0 (C7b) ; 101,3 (C7a) ; 74,0 (C8b) ; 71,3 (C8a) et tableau 2c.
**HRMS (ESI$^+$) :** $[M+Na]^+$ $C_{51}H_{48}NaO_{12}$ : m/z théorique : 875,3044, m/z mesurée : 875,3040 ; $[M+K]^+$ $C_{51}H_{48}KO_{12}$: m/z théorique : 891,2783, m/z mesurée : 891,2748

**2.5/ 4,6-*O*-benzylidéne-3-*O*-(2-méthylnaphtyl)-β-D-glucopyranos-2-uloside de benzyle (13a) 4,6-*O*-benzylidène-2-*C*-hydroxyl-3-*O*-(2-méthylnaphtyl)-β-D- glucopyranoside de benzyle (13b)**

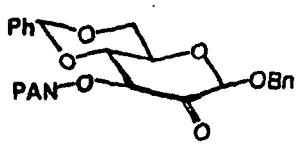
+
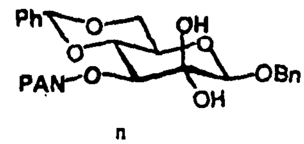

Le composé **10** (43,5 mg, 0,087 mmol) est mis en solution dans 1 mL d'un mélange DMSO/anhydride acétique (2:1; v/v). Après 20 heures d'agitation à température ambiante, le milieu est repris par 50 mL d'acétate d'éthyle et lavé par une solution aqueuse saturée d'hydrogénocarbonate de sodium et par une solution aqueuse saturée de chlorure de sodium. Après séchage ($MgSO_4$) et concentration de la phase organique, le composé est purifié par chromatographie sur gel de silice [éther de pétrole/acétate d'éthyle (3:1 ; v/v)] pour conduire à l'obtention de 33 mg d'un mélange 5:1 du composé recherché **13a** et du composé hydraté **13b,** avec un rendement de 76%.
Solide blanc ; **Rf** (EP/AcOEt, 3:1) 0,3.
**Produit 13a :**
**RMN $^1$H ($CDCl_3$, 400 MHz)** δ (ppm) : 7,75-7,66 (m, 3H, H arom.) ; 7,57-7,54 (m, 1H, H arom.) ; 7,43-7,20 (m, 13H, H arom.) ; 5,49 (s, 1H, H7) ; 5,03 (d, 1H, H9, $J_{H9-H9'}$= 12,7 Hz) ; 4,84 (d, 1H, H8, $J_{H8-H8'}$ = 12,0 Hz) ; 4,80 (d, 1H, H9') ; 4,72 (s, 1H, H1); 4,62 (d, 1H, H8') ; 4,35 (dd, 1H, H6, $J_{H5-H6}$ = 4,8 Hz, $J_{H5-H6'}$ = 10,4 Hz) ; 4,13 (d, 1H, $H_3$, $J_{H3-H4}$ = 10,2 Hz) ; 3,90 (t, 1H, H4, $J_{H4-H5}$ = 10,2 Hz) ; 3,77 (t, 1H, H6', $J_{H5-H6'}$ = 10,4 Hz) ; 3,58 (ddd, 1H, H5).
**RMN $^{13}$C ($CDCl_3$, 100 MHz)** δ (ppm) : 196,7 (C2) ; 136,8, 135,9, 134,6, 133,1, 133,0 (5C, C quat. arom.); 129,2, 128,6, 128,3, 128,2, 128,1, 127,9, 127,6, 127,1, 126,7, 126,1, 126,0, 125,9, 125,7 (C arom.) ; 101,2 (C7) ; 99,0 (C1) ; 82,0 (C4) ; 81,8 (C3) ; 73,1 (C9) ; 70,5 (C8) ; 68,5 (C6) ; 66,4 (C5).
**Produit 13b :**
**RMN $^1$H ($CDCl_3$, 400 MHz)** δ (ppm) : 7,75-7,66 (m, 3H, H arom.) ; 7,63-7,59 (m, 1H, H arom.) ; 7,43-7,20 (m, 13H, H arom.) ; 5,49 (s, 1H, H7) ; 4,97 (d, 1H, H9, $J_{H9-H9'}$ = 11,9 Hz) ; 4,94 (d, 1H, H9') ; 4,84 (d, 1H, H8, $J_{H8-H8'}$ = 11,7 Hz) ; 4,58 (d, 1H, H8'); 4,38 (s, 1H, H1); 4,28 (dd, 1H, H6, $J_{H5-H6}$ = 4,8 Hz, $J_{H6-H6'}$ = 10,4 Hz) ; 3,84 (t, 1H, H4, $J_{H3-H4}$ = $J_{H4-H5}$ = 9,9 Hz) ; 3,80 (dd, 1H, H6', $J_{H5-H6'}$ = 9,7 Hz) ; 3,62 (d, 1H, H3) ; 3,34 (ddd, 1H, H5); 1,97, 1,95 (2s, 2H, OH2).
**RMN $^{13}$C ($CDCl_3$, 100 MHz)** δ (ppm) : 136,9, 136,1, 134,8, 133,1, 133,0 (5C, C quat. arom.) ; 129,0, 128,6, 128,3, 128,2, 128,1, 127,9, 127,6, 127,1, 126,7, 126,2, 126,0, 125,9, 125,7 (C arom.) ; 101,4 (C7) ; 100,5 (C1) ; 93,9 (C2) ; 79,9 (C4) ; 79,8 (C3) ; 74,9 (C9) ; 71,5 (C8) ; 68,5 (C6) ; 66,5 (C5).

**2.6/ 2-*O*-benzoyl-4,6-*O*-benzylidéne-3-*O*-(2-méthylnaphtyl)-β-D-glucopyranosyl-(1→3)-4,6-*O*-benzylidène-β-D-glucopyranos-2-uloside de benzyle (Produit 14a) 2-*O*-benzoyl-4,6-*O*-benzylidéne-3-*O*-(2-méthylnaphtyl)-β-D-glucopyranosyl-(1→3)-4,6-*O*-benzylidène-2-C-hydroxyl-β-D- glucopyranoside de benzyle (Produit 14b)**

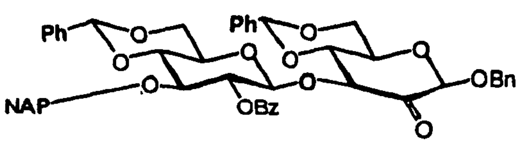
+
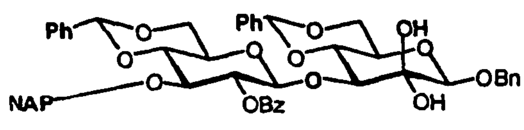

Le composé **11** (6 mg, 0,007 mmol) est mis en solution dans 1 mL d'un mélange DMSO/anhydride acétique (2:1 ; v/v). Après 20 heures d'agitation à température ambiante, le milieu est repris par 50 mL d'acétate d'éthyle et lavé par une solution aqueuse saturée d'hydrogénocarbonate de sodium et par une solution aqueuse saturée de chlorure de sodium. Après séchage ($MgSO_4$) et concentration de la phase organique, le composé est purifié par chromatographie sur gel de silice [éther de pétrole/acétate d'éthyle (2:1 ; v/v)] pour conduire à l'obtention de 6 mg d'un mélange 1:5 du

composé recherché **14a** et du composé hydraté **14b,** avec un rendement quantitatif.
Solide blanc ; **Rf** (EP/AcOEt, 2:1) 0,3.

**Produit 14a :**

**RMN $^{13}$C ($CDCl_3$, 100 MHz)** δ (ppm): Signal caractéristique à 192,2 (C2a).
**HRMS (ESI$^+$):** $[M+Na]^+$ $C_{51}H_{46}NaO_{12}$ : m/z théorique : 873,2887 ; m/z mesurée : 873,2886 ; $[M'+Na]^+$ $C_{51}H_{48}NaO_{13}$ : m/z théorique : 891,2993, m/z mesurée: 891,2982.

**Produit 14b :**

**RMN $^1$H ($CDCl_3$, 400 MHz)** δ (ppm) : 7,92-7,88 (m, 2H, H arom.) ; 7,73-7,22 (m, 25H, H arom.) ; 5,56 (s, 1H, H7) ; 5,50 (s,1H, H7); 5,38 (t, 1H, H2b, $J_{H1b-H2b}$ = $J_{H2b-H3b}$ = 8,0 Hz); 4,96 (d, 1H, H8b, $J_{H8b-H8'b}$ = 12,4 Hz) ; 4,90 (d, 1H, H8a, $J_{H8a-H8'a}$ = 12,0 $H_z$) ; 4,89 (d, 1H, H8'b) ; 4,86 (d, 1H, H1b) ; 4,58 (d, 1H, H8'a) ; 4,34 (dd, 1H, H6b, $J_{H5b-H6b}$ = 4,9 Hz, $J_{H6b-H6'b}$ = 10,6 Hz) ; 4,30 (s, 1H, H1a) ; 4,27 (dd, 1H, H6a, $J_{H5a-H6a}$ = 5,1 Hz, $J_{H6a-H6'a}$ = 10,6 Hz) ; 3,99-3,76 (m, 5H, H4a, H6'a, H3b, H4b, H6'b) ; 3,75 (d, 1H, H3a, $J_{H3a-H4a}$ = 9,5 Hz) ; 3,48 (dt, 1H, H5b, $J_{H4b-H5b}$ = $J_{H5b-H6'b}$ = 9,5 Hz) ; 3,36 (dt, 1H, H5a, $J_{H4a-H5a}$ = $J_{H5a-H6'a}$ = 10,0 Hz).
**RMN $^{13}$C ($CDCl_3$, 100 MHz)** δ (ppm) : 166,3 (OCOPh); 137,2, 137,1, 136,1, 135,1, 132,9 (C quat. arom.); 133,3, 133,0, 129,9, 129,5, 129,1, 129,0, 128,6, 128,5, 128,4, 128,3, 128,2, 128,1, 128,0, 127,9, 127,6, 127,0, 126,3, 126,2, 126,1, 125,9, 125,8 (C arom.) ; 103,0 (C1b) ; 101,2, 101,0 (C7) ; 100,4 (C1a) ; 93,4 (C2a) ; 81,9 (C3a) ; 81,0 (C4b) ; 78,1 (C4a) ; 77,6 (C3b) ; 74,4 (C2b) ; 73,9 (C8b) ; 71,4 (C8a) ; 68,6 (C6a) ; 68,4 (C6b) ; 66,8 (C5a) ; 65,9 (C5b).

**2.7/ 4,6-*O*-benzylidéne-3-*O*-(2-méthylnaphtyl)-β-D- mannopyranoside de benzyle (Produit 16)**

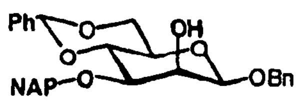

A une solution du mélange **13a/13b** (33 mg, 0,067 mmol) dans le THF (0,5 mL) est additionné, à -80 °C, un équivalent de L-Sélectride (1M dans THF ; 70 μL, 0,070 mmol). Après 15 minutes d'agitation à -80 °C, le milieu est dilué par 10 mL de dichlorométhane et lavé par une solution aqueuse d'acide chlorhydrique à 10%, par une solution aqueuse saturée d'hydrogénocarbonate de sodium et par une solution aqueuse saturée **de** chlorure de sodium. Après séchage sur $MgSO_4$ et évaporation du solvant, le composé est purifié par chromatographie sur gel de silice [éther de pétrole/acétate d'éthyle (3:1 ; v/v)] pour conduire à l'obtention de 28 mg du composé mannosidique recherché **16** avec un rendement de 85%.

**Produit 16 :** solide blanc ; **Rf** (EP/AcOEt, 3:1) 0,3.
**RMN $^1$H ($CDCl_3$, 400 MHz)** δ (ppm) : 7,85-7,79 (m, 3H, H arom.) ; 7,73-7,71 (m, 1H, H arom.) ; 7,55-7,30 (m, 13H, H arom.) ; 5,65 (s, 1H, H7) ; 4,99 (d, 1H, H9, $J_{H9-H9'}$ = 12,7 Hz) ; 4,96 (d, 1H, H9') ; 4,94 (d, 1H, H8, $J_{H8-H8'}$ = 12,0 Hz) ; 4,65 (d, 1H, H8') ; 4,52 (d, 1H, H1, $J_{H1-H2}$ = 1,0 Hz) ; 4,37 (dd, 1H, H6, $J_{H5-H6}$ = 5,1 Hz, $J_{H6-H6'}$ = 10,4 Hz) ; 4,21 (t, 1H, H4, $J_{H3-H4}$ = $J_{H4-H5}$ = 9,4 Hz) ; 4,14 (dd, 1H, H2, $J_{H2-H3}$ = 3,3 Hz) ; 3,94 (t, 111, 116', $J_{H5-H6'}$= 10,4 Hz) ; 3,66 (dd, 1H, H3) ; 3,33 (ddd, 1H, H5).
**RMN $^{13}$C ($CDCl_3$, 100 MHz)** δ (ppm) 137,4, 136,5, 135,3, 133,1, 133,0 (5C, C quat. arom.); 129,2, 129,0, 128,6, 128,5, 128,3, 128,2, 128,1, 127,9, 127,6, 126,7, 126,2, 126,1, 125,9, 125,7 (C arom.); 101,6 (C7); 98,6 (C1; $^1J_{C1-H1}$ =158 Hz); 78,4 (C4); 76,4 (C3) ; 72,4 (C9) ; 70,7 (C8) ; 70,0 (C2) ; 68,6 (C6) ; 66,9 (C5).

**2.8/ 2-*O*-benzoyl-4,6-*O*-benzylidène-3-*O*-(2-méthylnaphtyl)-β-D-glucopyranosyl-(1→3)-4,6-*O*-benzylidène-β-D-mannpyranoside de benzyle (Produit 17)**

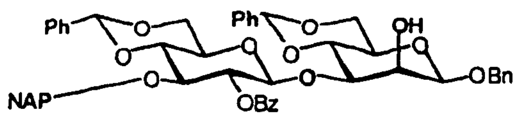

Le composé **14a/14b** (5 mg, 0,006 mmol) en solution dans 1 mL de THF est additionné à -90 °C de L-Sélectride (solution 1M dans THF ; 6 µL, 0,006 mmol). Après 1 heure d'agitation à basse température, le milieu est neutralisé par de l'acide acétique, dilué par 20 mL de dichlorométhane et lavé par une solution aqueuse à 10% d'acide chlorhydrique, par une solution saturée d'hydrogénocarbonate de sodium et par une solution aqueuse saturée de chlorure de sodium. Après séchage ($MgSO_4$) et concentration de la phase organique, 5 mg du composé recherché **17** (0,006 mmol) sont obtenus avec un rendement quantitatif.

**Produit 17 :** solide blanc ; **Rf** (EP/AcOEt, 2:1) 0,3.
**RMN $^1$H ($CDCl_3$, 400 MHz)** δ (ppm) : 7,90-7,20 (m, 20H, H arom.) ; 5,55 (s, 1H, H7); 5,48 (s, 1H, H7) ; 5,35 (t, 1H, H2b, $J_{H1b-H2b}$ = $J_{H2b-H3b}$ = 7,5 Hz) ; 4,94 (d, 1H, H8b, $J_{H8b-H8'b}$ = 12,2 Hz); 4,86 (d, 1H, H8'b) ; 4,81 (d, 1H, H8a, $J_{H8a-H8'a}$ =11,9 Hz) ; 4,79 (d, 1H, H1b); 4,56 (d, 1H, H8'a) ; 4,37 (d, 1H, H1a, $J_{H1a-H2}$ = 0,9 Hz) ; 4,35-4,17 (m, 2H, H6a, H6b); 4,07 (t, 1H, H4b, $J_{H3b-H4b}$ = $J_{H4b-H5b}$ = 9,3 Hz) ; 3,94 (t, 1H, H3a, $J_{H2a-H3a}$ = $J_{H3a-H4a}$ = 9,0 Hz) ; 3,90-3,68 (m, 5H, H2a, H4a, H3b, H6'a, H6'b) ; 3,56-3,24 (m, 2H, H5a, H5b).
**RMN $^{13}$C ($CDCl_3$, 100 MHz)** δ (ppm) : 166,3 (OCOPh) ; 137,2, 135,1, 133,2 (C quat. arom.); 129,9, 129,8, 129,7, 129,0, 128,5, 128,4, 128,3, 128,2, 128,1, 128,0, 127,9, 127,8, 127,5, 127,0, 126,3, 126,1, 126,0, 125,9, 125,8, 125,7 (C arom.); 101,2, 101,0 (C7) ; 100,4 (C1b); 98,3 (C1a); 81,8 (C3a) ; 80,9 (C4b) ; 78,4 (C4a) ; 77,7 (C3b) ; 74,0 (C2b); 73,7 (C8b); 70,5 (C8a); 69,4 (C2a); 68,6, 68,5 (C6a, C6b); 67,0 (C5a) ; 66,3 (C5b).

**EXEMPLE 3: Préparation de β-D-glucopyranosyl-(1→3)-β-D-glucopyranosyl-(1→3)-β-D-glucopyranosyl-(1→3)-β-D-glucopyranosyl-(1→3)-β-D-glucopyranosyl-(1→3)-β-D-mannopyranose (Produit A2)**

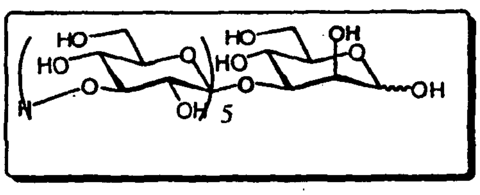

Le produit souhaité a été préparé selon un protocole comparable à celui décrit pour le produit A1 en appliquant au produit 9 les étapes successives [d], [c], [d], [c], puis [e] à [i].
On a ainsi obtenu le produit A2 qui se présente sous la forme d'une mousse blanche; **Rf** (AcOEt/iPrOH/$H_2O$, 3:2:2) 0,2.
**RMN $^1$H ($D_2O$, 400 MHz)** δ (ppm) : 5,19 (d, 1H, H1aα, $J_{H1a-H2a}$ = 2,2 Hz).
Autres signaux :

**RMN $^1$H ($D_2O$, 400 MHz) δ (ppm)** : 4,75 (d, 3H, 3 H1, $J_{H1-H2}$ = 8,0 Hz) ; 4,71 (d, 2H, 2 H1, $J_{H1-H2}$ = 7,8 Hz) ; 3,93-3,81 (m, 8H, H3a, H4a, H6a, H6b, H6c, H6d, H6e, H6f) ; 3,80-3,63 (m, 11H, H2a, H3b, H3c, H3d, H3e, H6'a, H6'b, H6'c, H6'd, H6'e, H6'f) ; 3,55-3,40 (m, 11H, H2b, H2c, H2d, H2e, H3f, H4b, H4c, H4d, H4e, H5a, H5b, H5c, H5d, H5e, H5f) ; 3,36 (t, 1H, H4f, $J_{H3f-H4f}$ = $J_{H4f-H5f}$ = 9,3 Hz) ; 3,31 (dd, 1H, H2f, $J_{H1f-H2f}$ = 8,0 Hz, $J_{H2f-H3f}$ = 9,3 Hz).
**HRMS (ESI$^+$):** $[M+Na]^+$ $C_{36}H_{62}NaO_{31}$ : m/z théorique : 1013,3173, m/z mesurée : 1013,3172; $[M+K]^+$ $C_{36}H_{62}KO_{31}$ : m/z théorique : 1029,2912, m/z mesurée : 1029,2920.

**EXEMPLE 4: Préparation du β-D- glucopyranosyl-(1→3)- β-D- glucopyranosyl-(1→3)-mannitol (produit B1) selon le schéma réactionnel 1**

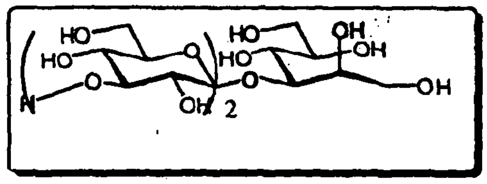

Dans 10 mL d'un mélange méthanol/eau (4:1 ; v/v) est introduit le trisaccharide déprotégé A1 (25 mg, 0,049 mmol). Le borohydrure de sodium (19 mg, mmol) est ajouté à température ambiante et le milieu est laissé sous agitation pendant 10 jours. Le milieu est alors neutralisé par ajout de quelques gouttes d'acide acétique puis mis à sec par coévaporation avec un mélange méthanol/acide acétique (9:1, v/v), puis par coévaporation avec du méthanol. Après lyophilisation, le composé est purifié par chromatographie de perméation de gel Sephadex G-10 (éluant: eau) et les fractions recueillies sont lyophilisées afin d'obtenir le produit recherché B1 qui se présente sous la forme d'une mousse blanche.

RMN $^1H$ ($D_2O$, 400 MHz) δ (ppm) : 4,82 (d, 1H, H1c, $J_{H1c\text{-}H2c}$ =8,0 Hz) ; 4,65 (d, 1H, H1b, $J_{H1b\text{-}H2b}$ = 8,1 Hz) ; 4,09-3,90 (m, 7H, H1a, H2a, H3a, H5a, H6a, H6b, H6c) ; 3,86-3,66 (m, 6H, H1'a, H4a, H6'a, H3b, H6'b, H6'c) ; 3,64-3,38 (m, 7H, H2b, H4b, H5b, H2c, H3c, H4c, H5c).
RMN $^{13}C$ ($D_2O$, 100 MHz) δ (ppm) : 103,1 (C1c) ; 102,5 (C1b) ; 84,4 (C3b) ; 77,3 (C3a) ; 76,3 (C5c) ; 75,8 (C3c) ; 75,5 (C5b) ; 73,7 (C2c) ; 73,4 (C2b) ; 70,9, 70,8 (2C, C2a, C5a); 69,9 (C4c) ; 69,6 (C4a) ; 68,8 (C4h) ; 63,4 (C6a) ; 62,6 (C1a) ; 61,2, 61,0 (2C, C6b, C6c).

**Exemple 5: Préparation du produit A1 selon le schéma réactionnel 2.**

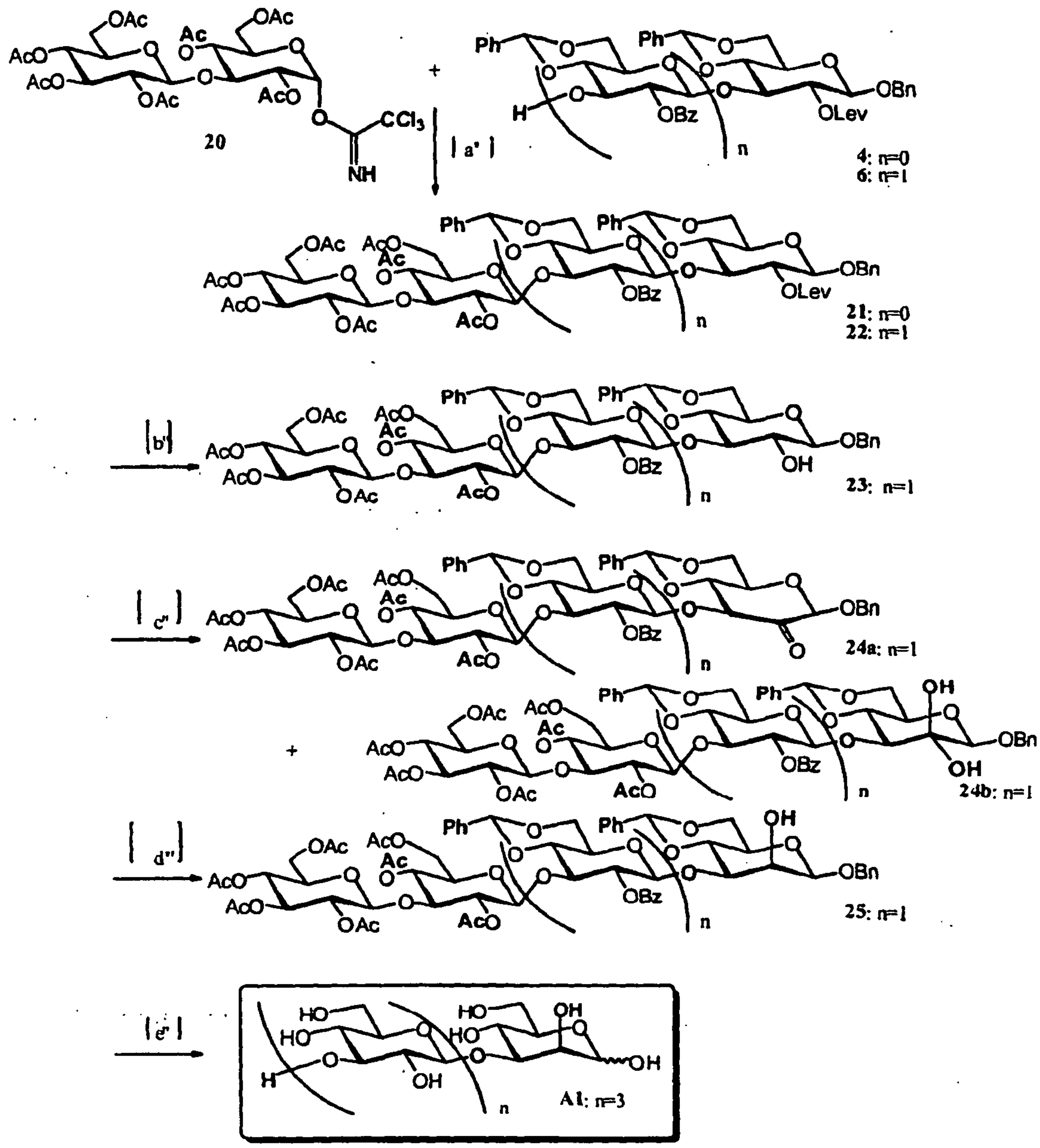


**5.1/ Préparation de 2,3,4,6-tétra-*O*-acétyl-β-D-glucopyranosyl-(1→3)-2,4,6-tri-*O*-acétyl-β-D-glucopyranosyl-(1→3)-4,6-*O*-benzylidène-2-*O*-levulinoyl-β-D- glucopyranoside de benzyle (Produit 21)**

Dans un ballon sont introduits le donneur 20 [Blattner, R.; Furneaux, R. H.; Pakulski, Z. Carbohydr. Res. 2006, 341, 2115-2125.] (50 mg, 0,064 mmol) et l'accepteur 4 (27 mg, 0,058 mmol) dans le dichlorométhane anhydre à -50 °C, en présence de tamis moléculaire 4Å. Le triflate de triméthylsilyle (0,53 μL, mmol) est alors additionné et le mélange est agité vigoureusement pendant 2 heures. La réaction terminée, le milieu est neutralisé par de la triéthylamine, filtré puis concentré. Après purification par chromatographie sur gel de silice [éther de pétrole/acétate d'éthyle (2:1 ; v/v)], 22 mg du trisaccharide attendu 21 sont obtenus avec un rendement de 35%.

**Produit 21 :** solide blanc amorphe ; Rf (EP/AcOEt, 1:1) 0,2.
RMN $^1$H ($CDCl_3$, 400 MHz) δ (ppm) : 7,46-7,43 (m, 2H, H arom.) ; 7,37-7,27 (m, 8H, H arom.) ; 5,53 (s, 1H, H7a) ; 5,10 (t, 1H, H3c, $J_{H2c-H3c}$ = $J_{H3c-H4c}$ = 9,3 Hz) ; 5,04 (t, 1H, H4c, $J_{H4c-H5c}$ = 9,3 Hz) ; 5,02 (t, 1H, H2a, $J_{H1a-H2a}$ = 7,7 Hz, $J_{H2a-H3a}$ = 9,5 Hz) ; 4,95 (dd, 1H, H2b, $J_{H1b-H2b}$ = 8,2 Hz, $J_{H2b-H3b}$ = 6,0 Hz) ; 4,93 (dd, 1H, H4b, $J_{H3b-H4b}$ = 7,3 Hz, $J_{H4b-H5b}$ = 9,7 Hz) ; 4,87 (dd, 1H, H2c, $J_{H1c-H2c}$ = 8,2 Hz) ; 4,86 (d, 1H, H8a, $J_{H8a-H8'a}$ = 12,2 Hz) ; 4,62 (d, 1H, H1c) ; 4,57 (d, 1H, H8'a) ; 4,55 (d, 1H, H1b) ; 4,50 (d, 1H, H1a) ; 4,34 (dd, 1H, H6c, $J_{H5c-H6c}$ = 2,0 Hz, $J_{H6c-H6'c}$ = 12,4 Hz) ; 4,33 (dd, 1H, H6a, $J_{H5a-H6a}$ = 4,6 Hz, $J_{H6a-H6'a}$ = 10,4 Hz) ; 4,15-4,01 (m, 3H, H6b, H6'b,

H6'c) ; 3,93 (t, 1H, H3a, $J_{H3a-H4a}$ = 9,5 Hz) ; 3,91 (dd, 1H, H3b) ; 3,79 (t, 1H, H6'a, $J_{H5a-H6'a}$ = 10,4 Hz) ; 3,69 (t, 1H, H4a, $J_{H4a-H5a}$ = 9,5 Hz) ; 3,69 (ddd, 1H, H5c, $J_{H5c-H6'c}$ = 4,0 Hz) ; 3,54 (ddd, 1H, H5b, $J_{H5b-H6b}$ = 2,4 Hz, $J_{H5b-H6'b}$ = 4,0 Hz) ; 3,40 (ddd, 1H, H5a) ; 2,87-2,80 (m, 1H, H10a) ; 2,70-2,62 (m, 2H, H10'a, H11a) ; 2,50-2,42 (m, 1H, H11'a) ; 2,20 (s, 3H, H13a) ; 2,04, 1,99, 1,97, 1,96, 1,94 (6 s, 21H, $OCOCH_3$).
RMN $^{13}C$ ($CDCl_3$, 100 MHz) δ (ppm) : 206,1 (C12a); 171,0 (C9a) ; 170,8, 170,5, 170,4, 169,3, 169,2, 169,1 (7C, $OCOCH_3$); 137,0, 136,8 (2C, C quat. arom.); 129,0, 128,5, 128,4, 128,3, 128,1, 128,0, 127,9, 127,8, 125,9 (C arom.) ; 100,9 (C7a); 100,7 (C1c) ; 100,1 (C1b) ; 99,8 (C1a) ; 78,7 (C3b) ; 78,6 (C4a) ; 77,7 (C3a) ; 73,8 (C2a) ; 73,0 (C3c) ; 72,6 (C2b) ; 71,5 (C5b) ; 71,4 (C5c) ; 70,9 (C2c) ; 70,7 (C8a) ; 68,5 (C6a) ; 68,1 (C4c) ; 68,0 (C4b); 66,5 (C5a); 62,2 (C6b) ; 61,6 (C6c); 37,6 (C11a) ; 30,1 (C13a) ; 27,6 (C10a) ;21,0, 20,9, 20,7, 20,6, 20,5, 20,4 (7C, $OCOCH_3$).

### 5.2/ Préparation de 2,3,4,6-tétra-*O*-acétyl-β-D-glucopyranosyl-(1→3)-2,4,6-tri-*O*-acétyl-β-D-glucopyranosyl-(1→3)-2-*O*-benzoyl-4,6-*O*-benzylidéne-β-D-glucopyranosyl-(1→3)-4,6-*O*-benzylidène-2-*O*-levulinoyl-β-D-glucopyranoside de benzyle (Produit 22)

Dans un ballon sont introduits le donneur disaccharidique 37 (106 mg, 0,136 mmol) et l'accepteur 71 (100 mg, 0,123 mmol) dans le dichlorométhane anhydre à -50 ˚C, en présence de tamis moléculaire 4Å. Le triflate de triméthylsilyle (1,1 µL, 0,006 mmol) est additionné ct la réaction est agitée pendant 60 minutes. Le milieu est neutralisé par de la triéthylamine, filtré puis concentré. Après purification par chromatographie sur gel de silice [éther de pétrole/acétate d'éthyle (1:1 ; v/v)], 146 mg de l'orthoester tétrasaccharidique sont obtenus avec un rendement de 83%. Le composé ainsi obtenu est directement engagé dans une réaction de réarrangement intramoléculaire en présence de triflate de triméthylsilyle (2,5 µL, 0,014 mmol) dans le dichlorométhane à 0 ˚C. Après 3 heures d'agitation, le milieu est neutralisé par de la triéthylamine, puis concentré. Après purification par chromatographie sur gel de silice [éther de pétrole/acétate d'éthyle (1:1 ; v/v)], 101 mg du produit attendu 22 sont obtenus avec un rendement de 69%.

**Produit 22** : solide blanc ; Pf (˚C) 119 ; Rf(EP/AcOEt, 1:1) 0,3.
RMN $^1H$ ($CDCl_3$, 400 MHz) δ (ppm) : 8,02-7,99 (m, 2H, H arom.) ; 7,61-7,12 (m, 18H, H arom.) ; 5,59 (s, 1H, H7b) ; 4,83 (d, 1H, H8a, $J_{H8a-H8'a}$ = 12,2 Hz) ; 4,57 (s, 1H, H7a) ; 4,56 (d, 1H, H8'a) ; 2,73-2,65 (m, 1H, H10a); 2,63-2,51 (m, 1H, H10'a) ; 2,48-2,40 (m, 1H, H11a); 2,35-2,26 (m, 1H, H11b); 2,08 (s, 3H, H13a); 2,08, 2,04, 2,01, 2,00, 1,97, 1,94 (7s, 21H, $OCOCH_3$) et tableau 4a.
RMN $^{13}C$ ($CDCl_3$, 100 MHz) δ (ppm) : 206,0 (C12a); 171,2 (C9a); 170,8, 170,5, 170,3, 169,4, 169,3, 169,1, 168,7 (6C, $OCOCH_3$); 165,0 (OCOPh); 137,3, 136,9, 136,7 (3C, C quat. arom.); 133,3, 129,8, 129,7 (C arom.); 129,6 (C quat, arom.); 129,5, 128,5, 128,4, 128,3, 128,2, 127,9, 127,8, 126,5, 126,0, 125,8 (C arom.); 102,2 (C7b) ; 99,7 (C7a); 70,7 (C8a); 37,6 (C11a); 29,4 (C13a); 27,7 (C10a); 21,0, 20,9, 20,6, 20,5, 20,4, 20,3 (7C, $OCOCH_3$) et tableau 4b.
HRMS ($ESI^+$): $[M+Na]^+$ $C_{71}H_{80}NaO_{31}$: m/z théorique : 1451,4581, m/z mesurée : 1451,4579 ; $[M+K]^+$ $C_{71}H_{80}KO_{31}$: m/z théorique : 1467,4321, m/z mesurée : 1467,4346.

### 5.3/ 2,3,4,6-tétra-*O*-acétyl-β-D-glucopyranosyl-(1→3)-2,4,6-tri-*O*-acétyl-β-D-glucopyranosyl-(1→3)-2-*O*-benzoyl-4,6-*O*-benzylidène-β-D-glucopyranosyl-(1→3)-4,6-*O*-benzylidéne-β-D-glucopyranoside de benzyle (Produit 23)

Le tétrasaccharide 22 (430 mg, 0,301 mmol) est mis en solution dans 6 mL de pyridine puis est additionné goutte à goutte de 6 mL d'une solution 1M d'hydrazine dans un mélange pyridine/acide acétique (3:2, v/v). Après 3 heures d'agitation, la réaction est stoppée par ajout de 1,7 mL de 2,4-pentane-dione et concentrée sous pression réduite. Le milieu est repris par 50 mL de dichlorométhane et lavé par une solution d'acide chlorhydrique à 10%, par une solution aqueuse saturée d'hydrogénocarbonate de sodium et par une solution aqueuse saturée de chlorure de sodium. Après séchage ($MgSO_4$) et concentration, le produit est purifié par chromatographie sur gel de silice [éther de pétrole/acétate d'éthyle (1:1 ; v/v)] pour conduire à 260 mg du composé déprotégé 23 avec un rendement de 65%.

**Produit 23** : solide blanc ; Pf (˚C) 136 ; Rf (EP/AcOEt, 1:1) 0,3; $[\alpha]_D^{20}$ - 29,6 (c=1,0, $CH_2Cl_2$).
RMN $^1H$ ($CDCl_3$, 400 MHz) δ (ppm): 8,11-8,09 (dd, 2H, H arom.); 7,67-7,17 (m, 18H, H arom.); 5,61 (s, 1H, H7); 5,08 (s, 1H, H7); 4,92 (d, 1H, H8a, $J_{H8a-H8'a}$ = 11,7 Hz); 4,61 (d, 1H, H8'a); 2,09, 2,03, 2,01, 2,00, 1,99 (7s, 21H, $OCOCH_3$) et tableau 4a. RMN $^{13}C$ ($CDCl_3$, 100 MHz) δ (ppm) : 170,8, 170,5, 170,2, 169,3, 169,2, 169,0, 168,6 (7C, $OCOCH_3$); 165,1 (OCOPh); 137,1, 137,0, 136,5 (3C, C quat, arom.); 133,3 (C arom.); 129,5 (C quat. arom.); 129,7, 129,1, 128,9, 128,8, 128,7, 128,5, 128,4, 128,2, 128,1, 128,0, 127,9, 127,8, 126,0, 125,8, 125,2 (C arom.); 101,4 (C7); 100,4 (C7); 71,3 (C8a); 20,6, 20,5, 20,4, 20,3, 20,2 (7C, $OCOCH_3$) et tableau 4b.

**5.4/ Préparation de 2,3,4,6-tétra-*O*-acétyl-β-D-glucopyranosyl-(1→3)-2,4,6-tri-*O*-acétyl-β-D-glucopyranosyl-(1→3)-2-*O*-benzoyl-4,6-*O*-benzylidéne-β-D-glucopyranosyl-(1→3)-4,6-*O*-benzylidène-β-D-glucopyranos-2-uloside de benzyle (Produit 24a) et de 2,3,4,6-tétra-*O*-acétyl-β-D-glucopyranosyl-(1→3)-2,4,6-tri-*O*-acétyl-β-D-glucopyranosyl-(1→3)-2-*O*-benzoyl-4,6-*O*-benzylidène-β-D-glucopyranosyl-(1→3)-4,6-*O*-benzylidéne-2-C-hydroxyl-β-D-glueopyranoside** de **benzyle (Produit 24b)**

A une solution du produit 23 (260 mg, 0,195 mmol) dans 5 mL de dichlorométhane anhydre sont ajoutés la pyridine (0,5 mL) et le périodinane de Dess-Martin (2,6 mL d'une solution à 15%). Après 24 h à température ambiante, le milieu est additionné d'éther éthylique. Le précipité résultant est filtré sur célite et le filtrat concentré sous pression réduite. Le résidu est enfin purifié par chromatographie sur gel de silice [éther de pétrole/acétate d'éthyle (1:1 ; v/v)], pour conduire à 208 mg du produit oxydé 24a (Rdt=80%) en mélange (1:1) avec sa forme hydratée 24b.

24a/24b : solide blanc ; Rf (EP/AcOEt, 1:1) 0,2.
**Produit 24a** : RMN $^1H$ ($CDCl_3$, 400 MHz) δ (ppm) : Signaux caractéristiques 5,25 (s, 1H, H1a).
RMN $^{13}C$ ($CDCl_3$, 100 MHz) δ (ppm) : Signaux caractéristiques 196,6 (C2a) ; 96,8 (C1a); 70,6 (C8a).
**Produit 24b** : RMN $^1H$ ($CDCl_3$, 400 MHz) δ (ppm) : Signaux caractéristiques 4,87 (s, 1H, H1a).
RMN $^{13}C$ ($CDCl_3$, 100 MHz) δ (ppm) : Signaux caractéristiques 98,8 (C1a); 93,7 (C2a); 71,4 (C8a).

Autres signaux (24a/24b) :

RMN $^1H$ ($CDCl_3$, 400 MHz) δ (ppm) : 8,08-8,03 (m, 2H, H arom.); 7,67-7,45 (m, 8H, H arom.); 7,40-7,10 (m, 30H, H arom.); 5,65 (s, 1H, H7) ; 5,59 (s, 1H, H7); 5,26-5,18 (m, 2H, 2 H2b); 5,14-4,80 (m, 14H, H2c, 2 H2d, 2 H3d, 2 H4a, 2 H4b, 2 H8a, 2 H1); 4,73 (d, 1H, H8a, $J_{H8a-H8'a}$ = 11,9 Hz); 4,66-4,48 (m, 6H, H3a, H8'a, 2 H1, H6, H7); 4,42-4,26 (m, 6H, H3a, H1, H7, 3 H6); 4,20-3,40 (m, 28H, 2 H3b, 2 H3c, 2 H4c, 2 H4d, 2 H5a, 2 H5b, 2 H5c, 2 H5d, 2 H6'a, 2 H6'b, 2 H6'c, 2 H6'd, 4 H6); 2,08, 2,06, 2,04, 2,01, 2,00, 1,99, 1,98, 1,97, 1,96, 1,95(14 s, 42H, OCOC$H_3$).
RMN $^{13}C$ ($CDCl_3$, 100 MHz) δ (ppm) : 170,8, 170,5, 170,3, 169,4, 169,3, 169,2, 169,1, 168,8 (10C, O*C*OCH$_3$); 165,3 (2C, O*C*OPh); 137,1, 137,0, 136,6, 136,0, 135,6 (6C, C quat, arom.); 133,7, 133,6, 129,9, 129,4, 129,3, 129,2, 129,1, 128,8, 128,7, 128,6, 128,5, 128,4, 128,3, 128,2, 127,9, 127,7, 126,4, 126,2, 125,9, 125,8 (C arom., 2 C quat. arom.); 101,8, 101,6, 100,4, 100,2 (4C, C7) ; 100,9, 100,8, 100,6, 99,2, 98,8, 96,8 (8C, C1); 80,6, 79,8, 79,3, 79,0, 78,7, 78,1, 77,8, 77,4, 77,2, 77,1 (10C, C3a, C3b, C3c, C4a, C4b); 74,8 (2C, C2b); 73,3, 73,0, 72,8, 72,3 (4C, C2c, C3d); 71,6, 71,5 (4C, C5c, C5d); 71,0, 70,8 (2C, C2d); 68,7, 68,6, 68,4 (4C, C6a, C6b); 68,0, 67,9 (4C, C4c, C4d); 67,0, 66,8 (2C, C5a); 65,6, 65,1 (2C, C5b) ; 62,0 (2C, C6d); 61,6 (2C, C6c); 21,1, 20,7, 20,6, 20,5, 20,4, 20,3 (14C, OCO*C*H$_3$).

**5.5/ 2,3,4,6-tétra-*O*-acétyl-β-D-glucopyranosyl-(1→3)-2,4,6-tri-*O*-acétyl-β-D-glucopyranosyl-(1→3)-2-*O*-benzoyl-4,6-*O*-benzylidène-β-D-glucopyranosyl-(1→3)-4,6-*O*-benzylidène-β-D-mannopyranoside de benzyle (Produit 25)**

Le produit 24a/24b (66 mg, 0,050 mmol) en solution dans un mélange dichlorométhane/THF est additionné à -90°C de L-Sélectride (solution 1 M dans THF ; 150 μL, 0,150 mmol). Après 2 heures d'agitation à basse température, le milieu est neutralisé par ajout d'acide acétique, dilué par 20 mL de dichlorométhane et lavé par une solution aqueuse saturée d'acide chlorhydrique, une solution aqueuse saturée d'hydrogénocarbonate de sodium et par de l'eau. Après séchage sur $MgSO_4$ et concentration, le milieu est purifié par chromatographie sur gel de silice [dichlorométhane/méthanol (98:2 ; v/v)], pour conduire au produit réduit souhaité 25 (66 mg, 0,050 mmol) avec un rendement quantitatif.

**Produit 25** : solide blanc ; Rf ($CH_2Cl_2$/MeOH, 98:2) 0,2.
RMN $^1H$ ($CDCl_3$, 400 MHz) δ (ppm) : 8,08-8,03 (m, 2H, H arom.) ; 7,67-7,10 (m, 18H, H arom.); 5,62 (s, 1H, H7) ; 5,22 (m, 1H, H2b, $J_{H1b-H2b}$ = $J_{H2b-H3b}$ = 5,1 Hz); 5,04 (t, 1H, H3d, $J_{H2d-H3d}$ = $J_{H3d-H4d}$ = 8,0 Hz); 5,03-4,91 (m, 5H, H1b, H2c, H4c, H4d, H7) ;4,90 (d, 1H, H8a, $J_{H8a-H8'a}$ = 11,9 Hz); 4,84 (dd, 1H, H2d, $J_{H1d-H2d}$ = 8,2 Hz); 4,67 (d, 1H, H1c, $J_{H1c-H2c}$ = 8,0 Hz); 4,63 (d, 1H, H8'a); 4,47 (d, 1H, H1a, $J_{H1a-H2a}$ < 1,0 Hz) ; 4,44 (d, 1H, H1d); 4,39-4,31 (m, 2H, H6a, H6c); 4,18-3,94 (m, 8H, H2a, H3b, H4a, H4b, H6b, H6d, H6'c, H6'd); 3,93-3,84 (m, 2H, H3a, H6'a); 3,75 (t, 1H, H3c, $J_{H2c-H3c}$ = $J_{H3c-H4c}$ = 9,3 Hz) ; 3,64 (t, 1H, H6'b, $J_{H5b-H6'b}$ = $J_{H6b-H6'b}$ 10,0 Hz); 3,62-3,52 (m, 3H, H5b, H5c, H5d); 3,34 (dt, 1H, H5a, $J_{H4a-H5a}$ = $J_{H5a-H6'a}$ = 9,8 Hz, $J_{H5a-H6a}$ = 4,6 Hz) ; 2,05, 2,00, 1,97, 1,95, 1,94 (7s, 21 H, OCOC$H_3$).
RMN $^{13}C$ ($CDCl_3$, 100 MHz) δ (ppm) : 170,8, 170,4, 170,3, 169,3, 169,2, 169,1, 168,9 (7C, O*C*OCH$_3$); 165,0 (O*C*OPh); 137,2, 137,1, 136,4, 129,2 (4C, C quat. arom.); 133,6, 129,7, 129,0, 128,8, 128,6, 128,5, 128,4, 128,3, 128,2, 128,0, 127,8, 126,1, 125,8 (C arom.); 101,7, (C7); 100,7 (C1d); 100,5 (C7); 99,7 (C1c); 98,3 (C1a); 97,3 (C1b); 78,7 (C3c); 78,4 (C3b); 77,5 (C4b); 76,3 (C4a); 75,7 (C3a); 74,2 (C2b); 72,9 (C3d); 72,4 (C2c); 71,5 (2C, C5c, C5d);

70,8 (C2d); 70,6 (C7a); 68,7 (C2a); 68,6 (C6b); 68,5 (C6a); 68,1 (C4c); 67,9 (C4d); 67,2 (C5a); 65,9 (C5b); 61,9 (C6d); 61,5 (C6c); 20,7, 20,6, 20;5, 20,4 (7C, $OCOCH_3$).

**5.6/ Préparation du produit A1**

Le composé 25 (60 mg, 0,045 mmol) est dissous dans un mélange dichlorométhane/méthanol (2:1, v/v; 10 mL) puis additionné de 2 équivalents de méthylate de sodium (0,1M dans MeOH, 0,9 mL, 0,090 mmol). Après 6 heures d'agitation à température ambiante, le milieu est neutralisé par ajout de résine Amberlite IR120-$H^+$, filtré puis concentré sous pression réduite. Le résidu ainsi obtenu est ensuite dissous dans 10 mL d'un mélange acétate d'éthyle/méthanol/dichlorométhane (2:2:1 ; v/v/v). Après ajout d'acétate de palladium (60 mg, 0,267 mmol), le milieu est agité vigoureusement à température ambiante sous atmosphère d'hydrogène pendant 7 jours. Après filtration sur célite, la phase hydroorganique est extraite au dichlorométhane, puis est mise à sec par coévaporation azéotropique avec de l'éthanol absolu. Le produit A1 (30 mg) est alors isolé avec un rendement quantitatif sur les trois dernières étapes.

**EXEMPLE 6 :**

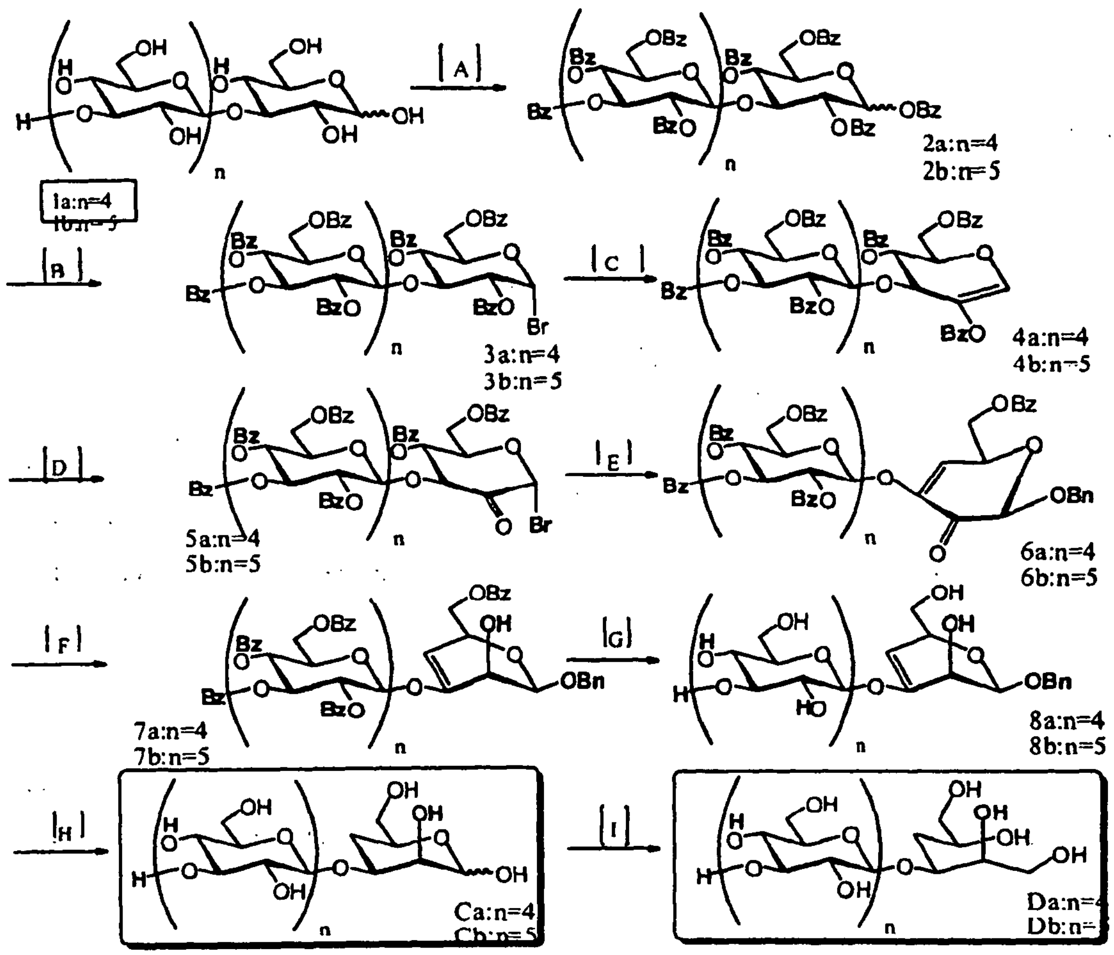

1a:n=4
1b:n=5
2a:n=4
2b:n=5
3a:n=4
3b:n=5
4a:n=4
4b:n=5
5a:n=4
5b:n=5
6a:n=4
6b:n=5
7a:n=4
7b:n=5
8a:n=4
8b:n=5
Ca:n=4
Cb:n=5
Da:n=4
Db:n=5

Le produit 1 a est obtenu comme décrit dans la demande de brevet FR2804684.

**6.1/ Préparation de 2,3,4,6-tétra-*O*-benzoyl-β-D-glucopyranosyl-(1→3)-2,4,6-tri-*O*-benzoyl-β-D-glucopyranosyl-(1→3)-2,4,6-tri-*O*-benzoyl-β-D-glucopyranosyl-(1→3)-2,4,6-tri-*O*-benzoyl-β-D-glucopyranosyl-(1→3)-1,2,4,6-tétra-*O*-benzoyl-(α,β)-D-glucopyranose (Produit 2a)**

L'oligosaccharide 1a (150 mg, 0,181 mmol) est dissous dans 1a pyridine (15 mL). Le milieu réactionnel est alors refroidi à 0 °C puis additionné de chlorure de benzoyle (4,2 mL, 36 mmol). Après 2 jours d'agitation à température

ambiante, le milieu est évaporé, dissous dans du dichlorométhane puis lavé avec une solution aqueuse d'acide chlorhydrique à 10%, avec une solution saturée d'hydrogénocarbonate de sodium et par une solution saturée de chlorure de sodium jusqu'à neutralité. La phase organique est alors séchée sur $MgSO_4$ et évaporée. Le résidu est purifié par chromatographie sur gel de silice [éther de pétrole/acétate d'éthyle (3:2 ; v/v)] pour donner le dérivé perbenzoylé 2a sous 1a forme d'un mélange d'anomères α/β : 1/1 avec un rendement de 94% (444 mg, 0,170 mmol).

Produit 2a : huile incolore ; Rf (EP/AcOEt, 1:1) 0,4.
RMN $^1$H ($CDCl_3$, 400 MHz) 8 (ppm) : Signaux caractéristiques pour 2a□ 6,65 (d, 1H, H1a, $J_{H1a-H2a}$=3,6 Hz) ; 5,47 (t, 1H, H4a, $J_{H3a-H4a}$ = $J_{H4a-HSa}$ = 9,4 Hz) ; 4,64 (t, 1H, H3a, $J_{H2a-H3a}$ = 9,4 Hz). Signaux caractéristiques pour 2a□6,07 (d, 1H, H1a, $J_{H1a-H2a}$ = 7,1 Hz); 5,57 (t, 1H, H4a, $J_{H3a-H4a}$ = $J_{H4a-H5a}$ = 8,9 Hz) ; 4,25 (dt, 1H, H5a, $J_{H5a-H6a}$ = 4,3 Hz, $J_{H5a-H6'a}$ = 8,9 Hz).
RMN $^{13}$C ($CDCl_3$, 100 MHz) δ (ppm) : Signaux caractéristiques pour 2a□ 87,7 (C1a); 76,3 (C3a); 72,4 (C2a) ; 70,3 (C5a); 67,6 (C4a). Signaux caractéristiques pour 2a□ 89,7 (C1a); 78,4 (C3a); 72,4 (2C, C2a, C5a); 67,6 (C4a); 62,0 (C6a).

Autres signaux :

RMN $^1$H ($CDCl_3$, 400 MHz) δ (ppm) : 8,08-7,92 (m, 68H, H arom.); 7,61-7,09 (m, 102H, H arom.); 5,45-5,37 (m, 3H, H2aβ, H3e); 5,26-5,18 (m, 5H, H2aα, H2e, H4e); 5,11-4,83 (m, 14H, H1b, H2b, H4b, H2c, H4c, H2d, H4d); 4,61-4,35 (m, 10H, H3aβ, H5aα, H6aβ, H6'aβ, H1c, H1d, H1e); 4,20-3,86 (m, 26H, H6aα, H6'aα, H3b, H6b, H6'b, H3c, H6c, H6'c, H3d, H6d, H6'd, H5e, H6e, H6'e); 3,80-3,65 (m, 6H, H5b, H5c, H5d).
RMN $^{13}$C ($CDCl_3$, 100 MHz) δ (ppm) : 166,0, 165,9, 165,8, 165,7, 165,6, 165,4, 164,9, 164,8, 164,7, 164,6, 164,5, 164,3, 164,1, 163,8, 163,6, 163,5 (OCOPh); 133,3, 132,7, 130,0, 129,3, 129,2, 129,1, 129,0, 128,9, 128,7 (C quat. arom.); 133,8, 133,6, 133,4, 133,2, 133,1, 133,0, 132,9, 132,8, 132,6, 129,9, 129,7, 129,6, 129,5, 129,4, 128,6, 128,5, 128,4, 128,3, 128,2, 128,1, 128,0, 127,9 (C arom.); 101,4, 101,3, 101,0, 100,8, 100,7 (8C, C1b, C1c, C1d, C1e); 78,1, 77,5, 77,4, 77,2 (6C, C3b, C3c, C3d); 73,4, 73,3, 73,2 (4C, 2 C2); 72,7, 72,6, 72,5, 72,4 (4C, C3e, C2); 71,8, 71,7, 71,6, 71,5 (8C, C5b, C5c, C5d, C5e); 71,3 (2C, C2e) ; 70,3, 70,2 (6C, C4b, C4c, C4d) ; 69,8 (2C, C4c); 63,5, 63,4, 63,1, 62,7 (9C, C6aα, C6b, C6c).

**6.2/ Préparation du bromure de 2,3,4,6-tétra-*O*-benzoyl-β-D-glucopyranosyl-(1→3)-2,4,6-tri-*O*-benzoyl-β-D-glucopyranosyl-(1→3)-2,4,6-tri-*O*-benzoyl-β-D-glucopyrunosyl-(1→3)-2,4,6-tri-*O*-benzoyl-β-D-glucopyranosyl-(1→3)-2,4,6-tri-*O*-benzoyl-α-D-glucopyranosyle (Produit 3a)**

L'oligosaccharide perbenzoylé **2a** (444 mg, 0,170 mmol) est mis en solution dans le dichlorométhane (10 mL), puis additionné, à 0 °C, d'acide bromhydrique à 33% wt dans l'acide acétique (0,6 mL, 3,417 mmol). Après 3,5 heures d'agitation, le milieu est dilué avec du dichlorométhane puis lavé avec une solution aqueuse saturée d'hydrogénocarbonate de sodium. La phase organique est ensuite séchée sur $MgSO_4$ et concentrée sous pression réduite pour conduire de manière quantitative à 435 mg de composé bromé **3a**.

**Produit 3a** : huile incolore.
**RMN $^1$H ($CDCl_3$, 400 MHz)** δ (ppm) : Signaux caractéristiques 6,55 (d, 1H, H1a, $J_{H1a-H2a}$ = 4,1 Hz) ; 5,35 (t, 1H, H4a, $J_{H3a-H4a}$ = $J_{H4a-H5a}$ = 9,9 Hz) ; 4,24 (dd, 1H, H6'a, $J_{H5a\cdot H6'a}$ = 4,6 Hz, $J_{H6-H6'a}$ = 12,4 Hz).
**RMN $^{13}$C ($CDCl_3$, 100 MHz)** δ (ppm) : Signaux caractéristiques 87,7 (C1a); 76,3 (C3a); 73,4 (C2a); 72,6 (C5a); 67,6 (C4a); 62,0 (C6a).

**Autres signaux :**

**RMN $^1$H ($CDCl_3$, 400 MHz)** δ (ppm) : 8,92-7,61 (m, 22H, H arom.) ; 7,48-6,93 (m, 58H, H arom.); 5,27 (t, 1H, H3e, $J_{H2e-H3e}$ = $J_{H3e-H4e}$ = 9,7 Hz) ; 5,09 (dd, 1H, H2e, $J_{H1e-H2e}$ = 7,9 Hz) ; 5,08 (t, 1H, H4e, $J_{H4e-H5e}$ = 9,7 Hz); 4,93-4,86 (m, 3H, 2 H2, H4) ; 4,80-4,70 (m, 4H, H2a, H1, 2 H4); 4,50-4,33 (m, 6H, H3a, H5a, H6a, H1e, 2 H1); 4,08-3,94 (m, 3H, H5e, H3, H6); 3,87-3,70 (m, 9H, 2 H3, 7 H6) ; 3,65-3,53 (m, 3H, H5b, H5c, H5d).
**RMN $^{13}$C ($CDCl_3$, 100 MHz)** δ (ppm) : 165,9, 165,8, 165,7, 165,4, 164,9, 164,8, 164,7, 164,6, 164,5, 164,3, 163,8, 163,5 (16C, OCOPh); 133,8, 133,4, 133,2, 133,1, 133,0, 132,9, 132,8, 132,6, 129,9, 129,7, 129,6, 129,5, 129,4, 129,3, 129,2, 129,0, 128,7, 128,6, 128,5, 128,4, 128,3, 128,2, 128,1, 128,0, 127,9 (C arom.); 101,3, 101,0, 100,8, 100,7 (4C, C1b, C1c, C1d, C1e); 78,4, 78,1, 77,4 (3C, C3b, C3c, C3d); 73,3, 73,2, 72,7 (3C, C2b, C2c, C2d); 72,5 (C3e); 71,8, 71,7, 71,6, 71,5 (4C, C5b, C5c, C5d, C5e); 71,3 (C2e); 70,2 (3C, C4b, C4c, C4d); 69,8 (C4e); 63,4, 63,1 (4C, C6b, C6c, C6d, C6e).

**6.3/ Préparation du 2,3,4,6-tétra-*O*-benzoyl-β-D-glucopyranosyl-(1→3)-2,4,6-tri-*O*-benzoyl-β-D-glucupyranosyl-(1→3)-2,4,6-tri-*O*-benzoyl-β-D-glucopyranosyl-(1→3)-2,4,6-tri-*O*-benzoyl-β-D-glucopyranosyl-(1-3)-2,4,6-tri-*O*-benzoyl-2-D-glu-hex-1-enopyranose (Produit 4a)**

Le brut précédemment obtenu (**3a** : 435 mg, 0,170 mmol) est dissous dans le dichlorométhane (10 mL) puis additionné de 1,8-diazabicyclo[5,4,0]undec-7-ène (40 µL, 0,270 mmol). Après 5,5 heures d'agitation à température ambiante, le milieu est dilué avec du dichlorométhane, lavé avec une solution aqueuse d'acide chlorhydrique à 10%, avec une solution aqueuse saturée d'hydrogénocarbonate de sodium, puis avec une solution aqueuse saturée de chlorure de sodium. La phase organique est séchée ($MgSO_4$), évaporée et le produit recherché est obtenu après purification sur gel de silice [éther de pétrole/acétate d'éthyle (3:2 ; v/v)]. Le composé cible **4a** (343 mg) est alors isolé avec un rendement de 81%.

**Produit 4a** : solide blanc ; **Rf** (EP/AcOEt, 3:2) 0,4.
**RMN $^1$H ($CDCl_3$, 400 MHz)** δ (ppm) : Signaux caractéristiques 6,63 (s, 1H, H1a); 5,77 (d, 1H, H3a, $J_{H3a\text{-}H4a}$ = 3,3 Hz) ; 4,73 (dd, 1H, H6a, $J_{H5a\text{-}H6a}$ = 9,0 Hz, $J_{H6a\text{-}H6'a}$ = 12,6 Hz); 4,40 (dd, 1H, H6'a, $J_{H5a\text{-}H6'a}$ = 3,1 Hz).
**RMN $^{13}$C ($CDCl_3$, 100 MHz)** δ (ppm) : Signaux caractéristiques 138,2 (C1a); 74,1 (C5a) ; 72,3 (C4a) ; 68,7 (C3a) ; 61,6 (C6a).

**Autres signaux :**

**RMN $^1$H ($CDCl_3$, 400 MHz)** δ (ppm) : 8,14-7,70 (m, 26H, H arom.); 7,63-7,03 (m, 54H, H arom.); 5,38 (t, 1H, H3e, $J_{H2e\text{-}H3e}$ **=** $J_{H3e\text{-}H4e}$ = 9,5 Hz); 5,21 (dd, 1H, H2e, $J_{H1e\text{-}H2e}$ = 8,0 Hz); 5,20 (t, 1H, H4e, $J_{H4e\text{-}H5e}$ = 9,5 Hz) ; 5,17 (t, 1H, H2b, $J_{H1b\text{-}H2b}$ = $J_{H2b\text{-}H36}$ = 8,8 Hz) ; 5,12 (t, 1H, H4b, $J_{H3b\text{-}H4b}$ **=** $J_{H4b\text{-}H5b}$ = 10,5 Hz); 5,05-4,97 (m, 2H, H4c, H2d); 4,94 (d, 1H, H1b); 4,94-4,88 (m, 2H, H2c, H4d) ; 4,71 (d, 1H, H1c, $J_{H1c\text{-}H2e}$ = 6,7 Hz) ; 4,59 (d, 1H, H1e, $J_{H1e\text{-}H2e}$ = 8,0 Hz); 4,58-4,53 (m, 3H, H4a, H5a, H1d); 4,46 (dd, 1H, H6b, $J_{H5b\text{-}H6b}$ = 3,1 Hz, $J_{H6b\text{-}H6'b}$ = 12,2 Hz); 4,31 (dd, 1H, H3b); 4,25 (dd, 1H, H6'b, $J_{H5b\text{-}H6'b}$ = 6,6 Hz); 4,15-3,87 (m, 9H, H5b, H3c, H6c, H6'c, H3d, H6d, H6'd, H6e, H6'e); 3,80 (dt, 1H, H5d, $J_{H5d\text{-}H6d}$ = 4,9 Hz, $J_{H5d\text{-}H6'd}$ = 9,3 Hz); 3,76-3,68 (m, 2H, H5c, H5e).
**RMN $^{13}$C ($CDCl_3$, 100 MHz)** δ (ppm) : 165,9, 165,8, 165,7, 165,4, 165,3, 165,1, 165,0, 164,8, 164,7, 164,6, 163,9, 163,7 (16C, OCOPh); 133,3, 133,2, 133,0, 132,9, 132,7, 130,1, 130,0, 129,9, 129,7, 129,6, 129,5, 129,3, 129,2, 129,1, 128,9, 128,7, 128,6, 128,5, 128,4, 128,3, 128,2, 128,1, 128,0, 127,8 (C2a, C arom.); 101,2 (C1b); 100,9 (C1c); 100,8 (C1e); 100,7 (C1d); 78,2, 78,0, 77,7 (3C, C3b, C3c, C3d) ; 73,5 (C2c); 73,2 (C2b); 72,8 (C2d); 72,7 (C5b); 72,6 (C3e); 72,1 (C5d); 71,6 (2C, C5c, C5e); 71,3 (C2e); 70,3 (C4c); 70,0 (C4d); 69,9, 69,8 (2C, C4b, C4e); 63,5, 63,4, 63,2 (3C, C6c, C6d, C6c); 63,1 (C6b).

**6.5/ Préparation du bromure de 2,3,4,6-tétra-*O*-benzoyl-β-D-glucopyranosyl-(1→3)-2,4,6-tri-*O*-benzoyl-β-D-glucopyranosyl-(1→3)-2,4,6-tri-*O*-benzoyl-β-D-glucopyranosyl-(1→3)-2,4,6-tri-*O*-benzoyl-β-D-glucopyranosyl-(1→3)-4,6-di-*O*-benzoyl-α-D-glucopyranos-2-ulosyle (Produit 5a)**

Le composé **5a** précédemment décrit (48 mg, 0,019 mmol) en solution dans le dichlorométhane (0,5 mL) est additionné de méthanol (1,6 µL, 0,040 mmol). Après 10 minutes d'agitation à température ambiante, le *N*-bromosuccinimide (10,8 mg, 0,060 mmol) est ajouté à 0 °C. Une fois la réaction terminée, le milieu est dilué avec du dichlorométhane puis lavé avec une solution aqueuse saturée glacée de thiosulfate de sodium et avec de l'eau glacée. La phase organique est ensuite séchée sur $MgSO_4$ et concentrée sous pression réduite pour conduire à **5a** avec un rendement quantitatif (47 mg, 0,019 mmol)..

**Produit 5a :** huile incolore.
**RMN $^1$H ($CDCl_3$, 400 MHz)** δ (ppm) : Signaux caractéristiques 6,25 (s, 1H, H1a); 5,44 (t, 1H, H4a, $J_{H3a\text{-}H4a}$ = $J_{H4a\text{-}H5a}$ = 10,1 Hz); 5,17 (d, 1H, H3a).
**RMN $^{13}$C ($CDCl_3$, 100 MHz)** δ (ppm) : Signaux caractéristiques 191,5 (C2a) ; 83,8 (C1a); 76,3 (C3a); 72,6 (C5a); 69,0 (C4a); 61,7 (C6a).

**Autres signaux :**

**RMN $^1$H ($CDCl_3$, 400 MHz)** δ (ppm) : 8,06-7,71 (m, 20H, H arom.); 7,61-7,12 (m, 55H, H arom.); 5,39 (t, 1H, H3e, $J_{H2e\text{-}H3e}$ = $J_{H3e\text{-}H4e}$ = 9,5 Hz); 5,22 (dd, 1H, H2e, $J_{H1e\text{-}H2e}$ = 8,0 Hz); 5,21 (t, 1H, H4e, $J_{H4e\text{-}H5e}$ = 9,5 Hz); 5,10 (dd, 1H, H2b, $J_{H1b\text{-}H2b}$ = 8,2 Hz, $J_{H2b\text{-}H3b}$ = 9,3 Hz); 5,06-4,98 (m, 4H, H1b, H4b, H4c, H2d); 4,89 (t, 1H, H4d, $J_{H3d\text{-}H4d}$ = $J_{H4d\text{-}H5d}$ = 9,5 Hz); 4,88 (dd, 1H, H2c, $J_{H1c\text{-}H2c}$ = 7,7 Hz, $J_{H2e\text{-}H3e}$ = 8,6 Hz); 4,74 (d, 1H, H1c, $J_{H1c\text{-}H2c}$ = 8,2 Hz); 4,69 (d, 1H, H1e); 4,66-4,55 (m, 3H, H5a, H6a, H1d); 4,39-4,26 (m, 2H, H3b, H6) ; 4,24-4,10 (m, 3H, 3 H6) ; 4,05-3,86

(m, 8H, H5b, H3c, H3d, 5 H6) ; 3,82-3,70 (m, 3H, H5c, H5d, H5e).

**RMN $^{13}$C ($CDCl_3$, 100 MHz)** δ (ppm) : 167,1, 165,9, 165,8, 165,7, 165,4, 164,9, 164,7, 164,6, 164,3, 164,1, 163,9, 163,6 (15C, OCOPh); 133,6, 133,3, 133,2, 133,1, 133,0, 132,9, 132,8, 132,7, 130,3, 130,0, 129,8, 129,7, 129,6, 129,5, 129,4, 129,3, 129,2, 129,1, 129,0, 128,8, 128,7, 128,6, 128,5, 128,4, 128,3, 128,2, 128,1, 128,0, 127,8, 127,6 (C arom.); 101,0, 100,9, 100,7, 100,6 (4C, C1b, C1c, C1d, C1e); 78,2, 78,0, 77,7 (3C, C3b, C3c, C3d); 73,6, 73,1, 72,8 (3C, C2b, C2c, C2d); 72,6 (C3e); 71,9, 71,6 (4C, C5b, C5c, C5d, C5e); 71,3 (C2e); 70,1, 69,9, 69,8 (4C, C4b, C4c, C4d, C4e); 63,5, 63,4, 63,1 (4C, C6b, C6c, C6d, C6e).

**6.6/ Préparation de 2,3,4,6-tétra-*O*-benzoyl-β-D-glucopyranosyl-(1→3)-2,4,6-tri-*O*-benzoyl-β-D-giucopyranosyl-(1→3)-2,4,6-tri-*O*-benzoyl-β-D-glucopyranosyl-(1→3)-2,4,6-tri-*O*-benzoyl-β-D-glucopyranosyl-(1→3)-6-*O*-benzoyl-4-désoxy-β-D-gluco-hex-3-enopyranos-2-uloside de benzyle (Produit 6a)**

Mode opératoire 1 :

L'alcool benzylique (7 μL, 0,068 mmol) et le bromure d'ulosyle **5a** (82 mg, 0,034 mmol) sont mis en solution dans le dichlorométhane (1,5 mL) en présence de tamis moléculaire. Après 5 minutes d'agitation vigoureuse, l'oxyde de triphénylphosphine (19 mg, 0,068 mmol) est additionné rapidement au mélange. Après 4 jours d'agitation à température ambiante, le milieu est filtré sur célite et concentré sous pression réduite. Le produit recherché **6a** (69 mg ; 0,029 mmol) est obtenu après purification sur gel de silice [éther de pétrole/acétate d'éthyle (3:2 ; v/v)] avec un rendement de 85% en mélange avec sa forme hydratée.

**Produit 6a** : huile incolore ; **Rf** (EP/AcOEt, 1:1) 0,5.

**RMN $^1$H ($CDCl_3$, 400 MHz)** δ (ppm) : Signaux caractéristiques 6,26 (d, 1H, H4a, $J_{H4a-H5a}$ = 3,6 Hz) ; 4,87 (s, 1H, H1a); 4,83 (d, 1H, H7a, $J_{H7a-H7'a}$ = 11,7 Hz); 4,68-4,61 (m, 1H, H5a); 4,60 (d, 1H, H7'a).

**RMN $^{13}$C ($CDCl_3$, 100 MHz)** δ (ppm) : Signaux caractéristiques 183,5 (C2a); 144,9 (C3a); 126,2 (C4a); 97,7 (C1a); 71,0 (C5a); 70,6 (C7a); 66,1 (C6a).

Mode opératoire 2 :

A une solution du donneur **5a** (50 mg, 0,020 mmol) et d'alcool benzylique (4,4 μL, 0,040 mmol) dans le dichlorométhane (0,75 mL) est ajouté à 0 °C le triflate d'argent (5,2 mg, 0,020 mmol). Après 3 heures d'agitation, le milieu réactionnel est neutralisé par ajout de triéthylamine, les sels d'argent sont filtrés sur célite et le filtrat est concentré sous vide. La purification sur gel de silice [éther de pétrole/acétate d'éthyle (3:2 ; v/v)] permet d'obtenir 38 mg (0,016 mmol) du composé désoxy 6a avec un rendement de 75%.

**Autres signaux :**

**RMN $^1$H ($CDCl_3$, 400 MHz)** δ (ppm) : 8,10-7,70 (m, 20H, H arom.); 7,60-7,12 (m, 55H, H arom.) ; 5,39 (t, 1H, H3e, $J_{H2e-H3e}$ = $J_{H3e-H4e}$ = 9,7 Hz); 5,28-5,17 (m, 5H, H1b, H2b, H4b, H2e, H4e); 5,09-5,02 (m, 4H, H2c, H4c, H2d, H4d); 4,95 (d, 1H, H1c, $J_{H1c-H2c}$ = 8,0 Hz); 4,63 (d, 1H, H1d, $J_{H1d-H2d}$ = 7,9 Hz); 4,61 (d, 1H, H1e, $J_{H1e-H2e}$ = 8,0 Hz); 4,53-4,40 (m, 3H, H6a, 2 H6) ; 4,38-4,32 (m, 2H, H3b, H6); 4,25-4,18 (m, 2H, H6'a, H3); 4,15-3,90 (m, 8H, H5b, H3, H5, 5 H6); 3,80-3,70 (m, 2H, H5e, H5).

**RMN $^{13}$C ($CDCl_3$, 100 MHz)** δ (ppm) : 165,8, 165,7, 165,4, 164,9, 164,6, 164,5, 163,9, 163,8 (14C, OCOPh); 136,1 (C quat. arom. $OCH_2$*Ph*); 133,3, 133,2, 133,1, 133,0, 132,9, 132,7, 129,9, 129,8, 129,7, 129,6, 129,5, 129,4, 129,3, 129,2, 129,0, 128,8, 128,6, 128,5, 128,4, 128,3, 128,2, 128,1, 128,0, 127,8 (C arom.); 100,9 (2C, C1d, C1e); 100,1 (C1c); 98,3 (C1b); 78,3, 78,0 (2C, C3c, C3d); 76,8 (C3b) ; 73,7 (2C, C2c, C2d) ; 73,0 (C2b) ; 72,8 (C5b); 72,6 (C3e) ; 72,1 (1C, C5); 71,6 (C2e); 71,3 (2C, C5e, C5) ; 69,9, 69,8 (3C, C4c, C4d, C4e); 69,3 (C4b); 63,4 (2C, C6e, C6); 63,1 (2C, C6b, C6).

**HRMS (ESt$^+$)** : [M+Na]$^+$ $C_{135}H_{11}NaO_{39}$ m/z théorique: 2377,6522
m/z mesurée: 2377,6516

**6.7/ Préparation de 2,3,4,6-tétra-*O*-benzoyl-β-D-glucopyranosyl-(1→3)-2,4,6-tri-*O*-benzoyl-β-D-glucopyranosyl-(1→3)-2,4,6-tri-*O*-benzoyl-β-D-glucopyranosyl-(1→3)-2,4,6-tri-*O*-benzoyl-β-D-glucopyranosyl-(1→3)-**

**6-*O*-benzoyl-4-désoxy-β-D-manno-hex-3-enopyranosyle de benzyle (Produit 7a)**

Le composé 6a (69 mg, 0,029 mmol) est solubilisé dans le THF (0,7 mL) puis additionné à -78 °C de L-Sélectride (solution à 1M dans THF ; 28 μL, 0,028 mmol). Après 90 minutes à -78 °C, la réaction est supposée terminée et neutralisée par ajout de quelques gouttes d'acide acétique. Une fois le milieu revenu à température ambiante, du dichlorométhane est ajouté et la phase organique est lavée par une solution aqueuse d'acide chlorhydrique à 10%, par une solution aqueuse saturée d'hydrogénocarbonate de sodium et par une solution aqueuse saturée de chlorure de sodium. Le composé **7a** souhaité est ainsi obtenu après séchage ($MgSO_4$) et concentration de la phase organique (68 mg ; 0,029 mmol ; 100%).

**Produit 7a** : huile incolore ; **Rf** (EP/AcOEt, 1:1) 0,5.
**RMN $^1$H ($CDCl_3$, 500 MHz)** δ (ppm) : Signaux caractéristiques 4,94 (d, 1H, H4a, $J_{H4a-H5a}$ = 1,1 Hz); 4,86 (d, 1H, H7a, $J_{H7a-H7'a}$ = 12,0 Hz); 4,59 (d, 1H, H7'a); 4,44 (d, 1H, H1a, $J_{H1a-H2a}$ = 2,2 Hz); 4,25-4,20 (m, 2H, H5a, H6a).
**RMN $^{13}$C ($CDCl_3$, 100 MHz)** δ (ppm) : Signaux caractéristiques 151,4 (C3a); 104,1 (C4a); 97, (C1a); 70,4 (C5a); 70,1 (C7a); 66,2 (C6a); 65,1 (C2a).

**Autres signaux :**

**RMN $^1$H ($CDCl_3$, 500 MHz)** δ (ppm) : 8,03-7,70 (m, 20H, H arom.); 7,61-7,13 (m, 55H, H arom.); 5,39 (t, 1H, H3e, $J_{H2e-H3e}$ = $J_{H3e-H4e}$ = 9,6 Hz); 5,30 (t, 1H, H4b, $J_{H3b-H4b}$ = $J_{H4b-H5b}$ = 8,3 Hz); 5,22 (dd, 1H, H2e, $J_{H1e-H2e}$ = 7,9 Hz); 5,20 (t, 1H, H4e, $J_{H4e-H5e}$ = 9,6 Hz); 5,18-5,16 (m, 2H, H1b, H2b); 5,07-4,98 (m, 4H, H2c, H4c, H2d, H4d) ; 4,85 (d, 1H, H1c, $J_{H1c-H2c}$ = 7,9 Hz); 4,62 (d, 2H, H1d, H1e, $J_{H1d-H2d}$ = 7,9 Hz); 4,51 (dd, 1H, H6, $J_{H5-H6}$ = 3,8 Hz, $J_{H6-H6'}$ = 12,3 Hz); 4,36-4,30 (m, 2H, H3b, H6); 4,20-4,14 (m, 2H, H6'a, H3); 4,12-3,90 (m, 10H, H2a, H5b, H3, H5, 6 H6); 3,77-3,70 (m, 2H, H5e, H5).
**RMN $^{13}$C ($CDCl_3$, 100 MHz)** δ (ppm) : 166,1, 165,9, 165,8, 165,7, 165,4, 164,9, 164,7, 164,3, 163,9, 163,8 (14C, OCOPh) ; 136,6 (C quat. arom. $OCH_2$*Ph*); 133,4, 133,2, 133,1, 133,0, 132,9, 132,7, 129,9, 129,7, 129,6, 129,5, 129,4, 129,3, 129,2, 129,0, 128,8, 128,6, 128,5, 128,4, 128,3, 128,2, 128,1, 128,0, 127,8 (C arom.); 100,9 (2C, C1d, C1e); 100,6 (C1c); 97,3 (C1b); 78,3, 77,9 (2C, C3c, C3d); 77,4 (C3b); 73,5 (1C, C2); 72,9 (2C, C2b, C2); 72,6 (C3e); 72,0 (C5b); 71,6 (3C, C5e, 2 C5); 71,3 (C2e); 70,4 (2C, C4c, C4d); 69,8 (C4e); 69,3 (C4b); 63,5, 63,3, 63,1 (4C, C6b, C6c, C6d, C6e).

| | | |
|---|---|---|
| **HRMS (ESI$^+$) :** | $[M+Na]^+$ $C_{135}H_{112}NaO_{39}$ | m/z théorique: 2379,6678<br>m/z mesurée: 2379,6667 |
| | $[M+K]^+$ $C_{135}H_{112}KO_{39}$ | m/z théorique: 2395,6418<br>m/z mesurée: 2395,6542 |

**6.8/ Préparation de β-D-glucopyranosyl-(1→3)-β-D-glucopyranosyl-(1→3)-β-D-glucopyranosyl-(1→3)-β-D-glucopyranosyl-(1→3)-4-désoxy-β-D-manno-hex-3-enopyranosyle de benzyle (Produit 8a)**

Dans un ballon sont introduits successivement le composé **7a** (65 mg, 0,028 mmol) dans un mélange méthanol/dichlorométhane (2:1, v/v) puis le méthylate de sodium (solution à 1M dans THF ; 0,56 mL, 0,056 mmol). Après 6 heures d'agitation à température ambiante, le milieu est neutralisé par ajout de résine Amberlite IR120-$H^+$, filtré puis concentré sous pression réduite. Le composé débenzoylé **8a** est obtenu après chromatographie [dichlorométhane/méthanol (99:1 ; v/v)] avec un rendement quantitatif (25 mg ; 0,028 mmol).

**Produit 8a** : huile incolore.
**RMN $^1$H ($CD_3OD$, 400 MHz)** δ (ppm) : Signaux caractéristiques 5,06 (d, 1H, H4a, $J_{H4a-H5a}$ = 1,5 Hz); 4,82 (d, 1H, H7a, $J_{H7a-H7'a}$ = 12,2 Hz); 4,60 (d, 1H, H7'a); 4,49 (d, 1H, H1a, $J_{H1a-H2a}$ = 1,8 Hz); 4,12-4,08 (m, 1H, H5a); 3,87 (d, 1H, H2a).
**RMN $^{13}$C ($CD_3OD$, 100 MHz)** δ (ppm) : Signaux caractéristiques 153,5 (C3a); 103,8 (C4a); 99,8 (C1a); 71,2 (C7a) ; 67,2 (C2a) ; 65,6 (C6a).

**Autres signaux :**

RMN $^1$H ($CD_3OD$, 400 MHz) δ (ppm) : 7,35-7,16 (m, 5H, H arom.); 4,71 (d, 1H, H1b, $J_{H1b-H2b}$ = 7,5 Hz); 4,54 (d 1H, H1, $J_{H1-N2}$ = 8,0 Hz); 4,53 (d, 1H, H1, $J_{H1-H2}$ = 7,8 Hz); 4,46 (d, 1H, H1e, $J_{H1e-H2e}$ = 7,8 Hz); 3,80-3,73 (m, 4H, 4 H6); 3,60-3,43 (m, 10H, H6a, H6'a, H2b, 3 H3, 4 H6) ; 3,40-3,32 (m, 2H, H2c, H2d) ; 3,30-3,17 (m, 8H, H3e, 3 H4, 4 H5) ; 3,16 (dd, 1H, H2e, $J_{H2e-H3e}$ = 9,3 Hz) ; 3,15 (t, 1H, H4e, $J_{H3e-H4e}$ = $J_{H4e-H5e}$ = 9,8 Hz).
**RMN $^{13}$C ($CD_3OD$, 100 MHz)** δ (ppm) : 138,9 (C quat. arom. $OCH_2$*Ph*) ; 129,6, 129,4, 128,9 (C arom.) ; 105,1, 104,6 (3C, C1c, C1d, C1e) ; 100,5 (C1b) ; 87,4, 87,2, 87,1 (3C, C3b, C3c, C3d) ; 78,1, 77,8, 77,7 (5C, C3e, C5b, C5c, C5d, C5e) ; 75,5 (C2e) ; 75,0, 74,9 (3C, C5a, C2c, C2d) ; 74,0 (C2b) ; 71,5 (C4e) ; 70,0, 69,9 (3C, C4b, C4c, C4d) ; 62,6, 62,5 (4C, C6b, C6c, C6d, C6e).

**6.9/ Préparation de β-D- glucopyranosyl-(1→3)- β-D- glucopyranosyl-(1→3)- β-D- glucopyranosyl-(1→3)- β-D-glucopyranosyl-(1→3)- 4-désoxy-(α,β)-D-mannopyranose (Produit Ca)**

Après dissolution du composé **8a** (24 mg, 0,027 mmol) dans le méthanol, de l'acétate de palladium (25 mg, 0,111 mmol) est additionné au milieu. Après 7 jours d'agitation sous atmosphère d'hydrogène à température ambiante, le milieu est filtré puis concentré sous pression réduite. Le composé **Ca** est obtenu après purification par exclusion stérique Sephadex G-10 (éluant : eau) et lyophilisation des fractions récoltées (21 mg ; 0,027 mmol ; 100%).

**Ca** : mousse blanche ; **Rf** (AcOEt/*i*PrOH/$H_2O$, 3:2:2) 0,2.
**RMN $^1$H ($D_2O$, 400 MHz)** δ (ppm) : Signaux caractéristiques 5,12 (m, 1H, H1aα) ; 1,72-1,60 (m, 2H, H4a, H4'a).
**RMN $^{13}$C ($D_2O$, 100 MHz)** δ (ppm) : Signaux caractéristiques 96,0 (C1a) ; 67,2 (C2a) ; 61,0 (C6a) ; 28,8 (C4a).

**Autres signaux :**

**RMN $^1$H ($D_2O$, 400 MHz)** δ (ppm) : 4,68 (d, 2H, 2 H1, $J_{H1-H2}$ = 8,2 Hz) ; 4,64 (d, 2H, 2 H1, $J_{H1-H2}$ = 8,0 Hz); 3,86-3,73 (m, 5H, H6a, H6b, H6c, H6d, H6e); 3,71-3,56 (m, 10H, H2a, H5a, H6'a, H3b, H6'b, H3c, H6'c, H3d, H6'd, H6'e) ; 3,55-3,33 (m, 13H, H3a, H5a, H2b, H4b, H5b, H2c, H4c, H5c, H2d, H4d, H5d, H3e, H5e) ; 3,31 (t, 1H, H4e, $J_{H3e-H4e}$ = $J_{H4e-H5e}$ = 9,3 Hz) ; 3,24 (dd, 1H, H2e, $J_{H1e-H2e}$ = 8,0 Hz, $J_{H2e-H3e}$ = 9,3 Hz).
**RMN $^{13}$C ($D_2O$, 100 MHz)** δ (ppm) : 103,1, 102,8 (4C, C1b, C1c, C1d, C1e) ; 84,5, 84,4 (4C, C3a, C3b, C3c, C3d) ; 76,3, 75,9, 75,8 (6C, C5a,C5b, C5c, C5d, C3e, C5e) ; 73,8, 73,6 (5C, C2a, C2b, C2c, C2d, C2e) ; 69,9 (C4c) ; 64,4 (3C, C4b, C4c, C4d) ; 61,0 (4C, C6b, C6c, C6d, C6e).

**HRMS (ESI$^+$) :** [M+Na]$^+$ $C_{30}H_{52}NaO_{25}$ m/z théorique: 835,2695
m/z mesurée: 835,2694

**EXEMPLE 7:**

Le produit 1b est préparé selon le mode opératoire décrit dans la demande de brevet FR2804684, selon le mode opératoire de l'exemple 6.

**7.1/ Préparation de 2,3,4,6-tétra-*O*-benzoyl-β-D-glucopyranosyl-(1→3)-2,4,6-tri-*O*-benzoyl-β-D-glucopyranosyl-(1→3)-2,4,6-tri-*O*-benzoyl-β-D-glucopyranosyl-(1→3)-2,4,6-tri-*O*-benzoyl-β-D-glucopyranosyl-(1→3)-2,4,6-tri-*O*-benzoyl-β-D-glucopyranosyl-(1→3)-1,2,4,6-tétra-*O*-benzoyl-(α,β)-D-glucopyranose (Produit 2b)**

L'oligosaccharide **2a** (200 mg, 0,202 mmol) est dissous dans la pyridine (20 mL). Le milieu réactionnel est alors refroidi à 0 °C puis additionné de chlorure de benzoyle (5,2 mL, 44,4 mmol). Après 2 jours d'agitation à température ambiante, le milieu est évaporé, dissous dans du dichlorométhane puis lavé avec une solution aqueuse d'acide chlorhydrique à 10%, avec une solution saturée d'hydrogénocarbonate de sodium et par une solution saturée de chlorure de sodium jusqu'à neutralité. La phase organique est alors séchée sur $MgSO_4$ et évaporée. Le résidu est purifié par chromatographie sur gel de silice [éther de pétrole/acétate d'éthyle (1:1 ; v/v)] pour donner le dérivé perbenzoylé **2a** sous la forme d'un mélange d'anomères α/β :1/1 avec un rendement de 85% (545 mg, 0,178 mmol).

**Produit 2b** : huile incolore ; **Rf** (EP/AcOEt, 1:1) 0,4.
**RMN $^1$H ($CDCl_3$, 400 MHz)** δ (ppm) : Signaux caractéristiques pour **2b**α 6,60 (d, 1H, H1a, $J_{H1a-H2a}$ = 3,8 Hz) ; 5,42 (t, 1H, H4a, $J_{H3a-H4a}$ = $J_{H4a-H5a}$ = 9,8 Hz) ; Signaux caractéristiques pour **2b**β 6,02 (d, 1H, H1a, $J_{H1a-H2a}$ = 7,1 Hz) ; 5,52 (t, 1H, H4a, $J_{H3a-H4a}$ = $J_{H4a-H5a}$ = 9,0 Hz) ; 4,23-4,18 (m, 1H, H5a).
**RMN $^{13}$C ($CDCl_3$, 100 MHz)** δ (ppm) : Signaux caractéristiques pour **2b**α 89,7 (C1a) ; 76,3 (C3a) ; 72,7 (C2a) ;

70,2 (C5a) ; 68,8 (C4a) ; Signaux caractéristiques pour **2b**β 92,0 (C1a) ; 78,3 (C3a) ; 72,9 (C5a) ; 72,5 (C2a) ; 68,8 (C4a) ; 62,7 (C6a).

**Autres signaux :**

**RMN $^1$H ($CDCl_3$, 400 MHz)** δ (ppm) : 8,01-7,65 (m, 60H, H arom.) ; 7,55-7,01 (m, 140H, H arom.) ; 5,36-5,30 (m, 3H, H2aβ, H3f) ; 5,20-5,12 (m, 5H, H2aα, H2f, H4f) ; 5,07-4,89 (m, 8H, 2 H2, 2 H4) ; 4,88-4,72 (m, 10H, H1b, 2 H2, 2 H4) ; 4,61-4,30 (m, 13H, H3aα, H3aβ, H5aα, H6aβ, H6'aβ, H1c, H1d, H1e, H1f); 4,12-3,78 (m, 32H, H6aα, H6'aα, H3b, H6b, H6'b, H3c, H6c, H6'c, H3d, H6d, H6'd, H3e, H6e, H6'e, H5f, H6f, H6'f) ; 3,73-3,56 (m, 8H, H5b, H5c, H5d, H5e).
**RMN $^{13}$C ($CDCl_3$, 100 MHz)** δ (ppm) : 166,0, 165,9, 165,8, 165,7, 165,4, 164,9, 164,7, 164,6, 164,4; 164,3, 164,1, 163,8, 163,6, 163,5 (OCOPh); 133,5, 133,2, 133,1, 133,0, 132,8, 132,6, 130,0, 129,9, 129,7, 129,6, 129,5, 129,4, 129,3, 129,2, 129,1, 129,0, 128,9, 128,7; 128,6, 128,5, 128,4, 128,2, 128,0, 127,9 (C arom.); 101,4, 101,0, 100,9, 100,8, 100,7 (10C, C1b, C1c, C1d, C1e, C1f) ; 78,3, 78,2, 78,1, 77,9 (8C, C3b, C3c, C3d, C3e) ; 73,3, 73,2, 73,1 (6C, 3 C2) ; 72,9 (2C, C2) ; 72,5 (C3f) ; 72,1 (C5f) 71,7, 71,6 (8C, C5b, C5c, C5d, C5e) ; 71,3 (2C, C2f) ; 70,2 (4C, 2 C4) ; 70,0 (4C, 2C4) ; 69,8 (2C, C4f) ; 63,7, 63,4, 63,1 (11C, C6aα, C6b, C6c, C6d, C6e, C6f).

| | | |
|---|---|---|
| **HRMS ($ESI^+$) :** | $[M+Na]^+$ $C_{176}H_{142}NaO_{51}$ | m/z théorique: 3093,8416<br>m/z mesurée: 3093,8418 |
| | $[M+K]^+$ $C_{176}H_{142}KO_{51}$ | m/z théorique: 3109,8155<br>m/z mesurée: 3109,8119 |

**7.2/ Préparation du bromure de 2,3,4,6-tétra-*O*-benzoyl-β-D-glucopyranosyl-(1→3)-2,4,6-tri-*O*-benzoyl-β-D-glucopyranosyl-(1→3)-2,4,6-tri-*O*-benzoyl-β-D-glucopyranosyl-(1→3)-2,4,6-tri-*O*-benzoyl-β-D-glucopyranosyl-(1→3)-2,4,6-tri-*O*-benzoyl-β-D-glucopyranosyl-(1→3)-2,4,6,tri-*O*-benzoyl-α-D- glucopyranosyle (Produit 3b)**

La bromation du composé **2b** (643 mg, 0,209 mmol) est réalisée comme décrit pour **3a** dans le dichlorométhane (15 mL) en présence d'acide bromhydrique (1,1 mL, 6,364 mmol) pour donner de manière quantitative 633 mg de composé bromé **3b**.

**Produit 3b :** huile incolore.
**RMN $^1$H ($CDCl_3$, 400 MHz)** δ (ppm) : Signaux caractéristiques 6,68 (d, 1H, H1a, $J_{H1a-H2a}$ = 3,8 Hz) ; 5,48 (t, 1H, H4a, $J_{H3a-H4a}$ = $J_{H4a-H5a}$ = 9,7 Hz) ; 4,82 (dd, 1H, H2a, $J_{H2a-H3a}$ = 9,7 Hz) ; 4,61 (t, 1H, H3a) ; 4,60 (dd, 1H, H6a, $J_{H5a-H6a}$ = 3,8 Hz, $J_{H6a-H6'a}$ = 12,2 Hz) ; 4,55-4,51 (m, 1H, H5a) ; 4,37 (dd, 1H, H6'a, $J_{H5a-H6'a}$ = 4,2 Hz).
**RMN $^{13}$C ($CDCl_3$, 100 MHz)** δ (ppm) : Signaux caractéristiques 87,7 (C1a) ; 76,3 (C3a) ; 73,4 (C2a) ; 72,7 (C5a) ; 67,6 (C4a) ; 62,0 (C6a).

**Autres signaux :**

**RMN $^1$H ($CDCl_3$, 400 MHz)** δ (ppm) : 8,00-7,71 (m, 26H, H arom.) ; 7,61-7,03 (m, 69H, H arom.) ; 5,39 (t, 1H, H3f, $J_{H2f-H3f}$ = $J_{H3f-H4f}$ = 9,7 Hz) ; 5,22 (dd, 1H, H2f, $J_{H1f-H2f}$ = 7,8 Hz) ; 5,21 (t, 1H, H4f, $J_{H4f-H5f}$ = 9,7 Hz) ; 5,05-4,97 (m, 4H, 3 H2, H4) ; 4,94 (t, 1H, H4, $J_{H4-H5}$ = 9,3 Hz) ; 4,88 (d, 1H, H1, $J_{H1-H2}$ = 8,2 Hz) ; 4,87-4,77 (m, 3H, H2, 2 H4) ; 4,55 (d, 1H, H1f) ; 4,49 (d, 1H, H1, $J_{H1-H2}$ = 7,7 Hz) ; 4,42 (d, 1H, H1, $J_{H1-H2}$ = 7,5 Hz) ; 4,40 (d, 1H, H1, $J_{H1-H2}$ = 7,5 Hz) ; 4,19-4,12 (m, 2H, H3, H6) ; 4,08-3,81 (m, 12H, 3 H3, 9 H6) ; 3,77-3,71 (m, 1H, H5f) ; 3,71-3,60 (m, 4H, H5b, H5c, H5d, H5e).
**RMN $^{13}$C ($CDCl_3$,100 MHz)** δ (ppm) : 165,9, 165,8, 165,7, 165,4, 164,9, 164,8, 164,7, 164,6, 164,5, 164,3, 163,8, 163,5 (19C, OCOPh) ; 133,8, 133,4, 133,2, 133,1, 133,0, 132,8, 132,6, 129,9, 129,7, 129,6, 129,5, 129,4, 129,3, 129,2, 129,1, 129,0, 128,9, 128,7, 128,6, 128,5, 128,4, 128,3, 128,2, 128,1, 128,0, 127,9 (C arom.) ; 101,3, 101,0, 100,8, 100,7 (5C, C1b, C1c, C1d, C1e, C1f) ; 78,4, 78,1, 77,8, 77,4 (4C, C3b, C3c, C3d, C3e) ; 73,3, 73,2, 72,9 (4C, C2b, C2c, C2d, C2e) ; 72,5 (C3f) ; 71,8, 71,6 (5C, C5b, C5c, C5d, C5e, C5f) ; 71,3 (C2f) ; 70,2 (4C, C4b, C4c, C4d, C4e) ; 69,8 (C4f) ; 63,5, 63,4,63,1 (5C, C6b, C6c, C6d, C6e, C6f).

**7.3/ Préparation de 2,3,4,6-tétra-*O*-benzoyl-β-D-glucopyranosyl-(1→3)-2,4,6-tri-*O*-benzoyl-β-D-glucopyranosyl-(1→3)-2,4,6-tri-*O*-benzoyl-β-D-glucopyranosyl-(1→3)-2,4,6-tri-*O*-benzoyl-β-D-glucopyranosyl-(1→3)-**

**2,4,6-tri-*O*-benzoyl-β-D-glucopyranusyl-(1→3)-2,4,6-tri-*O*-benzoyl-2-D-glu-hex-1-enopyranose (Produit 4b)**

Le composé **3b** (550 mg, 0,181 mmol) est engagé dans une réaction d'élimination suivant le même mode opératoire que celui décrit pour **4a** dans le dichlorométhane (10 mL) en présence de DBU (35 μL, 0,235 mmol). La purification sur colonne de gel de silice [éther de pétrole/acétate d'éthyle (3:2 ; v/v)] permet d'obtenir 458 mg du produit souhaité **4b** avec un rendement de 85%.

**4b** : solide blanc ; **Rf** (EP/AcOEt, 1:1) 0,5.
**RMN $^1$H ($CDCl_3$, 400 MHz)** δ (ppm) : Signaux caractéristiques 6,54 (s, 1H, H1a) 5,69 (d, 1H, H3a, $J_{H3a\text{-}H4a}$ = 1,6 Hz) ; 4,64 (dd, 1H, H6a, $J_{H5a\text{-}H6a}$ = 8,8 Hz, $J_{H6a\text{-}H6'a}$ = 12,4 Hz) ; 4,32 (dd, 1H, H6'a, $J_{H5a\text{-}H6'a}$ = 2,4 Hz).
**RMN $^{13}$C ($CDCl_3$, 100 MHz)** δ (ppm) : Signaux caractéristiques 138,2 (C1a) ; 74,1 (C5a) ; 72,3 (C4a) ; 68,6 (C3a) ; 61,6 (C6a).

**Autres signaux :**

**RMN $^1$H ($CDCl_3$, 400 MHz)** δ (ppm) : 7,95-7,62 (m, 25H, H arom.) ; 7,51-6,93 (m, 70H, H arom.) ; 5,29 (t, 1H, H3f, $J_{H2f\text{-}H3f}$ = $J_{H3f\text{-}H4f}$ = 9,7 Hz) ; 5,12 (dd, 1H, H2f, $J_{H1f\text{-}H2f}$ = 8,0 Hz) ; 5,11 (t, 1H, H4f, $J_{H4f\text{-}H5f}$ = 9,7 Hz) ; 5,10-5,12 (m, 1H, H2b, H4b) ; 4,96-4,80 (m, 4H, H1, 2 H2, H4) ; 4,78-4,71 (m, 3H, H2, 2 H4) ; 4,60 (d, 1H, H1, $J_{H1\text{-}H2}$ = 8,0 Hz) ; 4,52-4,44 (m, 3H, H4a, H5a, H1) ; 4,42-4,35 (m, 3H, 2 H1, H6, $J_{H1\text{-}H2}$ = 8,0 Hz) ; 4,07-4,01 (m, 1H, H5b) ; 3,98 (dd, 1H, H6, $J_{H5\text{-}H6}$ = 3,5 Hz, $J_{H6\text{-}H6'}$ = 11,9 Hz) ; 3,93-3,75 (m, 1H, 3 H3c, 8 H6) ; 3,74-3,55 (m, 4H, H5c, H5d, H5e, H5f).
**RMN $^{13}$C ($CDCl_3$, 100 MHz)** δ (ppm) : 165,8, 165,7, 165,4, 165,2, 165,1, 164,9, 164,7, 164,6, 163,8, 163,7, 163,6 (19C, OCOPh) ; 133,3, 133,2, 133,1, 133,0, 132,9, 132,8, 132,7, 132,6, 129,9, 129,8, 129,7, 129,6, 129,5, 129,4, 129,3, 129,2, 129,1, 129,0, 128,8, 128,6, 128,5, 128,4, 128,3, 128,2, 128,1, 128,0, 127,9, 127,7 (C2a, C arom.) ; 101,2 (C1b) ; 100,9, 100,8 100,7 (4C, C1c, C1d, C1e, C1f) ; 78,2, 78,0 (4C, C3b, C3c, C3d, C3e) ; 73,3 (1C, C2) ; 73,1 (C2b) ; 73,0 (C2) ; 72,7 (C5b) ; 72,6 (2C, C3f, C2) ; 72,0, 71,8, 71,6 (4C, C5c, C5d, C5e, C5f); 71,3 (C2f) ; 70,2, 70,1, 69,8, 69,7 (5C, C4b, C4c, C4d, C4e, C4f) ; 63,5, 63,3, 63,1 (5C, C6b, C6c, C6d, C6e, C6f).

| | | |
|---|---|---|
| **HRMS (ESI$^+$) :** | $[M+Na]^+$ $C_{169}H_{136}NaO_{49}$ | m/z théorique: 2971,8048<br>m/z mesurée: 2971,8034 |
| | $[M+K]^+$ $C_{169}H_{136}KO_{49}$ | m/z théorique: 2987,7787<br>m/z mesurée: 2987,7811 |

**7.4/ Préparation de bromure de 2,3,4,6-tétra-*O*-benzoyl-β-D-glucopyranosyl-(1→3)-2,4,6-tri-*O*-benzoyl-β-D-glucopyranosyl-(1→3)-2,4,6-tri-*O*-benzoyl-β-D-glucopyranosyl-(1→3)-2,4,6-tri-*O*-benzoyl-β-D-glucopyranosyl-(1→3)-2,4,6-tri-*O*-benzoyl-β-D-glucopyranosyl-(1→3)-4,6-di-*O*-benzoyl-α-D- glucopyranos- 2-ulosyle (Produit 5b)**

Le composé **5b** a été obtenu suivant le même mode opératoire que celui décrit pour la préparation de **5a** à partir de **4b** (124 mg, 0,042 mmol) dans 1,5 mL de dichlorométhane, de NBS (22,4 mg, 0,126 mmol) et de méthanol (3,4 μL, 0,084 mmol) avec un rendement quantitatif (123 mg, 0,042 mmol).

**Produit 5b :** huile incolore.
**RMN $^1$H ($CDCl_3$, 400 MHz)** δ (ppm) : Signaux caractéristiques 6,25 (s, 1H, H1a) ; 5,44 (t, 1H, H4a, $J_{H3a\text{-}H4a}$ = $J_{H4a\text{-}N5a}$ = 10,2 Hz) ; 5,16 (d, 1H, H3a).
**RMN $^{13}$C ($CDCl_3$, 100 MHz)** δ (ppm) : 1 Signaux caractéristiques 91,5 (C2a) ; 83,9 (C1a) ; 76,3 (C3a) ; 72,6 (C5a) ; 69,0 (C4a) ; 61,7 (C6a).

**Autres signaux :**

**RMN $^1$H ($CDCl_3$, 400 MHz)** δ (ppm) : 8,09-7,70 (m, 25H, H arom.) ; 7,60-6,84 (m, 65H, H arom.) ; 5,37 (t, 1H, H3f, $J_{H2f\text{-}H3f}$ = $J_{H3f\text{-}H4f}$ = 9,7 Hz) ; 5,20 (dd, 1H, H2f, $J_{H1f\text{-}H2f}$ = 8,0 Hz) ; 5,19 (t, 1H, H4f, $J_{H4f\text{-}H5f}$ = 9.7 Hz) ; 5,10 (dd, 1H, H2b, $J_{H1b\text{-}H2b}$ = 8,1 Hz, $J_{H2b\text{-}H3b}$ = 9,1 Hz) ; 5,07-4,96 (m, 4H, H1b, H2, 2 H4) ; 4,92-4,78 (m, 4H, 2 H2, 2 H4) ; 4,73 (d, 1H, H1, $J_{H1\text{-}H2}$ = 8,0 Hz) ; 4,66 (d, 1H, H1, $J_{H1\text{-}H2}$ = 8,0 Hz) ; 4,65-4,57 (m, 2H, H5a, H6) ; 4,54 (d, 1H, H1, $J_{H1\text{-}H2}$

= 8,0 Hz) ; 4,49 (d, 1H, H1, $J_{H1-H2}$ = 7,7 Hz) ; 4,47-4,41 (m, 1H, H6) ; 4,35 (dd, 1H, H6, $J_{H5-H6}$ = 4,9 Hz, $J_{H6-H6'}$ = 12,4 Hz) ; 4,28 (t, 1H, H3b, $J_{H3b-H4b}$ = 9,1 Hz) ; 4,19 (dd, 1H, H6, $J_{H5-H6}$ = 3,5 Hz, $J_{H6-H6'}$ = 12,2 Hz) ; 4,12 (dd, 1H, H6, $J_{H5-H6}$ = 6,2 Hz, $J_{H6-H6'}$ = 12,2 Hz) ; 4,05-3,81 (m, 11H, H5b, H3c, H3d, H3e, 7 H6) ; 3,80-3,62 (m, 4H, H5c, H5d, H5e, H5f).

**RMN $^{13}$C ($CDCl_3$, 100 MHz)** δ (ppm) : 165,9, 165,8, 165,7, 165,4, 164,9, 164,7, 164,6, 164,3, 163,8, 163,6 (18C, OCOPh) ; 133,6, 133,3, 133,2, 133,1, 133,0, 132,9, 132,6, 130,0, 129,8, 129,7, 129,6, 129,5, 129,4, 129,3, 129,2, 129,1, 129,0, 128,8, 128,6, 128,4, 128,3, 128,2, 128,1, 127,9 (C arom.) ; 101,0, 100,9, 100;7, 100,6 (5C, C1b, C1c, C1d, C1e, C1f) ; 78,3, 78,2, 78,1, 78,0 (4C, C3b, C3c, C3d, C3e) ; 73,5, 73,1, 73,0, 72,8 (4C, C2b, C2c, C2d, C2e); 72,6 (C3f); 71,9, 71,8, 71,6 (5C, C5b, C5c, C5d, C5e, C5f) ; 71,3 (C2f) ; 70,2, 70,1, 70,0, 69,8 (5C, C4b, C4c, C4d, C4e, C4f) ; 63,6, 63,5, 63,4, 63,3, 63,2 (5C, C6b, C6c, C6d, C6e, C6f).

7.5/ **Préparation de 2,3,4,6-tétra-*O*-benzoyl-β-D-glucopyranosyl-(1→3)-2,4,6-tri-*O*-benzoyl-β-D-glucopyranosyl-(1→3)-2,4,6-tri-*O*-benzoyl-β-D-glucopyranosyl-(1→3)-2,4,6-tri-*O*-benzoyl-β-D-glucopyranosyl-(1→3)-2,4,6-tri-*O*-benzoyl-β-D-glucopyranosyl-(1→3)-6-*O*-benzoyl-4-désoxy-β-D- gluco- hex- 3-enopyranos- 2-uloside de benzyle (Produit 6b)**

L'alcool benzylique (7 µL, 0,068 mmol) et le bromure d'ulosyle **5b** (183 mg, 0,063 mmol) sont mis en solution dans le dichlorométhane (1,5 mL) en présence de tamis moléculaire. Après 5 minutes d'agitation vigoureuse, l'oxyde de triphénylphosphine (19 mg, 0,068 mmol) est additionné rapidement au mélange. Après 4 jours d'agitation à température ambiante, le milieu est filtré sur célite, concentré sous pression réduite et purifié par chromatographie sur silice [éther de pétrole/acétate d'éthyle (3:2 ; v/v)]. Après évaporation des solvants, le brut ainsi obtenu est dissous dans le benzène (8 mL), puis additionné d'eau (200 µL, 11,1 mol) et d'hydrogénocarbonate de sodium (200 mg, 2,381 mmol). Le milieu est ensuite porté à 80°C pendant 1,5 heure. Après retour à température ambiante, la suspension est séchée sur MgSO4, filtrée et concentrée sous vide pour conduire au produit **6b** (120 mg ; 0,029 mmol) avec un rendement global de 67%.

**Produit 6b :** huile incolore ; **Rf** (EP/AcOEt, 1:1) 0,5.

**RMN $^1$H ($CDCl_3$, 400 MHz)** δ (ppm) : Signaux caractéristiques 6,26 (d, 1H, H4a, $J_{H4a-H5a}$ = 3,5 Hz) ; 4,87 (s, 1H, H1a) ; 4,83 (d, 1H, H7a, $J_{H7a-H7'a}$ = 11,7 Hz) ; 4,71-4,65 (m, 1H, H5a) ; 4,60 (d, 1H, H7'a).

**RMN $^{13}$C ($CDCl_3$, 100 MHz)** δ (ppm) : Signaux caractéristiques 183,6 (C2a) ; 144,9 (C3a) ; 126,3 (C4a) ; 97,7 (C1a) ; 71,0 (C5a) ; 70,6 (C7a) ; 66,1 (C6a).

<u>**Autres signaux :**</u>

**RMN $^1$H ($CDCl_3$, 400 MHz)** δ (ppm) : 8,06-7,71 (m, 25H, H arom.) ; 7,60-7,12 (m, 65H, H arom.) ; 5,37 (t, 1H, H3f, $J_{H2f-H3f}$ = $J_{H3f-H4f}$ = 9,5 Hz) ; 5,30-5,16 (m, 5H, H1b, H2b, H2f, H4b, H4f) ; 5,07-4,94 (m, 6H, H2c, H2d, H2e, H4c, H4d, H4e) ; 4,90 (d, 1H, H1c, $J_{H1c-H2c}$ = 8,0 Hz) ; 4,57 (d, 1H, H1, $J_{H1-H2}$ = 8,2 Hz) ; 4,55 (d, 2H, H1, $J_{H1-H2}$ = 8,0 Hz) ; 4,54-4,43 (m, 3H, H6a, 2 H6) ; 4,38-4,32 (m, 2H, H3b, H6) ; 4,24-4,20 (m, 2H, H3, H6'a) ; 4,16-4,04 (m, 4H, H6'a, H3, H5, H6) ; 4,02-3,82 (m, 8H, H3, 2 H5, 5 H6) ; 3,76-3,64 (m, 3H, H5f, 2 H5).

**RMN $^{13}$C ($CDCl_3$, 100 MHz)** δ (ppm) : 165,9, 165,8, 165,7, 165,4, 164,9, 164,7, 164,6, 164,4, 163,8, 163,6 (15C, OCOPh) ; 136,1 (C quat. arom. $OCH_2$*Ph*) ; 133,3, 133,1, 133,0, 132,9, 132,6, 130,0, 129,8, 129,7, 129,6, 129,5, 129,4, 129,3, 129,2, 129,1, 129,0, 128,8, 128,7, 128,6, 128,5, 128,4, 128,2, 128,1, 128,0 (C arom.); 101,0, 100,9, 100,8 (3C, C1d, C1e, C1f) ; 100,2 (C1c) ; 98,4 (C1b) ; 78,3, 78,2, 78,1 (3C, C3c, C3d, C3e) ; 77,6 (C3b) ; 73,3, 73,0, 72,9 (5C, C2b, C5b, C2c, C2d, C2e) ; 72,6 (C3f) ; 72,1, 72,0, 71,8 (3C, C5) ; 71,6 (C2f) ; 71,3 (2C, C5f, C5) ; 70,1, 70,0 (3C, C4c, C4d, C4e) ; 69,8 (C4f) ; 69,3 (C4b) ; 63,5, 63,4 (3C, C6f, 2 C6) ; 63,2, 63,1 (2C, C6b, C6).

| | | |
|---|---|---|
| **HRMS (ESI$^+$) :** | $[M+Na]^+$ $C_{162}H_{132}NaO_{47}$ | m/z théorique: 2851,7837<br>m/z mesurée: 2852,7830 |
| | $[M+K]^+$ $C_{162}H_{132}KO_{47}$ | m/z théorique: 2867,7576<br>m/z mesurée: 2867,7508 |

**7.6/ Préparation de 2,3,4,6-tétra-*O*-benzoyl-β-D-glucopyranosyl-(1→3)-2,4,6-tri-*O*-benzoyl-β-D-glucopyranosyl-(1→3)-2,4,6-tri-*O*-benzoyl-β-D-glucopyranosyl-(1→3)-2,4,6-tri-*O*-benzoyl-β-D-glucopyranosyl-(1→3)-2,4,6-tri-*O*-benzoyl-β-D-glucopyranosyl-(1→3)-6-*O*-benzoyl-4-désoxy-β-D- manno- hex- 3-enopyranosyle de**

**benzyle (Produit 7b)**

La réduction du composé **6b** (104 mg, 0,036 mmol) est réalisée suivant le même mode opératoire que celui décrit pour **7a** en présence de L-Sélectride (solution à 1M dans THF; 50 μL, 0,050 mmol) dans le THF (1 mL). Le composés 7b (105 mg ;0,036 mmol) est ainsi obtenu après traitements de manière quantitative.

**Produit 7b :** huile incolore ; **Rf** (EP/AcOEt, 1: 1) 0,5.
**RMN $^1$H ($CDCl_3$, 400 MHz)** δ (ppm) : Signaux caractéristiques 4,96 (d, 1H, H4a, $J_{H4a-H5a}$ < 1,0 Hz) ; 4,60 (d, 1H, H7'a, $J_{H7a-H7'a}$ = 11,7 Hz) ; 4,45 (d, 1H, H1a, $J_{H1a-H2a}$ = 2,2 Hz) ; 4,28-4,22 (m, 2H, H5a, H6a).
**RMN $^{13}$C ($CDCl_3$, 100 MHz)** δ (ppm) : Signaux caractéristiques 151,4 (C3a) ; 104,1 (C4a) ; 97,0 (C1a) ; 70,3 (C5a) ; 70,0 (C7a) ; 66,2 (C6a) ; 65,1 (C2a).

**Autres signaux :**

**RMN $^1$H ($CDCl_3$, 400 MHz)** δ (ppm) : 8,04-7,73 (m, 25H, H arom.) ; 7,60-7,10 (m, 65H, H arom.) ; 5,39 (t, 1H, H3f, $J_{H2f-H3f}$ = $J_{H3f-H4f}$ = 9,7 Hz) ; 5,34 (t, 1H, H4b, $J_{H3b-H4b}$ = $J_{H4b-H5b}$ = 8,6 Hz) ; 5,22 (dd, 1H, H2f, $J_{H1f-H2f}$ = 7,7 Hz) ; 5,21 (t, 1H, H4f, $J_{H4f-H5f}$ = 9,7 Hz) ; 5,19 (dd, 1H, H2b, $J_{H1b-H2b}$ = 7,1 Hz, $J_{H2b-H3b}$ = 9,5 Hz) ; 5,18 (d, 1H, H1b) ; 5,06-4,97 (m, 3H, H2, 2 H4) ; 4,90-4,81 (m, 4H, H7a, 2 H2, H4) ; 4,58 (d, 3H, 3H1, $J_{H1-H2}$ = 7,7 Hz) ; 4,53 (dd, 1H, H6, $J_{H5-H6}$ = 3,3 Hz, $J_{H6-H6'}$ = 12,2 Hz) ; 4,49 (d, 1H, H1, $J_{H1-H2}$ = 8,0 Hz) ; 4,39-4,32 (m, 2H, H3b, H6) ; 4,20-4,06 (m, 4H, H6'a, H3, H5, H6) ; 4,00-3,83 (m, 12H, H2a, H5f, 3 H3, 7 H6) ; 3,76-3,71 (m, 4H, 4 H5).
**RMN $^{13}$C ($CDCl_3$, 100 MHz)** δ (ppm) : 166,1, 165,9, 165,8, 165,7, 165,4, 164,9, 164,7, 164,6, 164,5, 164,4, 164,2, 163,8, 163,6 (17C, OCOPh) ; 136,5 (C quat. arom. $OCH_2$*Ph*) ; 133,4, 133,2, 133,1, 133,0, 132,9, 132,6, 129,9, 129,8, 129,7, 129,6, 129,5, 129,4, 129,3, 129,2, 129,1, 129,0, 128,9, 128,7, 128,6, 128,5, 128,4, 128,3, 128,2, 128,1, 128,0, 127,9 (C arom.); 101,0, 100,9, 100,7, 100,6 (4C, C1c, C1d, C1e, C1f) ; 97,3 (C1b) ; 78,2 (3C, C3c, C3d, C3e) ; 77,5 (C3b) ; 73,4, 73,1 (3C, C2c, C2d, C2e) ; 72,9 (C2b) ; 72,5 (C3f) ; 71,9 (C5b) ; 71,7 (1C, C5) ; 71,6 (3C, C5f, 2 C5) ; 71,2 (C2f) ; 70,1 (2C, C4) ; 69,7 (C4f) ; 69,3, 69,2 (2C, C4b, C2) ; 63,4, 63,3, 63,1 (5C, C6b, C6c, C6d, C6e, C6f).

| | | |
|---|---|---|
| **HRMS ($ESI^+$):** | $[M+Na]^+$ $C_{162}H_{134}NaO_{47}$ | m/z théorique: 2853,7993<br>m/z mesurée: 2853,7994 |
| | $[M+K]^+$ $C_{162}H_{134}KO_{47}$ | m/z théorique: 2869,7732<br>m/z mesurée: 2869,7650 |

**7.7/ Préparation de β-D-glucopyranosyl-(1→3)-β-D-glucopyranosyl-(1→3)-β-D-glucopyranosyl-(1→3)-β-D-glucopyranosyl-(1-3)-β-D-glucopyranosyl-(1→3)-4-désoxy-β-D-manno-hex-3-enopyranosyle de benzyle (Produit 8b)**

Le composé **7b** (105 mg, 0,036 mmol) est engagé dans une réaction de débenzoylation suivant le même mode opératoire que celui décrit pour **8a** dans un mélange dichlorométhane / méthanol (3:2 ; v/v) (2,5 mL) en présence MeONa (solution à 0,1 M dans MeOH ; 0,71 mL, 0,71 mmol). La purification sur colonne de gel de silice [acétate d'éthyle/méthanol (3:2 ; v/v)] permet d'obtenir **8b** (38 mg) avec un rendement quantitatif.

**Produit 8b :** huile incolore.
**RMN $^1$H ($CD_3OD$, 400 MHz)** δ (ppm) : Signaux caractéristiques 5,06 (d, 1H, H4a, $J_{H4a-H5a}$ = 1,6 Hz) ; 4,82 (d, 1H, H7a, $J_{H7a-H7'a}$ = 11,9 Hz) ; 4,60 (d, 1H, H7'a) ; 4,49 (d, 1H, H1a, $J_{H1a-H2a}$ = 1,8 Hz) ; 4,12-4,08 (m, 1H, H5a) ; 3,88 (d, 1H, H2a).
**RMN $^{13}$C ($CD_3OD$, 100 MHz)** δ (ppm) : Signaux caractéristiques 153,5 (C3a) ; 103,9 (C4a) ; 99,8 (C1a) ; 71,2 (C7a) ; 67,2 (C2a) ; 65,6 (C6a).

**Autres signaux :**

**RMN $^1$H ($CD_3OD$, 400 MHz)** δ (ppm) : 7,30-7,17 (m, 5H, H arom.) ; 4,71 (d, 1H, H1b, $J_{H1b-H2b}$ = 7,7 Hz) ; 4,55 (d, 3H, H1c, H1d, H1e, $J_{H1-H2}$ = 7,8 Hz) ; 4,47 (d, 1H, H1f, $J_{H1f-H2f}$ = 8,0 Hz) ; 3,81-3,72 (m, 5H, 5 H6) ; 3,62-3,44 (m, 12H, H6a, H6'a, H2b, 3 H3, 5 H6) ; 3,40-3,34 (m, 3H, H2c, H2d, H2e) ; 3,32-3,14 (m, 12H, H2f, H3f, H4f, 4 H4, 5 H5).

**RMN $^{13}C$ ($CD_3OD$, 100 MHz)** δ (ppm) : 138,9 (C quat. arom. $OCH_2$*Ph*) ; 129,4, 129,3, 128,9, 128,7 (C arom.) ; 105,2, 104,7 (4C, C1c, C1d, C1e, C1f) ; 100,5 (C1b) ; 87,4, 87,3, 87,1 (4C, C3b, C3c, C3d, C3e) ; 78,1, 77,8 (6C, C3f, C5b, C5c, C5d, C5e, C5f) ; 75,5 (C2f) ; 75,0, 74,9 (4C, C2c, C2d, C2e, C5a) ; 74,0 (C2b) ; 71,5 (C4f) ; 70,0, 69,9 (4C, C4b, C4c, C4d, C4e) ; 62,6, 62,5 (5C, C6b, C6c, C6d, C6e, C6f).

**7.8/ Préparation de β-D- glucopyranosyl-(1→3)- β-D- glucopyranosyl-(1→3)- β-D- glucopyranosyl-(1→3)- β-D-glucopyranosyl-(1→3)- β-D- glucopyranosyl-(1→3)- 4-désoxy-(α, β)-D-mannopyranose (Produit Cb)**

Le composé **8b** (60 mg, 0,056 mmol) est réduit et débenzylé suivant le même mode opératoire que celui décrit pour **Ca**, à savoir par hydrogénolyse en présence d'acétate de palladium (60 mg, 0,267 mmol). La purification sur colonne Sephadex G-10 conduit de manière quantitative au produit souhaité **Cb** en mélange α/β où le composé a est très largement majoritaire.

**Produit Cb :** mousse blanche.
**RMN $^{1}H$ ($D_2O$, 400 MHz)** δ (ppm) : Signaux caractéristiques 5,14 (d, 1H, H1aα, $J_{H1a-H2a}$ = 3,3 Hz) ; 1,75-1,60 (m, 2H, H4a).
**RMN $^{13}C$ ($D_2O$, 100 MHz)** δ (ppm) : Signaux caractéristiques 96,0 (C1aα) ; 61,0 (C6a) ; 26,0 (C4a).

__Autres signaux :__

**RMN $^{1}H$ ($D_2O$, 400 MHz)** δ (ppm) : 4,70 (d, 3H, H1, $J_{H1-H2}$ = 8,0 Hz) ; 4,66 (d, 2H, H1, $J_{H1-H2}$ = 8,2 Hz) ; 3,86-3,78 (m, 6H, H6a, H6b, H6c, H6d, H6e, H6f) ; 3,73-3,60 (m, 11H, H3a, H3b, H3c, H3d, H3e, H6'a, H6'b, H6'c, H6'd, H6'e, H6'f) ; 3,50-3,36 (m, 16H, H2a, H2b, H2c, H2d; H2e, H3f, H4b, H4c, H4d, H4e, H5a, H5b, H5c, H5d, H5e, H5f) ; 3,31 (t, 1H, H4f, $J_{H3f-H4f}$ = $J_{H4f=H5f}$ = 9,3 Hz) ; 3,26 (dd, 1H, H2f, $J_{H1f-H2f}$ = 8,0 Hz, $J_{H2f-H3f}$ = 9,0 Hz).
**RMN $^{13}C$ ($D_2O$, 100 MHz)** δ (ppm) : 103,1, 102,8 (5C, C1b, C1c, C1d, C1e, C1f) ; 84,5, 84,3 (5C, C3a, C3b, C3c, C3d, C3e) ; 76,3, 75,9 (6C, C3f, C5a, C5b, C5c, C5d, C5e, C5f) ; 73,8, 73,6 (6C, C2a, C2b, C2c, C2d, C2e, C2f) ; 69,9 (C4f) ; 68,4 (4C, C4b, C4c, C4d, C4e) ; 61,0 (5C, C6b, C6c, C6d, C6e, C6f).

**HRMS (ESI$^+$) :** $[M+Na]^+$ $C_{36}H_{62}NaO_{30}$ m/z théorique: 997,3224
m/z mesurée: 997,3201

**7.9/ Préparation de β-D- glucopyranosyl-(1→3)- β-D- glucopyranosyl-(1→3)- β-D- glucopyranosyl-(1→3)- β-D-glucopyranosyl-(1→3)- β-D- glucopyranosyl-(1→3)- 4-désoxy- D- mannitol (Produit Db)**

Le composé **Cb** (40 mg, 0,041 mmol) est dissous dans un mélange méthanol/eau (4:1, v/v) puis additionné de 10 équivalents de borohydrure de sodium (15,5 mg, 0,411 mmol). Après une semaine d'agitation à température ambiante, le milieu est neutralisé par ajout de quelques gouttes d'acide acétique, concentré sous pression réduite, coévaporé 3 fois avec 10 mL d'un mélange méthanol/acide acétique (9:1, v/v) et pour finir avec 3 fois 10 mL de méthanol. Le composé **Db** est obtenu après purification sur colonne de perméation de gel (Sephadex G-10, éluant : eau) et lyophilisation des fractions récoltées.

**Produit Db :** mousse blanche.
**RMN $^{1}H$ ($D_2O$, 400 MHz)** δ (ppm) : Signaux caractéristiques 3,97-3,90 (m, 1H, H3a) ; 1,70-1,40 (m, 2H, H4a).
**RMN $^{13}C$ ($D_2O$, 100 MHz)** δ (ppm) : Signaux caractéristiques 76,3 (C3a) ; 71,0 (C2a, C5a) ; 66,1 (C6a); 63,0 (C1a); 25,3 (C4a).

__Autres signaux :__

**RMN $^{1}H$ ($D_2O$, 400 MHz)** δ (ppm) : 4,70 (d, 3H, H1, $J_{H1-H2}$ = 8,0 Hz) ; 4,66 (d, 2H, H1, $J_{H1-H2}$ = 8,0 Hz) ; 3,87-3,76 (m, 9H, H1 a, H2a, H5a, H6a, H6b, H6c, H6d, H6e, H6f) ; 3,72-3,51 (m, 12H, H1'a, H3a, H3b, H3c, H3d, H3e, H6'a, H6'b, H6'c, H6'd, H6'e, H6'f) ; 3,50-3,36 (m, 14H, H2b, H2c, H2d, H2e, H3f, H4b, H4c, H4d, H4e, H5b, H5c, H5d, H5e, H5f) ; 3,31 (t, 1H, H4f, $J_{H3f-H4f}$ = $J_{H4f-H5f}$ = 9,3 Hz) ; 3,26 (dd, 1H, H2f, $J_{H1f-H2f}$ = 8,0 Hz, $J_{H2f-H3f}$ = 9,3 Hz).
**RMN $^{13}C$ ($D_2O$, 100 MHz)** δ (ppm) : 103,1, 102,8 (5C, C1b, C1c, C1d, C1e, C1f) ; 84,5, 84,3 (4C, C3b, C3c, C3d, C3e) ; 76,3 (C5f) ; 75,9 (5C, C3f, C5a, C5b, C5c, C5d, C5e) ; 73,8, 73,6 (5C, C2b, C2c, C2d, C2e, C2f) ; 69,9 (C4f) ; 68,4, 68,2 (4C, C4b, C4c, C4d, C4e) ; 61,0 (5C, C6b, C6c, C6d, C6e, C6f).

**HRMS (ESI+) :** $[M+Na]^+$ $C_{36}H_{64}NaO_{30}$ m/z théorique: 999,3380
m/z mesurée: 999,3411

Les exemples 8 à 11 montrent les activités biologiques de composés de l'invention. Dans ces exemples, les composés testés sont :

LAM5 : laminaripentaose,
LAM7 : laminariheptaose,

A2 est le composé préparé selon l'exemple 3,
A0 représente le composé suivant:

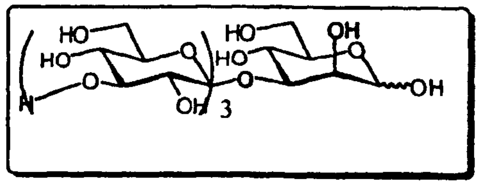

Le témoin utilisé est le PBS.
Les dosages utilisés sont de 100μg/souris.

**EXEMPLE 8: Effet de LAM5, LAM7, A2 et A0 sur la phagocytose de cellules de sang périphérique:**

On a injecté, par voie péritonéale, chez un groupe de souris Balb/c (Jackson laboratory, Bar Harbor, ME, USA) l'un ou l'autre de un témoin, LAM5, LAM7, A0 ou A2. 24 heures après l'injection les souris ont été sacrifiées. On a récupéré le sang périphérique à partir du plexus orbital dans de l'héparine (5 IU/ml) (Sigma).

Après comptage, un essai de phagocytose de particles de HEMA (microsphères synthétiques préparées à partir de copolymère de 2-hydroxyéthylméthacrylate) a été réalisé comme décrit dans: Rembaum et al. , 1976, Vetvicka et al. 1982, Bilej et al. , 1989. 0,1 ml of sang frais hépariné a été ajouté à 0,05 ml de particules de HEMA diluées (5x108/ml) et incubé pendant 60 minutes à 37° C sous agitation modérée occasionnelle.

A la fin de l'incubation, la suspension de cellules a été frottée sur des lamelles de microscope. Les frottis ont été évalués sous microscope optique après coloration Giemsa.

Les cellules englobant au moins trois particules ont été considérées comme positives.

Les résultats moyens sont représentés graphiquement sur la figure 1. Ils montrent clairement que aussi bien A0 que A2 stimulent fortement la phagocytose dans les monocytes et les granulocytes.

**EXEMPLE 9: Effet des oligosaccharides sur la phagocytose de cellules issues de la cavité péritonéale:**

On a injecté par voie intrapéritonéale un groupe de souris Balb/c (Jackson laboratory, Bar Harbor, ME, USA) avec l'un ou l'autre de un témoin, LAM7, LAM5, A0 ou A2.

Après 24 h, les souris ont été sacrifiées, les cellules péritonéales ont été recueillies dans un milieu Hanks (Sigma).

Après comptage des cellules dans un hémocytomètre, les cellules péritonéales ont été diluées tolx$10^7$ dans un milieu RPMI 1640 (Sigma) avec 5% de sérum de veau foetal (Hyclone, Logan, UT, USA).
2x$10^6$ cellules dans 0,2 mil de milieu RPMI 1640 additionné de 5% de sérum de veau foetal ont été mélangées avec le même volume de particules HEMA (5x$10^8$/ml).

La suspension a été incubée pendant 60 minutes at 37° C sous agitation modérée occasionnelle. L'incubation a été terminée par centrifugation (150 g pendant 5 minutes) et la boulette a été remise en suspension.

Les macrophages avec des particules ingérées ont été comptés sous microscope optique dans des frottis colorés par Accustain (colorant Wright modifié, Sigma).

Les cellules englobant au moins six particules ont été considérées comme étant positives.

Les résultats moyens sont représentés graphiquement sur la Figure 2.Ils montrent qu'aussi bien A0 que A2 stimulent fortement la phagocytose à la fois dans les monocytes et les granulocytes de macrophages péritonéaux.

**EXEMPLE 10: Effet de A0 et A2 sur un dénombrement différentiel dans le sang**

En utilisant les mêmes groupes expérimentaux que dans l'exemple 8, deux lamelles microscopiques supplémentaires à partir de chaque échantillon expérimental ont été préparées. Après coloration Giemsa, on a évalué au microscope optique, pour chaque lamelle la présence de types individuels de cellules, i. e. monocytes, lymphocytes et granulocytes.

Les résultats moyens sont représentés graphiquement sur la Figure 3. Ils montrent que à la fois A0 et A2 augmentent le nombre de monocytes et de granulocytes dans le sang périphérique.

**EXEMPLE 11: Effet de A0 et A2 sur un dénombrement différentiel dans les cellules péritonéales**

En utilisant les mêmes groupes expérimentaux que dans l'exemple 8, deux lamelles microscopiques supplémentaires à partir de chaque échantillon expérimental ont été préparées. Après coloration Giemsa, on a évalué au microscope optique, pour chaque lamelle la présence de types individuels de cellules, i. e. macrophages, lymphocytes et mastocytes.

Les résultats moyens sont représentés graphiquement sur la Figure 4. Ils montrent que à la fois A0 et A2 augmentent le nombre de monocytes et de granulocytes dans la cavité péritonéale.

**Tableau 1a:** ***Déplacements chimiques et multiplicité en RMN$^1$H des monosaccharides répondant à la formule :***

| 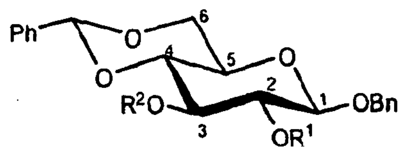 | | | | | | | | | |
|---|---|---|---|---|---|---|---|---|---|
| **Composé** | | | **δ (ppm) / $J_{Hn-Hn+1}$** | | | | | | |
| **n°** | **$R^1$** | **$R^2$** | **H-1/$J_{1,2}$** | **H-2/$J_{2,3}$** | **H-3/$J_{3,4}$** | **H-4/$J_{4,5}$** | **H-5/$J_{5,6}$** | **H-6/$J_{6,6'}$** | **H-6'/$J_{6',5}$** |
| **3** | Lev | NAP | 4,51/8,1 | 5,1219,1 | 3,76/9,1 | 3,83/9,1 | 3,44/5,1 | 4,39/10,4 | 3,85/10,4 |
| **4** | Lev | H | 4,56/8,0 | 4,99/9,1 | 3,61/9,1 | 3,86/9,1 | 3,42/5,1 | 4,36/10,4 | 3,80/10,4 |
| **10** | H | NAP | 4,50/7,6 | 3,70/8,9 | 3,72/8,9 | 3,76/9,9 | 3,45/5,1 | 4,38/10,2 | 3,84/10,2 |

**Tableau 1b:** ***Déplacements chimiques et multiplicité*** **en** ***RMN $^{13}$C*** **des** ***monosaccharides répondant à la formule :***

| 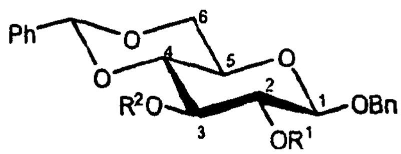 | | | | | | | | |
|---|---|---|---|---|---|---|---|---|
| **Composé** | | | **δ (ppm)** | | | | | |
| **n°** | **$R^1$** | **$R^2$** | **C-1** | **C-2** | **C-3** | **C-4** | **C-5** | **C-6** |
| **3** | Lev | NAP | 100,1 | 73,2 | 78,1 | 81,5 | 66,2 | 68,6 |
| **4** | Lev | H | 99,8 | 74,5 | 72,0 | 80,5 | 66,2 | 68,5 |
| **10** | H | NAP | 102,2 | 74,5 | 80,0 | 81,3 | 66,5 | 68,7 |

**Tableau 2a:** ***Déplacements chimiques et multiplicité*** **en** ***RMN*** $^{1}$***H des monosaccharides répondant à la formule :***

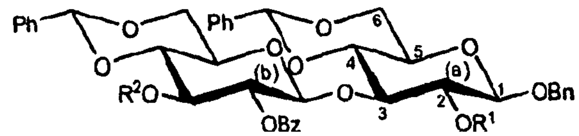

| Composé | | | δ (ppm)/multiplicité | | | | | | | | | | | | | |
|---|---|---|---|---|---|---|---|---|---|---|---|---|---|---|---|---|
| | | | Unité (b) | | | | | | | Unité (a) | | | | | | |
| n° | $R^1$ | $R^2$ | H-1/$J_{1,2}$ | H-2/$J_{2,3}$ | H-3/ $J_{3,4}$ | H-4/ $J_{4,5}$ | H-5/$J_{5,6}$ | H-6/$J_{6,6'}$ | H-6'/ $J_{6',5}$ | H-1/ $J_{1,2}$ | H-2/ $J_{2,3}$ | H-3/$J_{3,4}$ | H-4/$J_{4,5}$ | H-5/ $J_{5,6}$ | H-6/$J_{6,6'}$ | H-6'/$J_{6',5}$ |
| **5** | Lev | NAP | 4,9317,1 | 5,26/7,1 | 3,85/9,1 | 3,95/9,1 | 3,43/4.9 | 4,13/10,4 | 3,68/10,4 | 4,45/7,8 | 5.06/8,9 | 3,97/8,9 | 3,80/8.9 | 3,43/4,9 | 4,35/10,4 | 3,81/10,4 |
| **6** | Lev | H | 5,0216,8 | 5,1417,1 | 3,99/9,3 | 3.75/9.3 | 3.46/5.1 | 4,18/10,6 | 3,70/10,6 | 4,49/7,5 | 5,09/8,4 | 4,04/9,1 | 3.78/9.1 | 3,49/4,8 | 4,39/10,6 | 3,82/10,6 |
| **11** | H | NAP | 4,95/7,1 | 5,36/7,5 | 3,96/9,1 | 3,75/9,1 | 3,38/4,9 | 4,19/10,4 | 3,62/10.4 | 4,37/7,8 | 3,50/8,6 | 3,88/9.0 | 3.77/9.0 | 3,45/5,1 | 4,32/10,6 | 3,78/10,6 |

Tableau 2b: *Déplacements chimiques et multiplicité en RMN $^{13}C$ des monosaccharides répondant à la formule :*

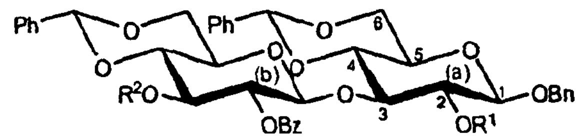

| Composé | | | δ (ppm) | | | | | | | | | | | |
|---|---|---|---|---|---|---|---|---|---|---|---|---|---|---|
| | | | Unité (b) | | | | | | Unité (a) | | | | | |
| **n°** | **$R^1$** | **$R^2$** | **C-1** | **C-2** | **C-3** | **C-4** | **C-5** | **C-6** | **C-1** | **C-2** | **C-3** | **C-4** | **C-5** | **C-6** |
| **5** | Lev | NAP | 100,1 | 74,0 | 78,1 | 80,7 | 65,9 | 88,8 | 99,8 | 72,9 | 77,5 | 79,7 | 66,3 | 68,6 |
| **6** | Lev | H | 100,0 | 75,6 | 72,5 | 80,5 | 65,8 | 68,6 | 99,7 | 72,9 | 77,6 | 79,6 | 66,2 | 68,6 |
| **11** | H | NAP | 101,1 | 74,0 | 77,8 | 81,3 | 66,0 | 68,6 | 102,1 | 74,1 | 79,2 | 81,1 | 66,5 | 68,7 |

Tableau 3a: *Déplacements chimiques et multiplicité en RMN $^1H$ des monosaccharides répondant à la formule :*

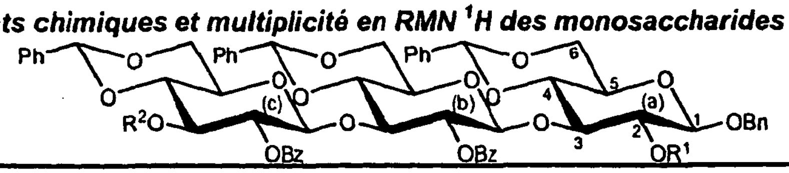

| Composé | | | δ (ppm)/multiplicité | | | | | | | | | | | | | |
|---|---|---|---|---|---|---|---|---|---|---|---|---|---|---|---|---|
| | | | Unité (c) | | | | | | | Unité (b) | | | | | | |
| n° | $R^1$ | $R^2$ | H-1 / $J_{1,2}$ | H-2 / $J_{2,3}$ | H-3 / $J_{3,4}$ | H-4 / $J_{4,5}$ | H-5 / $J_{5,6}$ | H-6 / $J_{6,6'}$ | H-6' / $J_{6',5}$ | H-1 / $J_{1,2}$ | H-2 / $J_{2,3}$ | H-3 / $J_{3,4}$ | H-4 / $J_{4,5}$ | H-5 / $J_{5,6}$ | H-6 / $J_{6,6'}$ | H-6' / $J_{6',5}$ |
| 7 | Lev | NAP | 5,14/ 7,5 | 5,39/ 8,2 | 4,05/ 8,0 | 3,98- 3,87/ nd | 3,62- 3,52/ 5,1 | 4,27/ 10,6 | 3,76/ 10,6 | 5,12/ 4,2 | 5,16- 5,11/ nd | 4,15- 4,08/ nd | 3,98- 3,87/ nd | 3,69- 3,60/ nd | 4,15- 4,08/ 10,4 | 3,49/ 10,4 |
| 8 | Lev | H | 5,14/ 7,8 | 5,20/ 7,8 | 3,94/ 7,8 | 3,62/ 7,8 | 3,49/ 4,9 | 4,19/ 10,4 | 3,66/ 10,4 | 5,11/ 4,0 | 5,14- 5,10/ nd | 4,09- 4,03/ nd | 4,09- 4,03/ nd | 3,66- 3,57/ nd | 4,09- 4,03/ 10,0 | 3,44/ 10,0 |
| 12 | H | NAP | 5,12/ 8,0 | 5,40/ 8,0 | 3,92/ 9,0 | 3,95/ 9,0 | 3,53/ 4,9 | 4,29/ 10,4 | 3,79/ 10,4 | 5,13/ 4,2 | 5,18/ 4,2 | 4,02/ 8,0 | 4,21/ 10,2 | 3,64/ 4,6 | 4,09/ 10,0 | 3,52/ 10,0 |

| Composé | | | δ (ppm)/multiplicité | | | | | | |
|---|---|---|---|---|---|---|---|---|---|
| | | | Unité (a) | | | | | | |
| n° | $R^1$ | $R^2$ | H-1 / $J_{1,2}$ | H-2 / $J_{2,3}$ | H-3 / $J_{3,4}$ | H-4 / $J_{4,5}$ | H-5 / $J_{5,6}$ | H-6 / $J_{6,6'}$ | H-6' / $J_{6',5}$ |
| 7 | Lev | NAP | 4,33/ 8,0 | 4,60/ 8,8 | 3,92/ 9,3 | 3,02/ 9,3 | 3.31/ 5,1 | 4,27/ 10,4 | 3,58/ 10,4 |
| 8 | Lev | H | 4,29/ 7,8 | 4,64/ 9,2 | 3,89/ 9,2 | 2,98/ 9,2 | 3,27/ 4,9 | 4,23/ 10,4 | 3,53/ 10,4 |
| 12 | H | NAP | 4,27/ 8,0 | 2,85/ 8,0 | 3,73/ 8,8 | 3,39- 3,32/ nd | 3,39- 3,32/ 3,8 | 4,32/ 11,0 | 3,75/ 11,0 |

Tableau 3b: *Déplacements chimiques et multiplicité en RMN $^{13}C$ des monosaccharides répondant à la formule :*

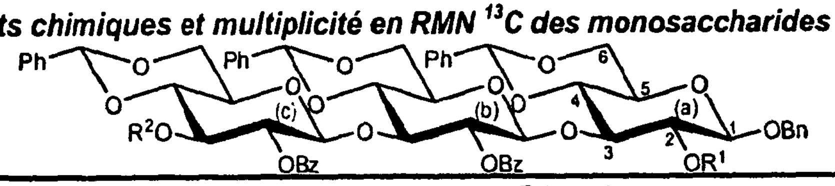

| Composé | | | δ (ppm) | | | | | | | | | | | |
|---|---|---|---|---|---|---|---|---|---|---|---|---|---|---|
| | | | Unité (c) | | | | | | Unité (b) | | | | | |
| n° | $R^1$ | $R^2$ | C-1 | C-2 | C-3 | C-4 | C-5 | C-6 | C-1 | C-2 | C-3 | C-4 | C-5 | C-6 |
| 7 | Lev | NAP | 98,0 | 73,2 | 76,1 | 81,4 | 66,1 | 68,7 | 97,3 | 72,2 | 77,6 | 78,1 | 65,1 | 68,7 |
| 8 | Lev | H | 97,8 | 74,6 | 72,4 | 80,8 | 66,0 | 68,6 | 97,4 | 72,3 | 76,2 | 78,7 | 65,1 | 68,7 |
| 12 | H | NAP | 98,6 | 73,3 | 78,2 | 81,3 | 66,1 | 68,6 | 99,6 | 73,5 | 77,9 | 77,6 | 65,1 | 68,7 |

| Composé | | | δ (ppm) | | | | | |
|---|---|---|---|---|---|---|---|---|
| | | | Unité (a) | | | | | |
| n° | $R^1$ | $R^2$ | C-1 | C-2 | C-3 | C-4 | C-5 | C-6 |
| 7 | Lev | NAP | 99,6 | 73,8 | 73,5 | 78,7 | 66,2 | 68,5 |
| 8 | Lev | H | 99,6 | 73,7 | 73,7 | 77,6 | 66,2 | 68,5 |
| 12 | H | NAP | 102,0 | 74,7 | 76,7 | 78,8 | 66,6 | 68,6 |

Tableau 3'a: *Déplacements chimiques et multiplicité en RMN $^1H$ des monosaccharides répondant à la formule :*

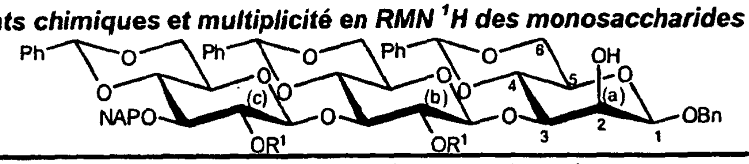

| Composé | | δ (ppm)/multiplicité | | | | | | | | | | | | | |
|---|---|---|---|---|---|---|---|---|---|---|---|---|---|---|---|
| | | Unité (c) | | | | | | | Unité (b) | | | | | | |
| n° | $R^1$ | H-1 / $J_{1,2}$ | H-2 / $J_{2,3}$ | H-3 / $J_{3,4}$ | H-4 / $J_{4,5}$ | H-5 / $J_{5,6}$ | H-6 / $J_{6,6'}$ | H-6' / $J_{6',5}$ | H-1 / $J_{1,2}$ | H-2 / $J_{2,3}$ | H-3 / $J_{3,4}$ | H-4 / $J_{4,5}$ | H-5 / $J_{5,6}$ | H-6 / $J_{6,6'}$ | H-6' / $J_{6',5}$ |
| 18 | Bz | 4,97 / 7,5 | 5,34 / 7,5 | 4,11 / 9,3 | 3,94 / 9,3 | 3,48 / 4,8 | 4,24 / 10,4 | 3,74 / 10,4 | 4,84 / 6,2 | 5,26 / 6,2 | 3,82 / 9,5 | 4,05 / nd | 3,55 / 4,6 | 4,18 / 10,2 | 3,74 / 10,2 |
| 19 | H | 4,57 / 7,7 | 3,79-3,68 / 9,5 | 3,83 / 9,5 | 3,66 / 9,5 | 3,38 / 4,8 | 4,30 / 10,4 | 3,75 / 10,4 | 4,57 / 5,1 | 3,79-3,68 / nd | 3,79-3,68 / m | 3,79-3,68 / m | 3,38 / 4,8 | 4,30 / 10,4 | 3,76 / 10,4 |

| Composé | | δ (ppm)/multiplicité | | | | | | |
|---|---|---|---|---|---|---|---|---|
| | | Unité (a) | | | | | | |
| n° | $R^1$ | H-1 / $J_{1,2}$ | H-2 / $J_{2,3}$ | H-3 / $J_{3,4}$ | H-4 / $J_{4,5}$ | H-5 / $J_{5,6}$ | H-6 / $J_{6,6'}$ | H-6' / $J_{6',5}$ |
| 18 | Bz | 4,35 / 0,7 | 3,82 / 10,2 | 3,70 / 9,3 | 3,94 / 9,3 | 3,25 / 4,8 | 4,32 / 10,4 | 3,76 / 10,4t |
| 92 | H | 4,59 / < 1,0 | 4,19 / 2,7 | 3,92 / 9,5 | 4,12 / 9,5 | 3,45 / 5,1 | 4,37 / 10,6 | 3,92 / 10,6 |

**Tableau 3'b: *Déplacements chimiques et multiplicité en RMN $^{13}C$ des monosaccharides répondant à la formule :***

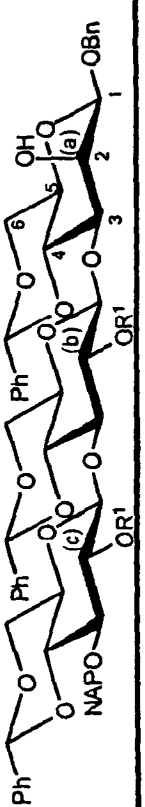

| Composé | | δ (ppm) | | | | | | | | | | | |
|---|---|---|---|---|---|---|---|---|---|---|---|---|---|
| | | Unité (c) | | | | | | Unité (b) | | | | | |
| **n°** | **$R^1$** | **C-1** | **C-2** | **C-3** | **C-4** | **C-5** | **C-6** | **C-1** | **C-2** | **C-3** | **C-4** | **C-5** | **C-6** |
| **18** | Bz | 100,1 | 73,5 | 77,8 | 80,9 | 66,0 | 68,6 | 98,8 | 74,0 | 78,0 | 78,3 | 66,1 | 68,6 |
| **19** | H | 105,7 | 73,0 | 83,6 | 78,6 | 66,9 | 68,3 | 101,3 | 75,1 | 79,9 | 80,8 | 66,7 | 68,5 |

| Composé | | δ (ppm) | | | | | |
|---|---|---|---|---|---|---|---|
| | | Unité (a) | | | | | |
| **n°** | **$R^1$** | **C-1** | **C-2** | **C-3** | **C-4** | **C-5** | **C-6** |
| **18** | Bz | 98,3 | 68,9 | 77,0 | 76,5 | 67,0 | 68,4 |
| **19** | H | 98,1 | 69,4 | 76,9 | 76,3 | 67,1 | 68,4 |

## Revendications

1. Composé de formule générale (I') ou de formule générale (II') :

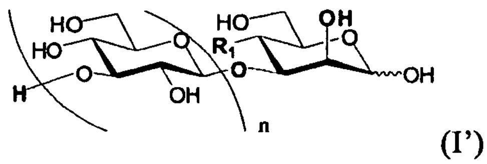

(I')

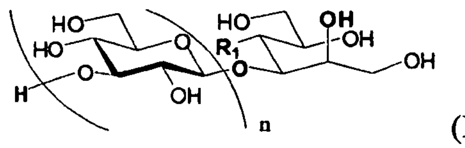

(II')

dans lesquelles $R_1$ représente H ou OH et n est un entier de 2 à 10, de préférence de 2 à 7, et plus préférentiellement encore égal à 3, 4 ou 5.

2. Composé selon la revendication 1, **caractérisé en ce qu'**il est choisi parmi
β-D-glucopyranosyl-(1→3)-β-D-glucopyranosyl-(1→3)-(α,β)-D-mannopyranose ;
β-D-glucopyranosyl-(1→3)-β-D-glucopyranosyl-(1→3)-β-D-glucopyranosyl-(1→3)-β-D-glucopyranosyl-(1→3)-β-D-glucopyranosyl-(1→3)-β-D-mannopyranose ;
β-D-glucopyranosyl-(1→3)-β-D-glucopyranosyl-(1→3)-4-désoxy-(α,β)-D-désoxy-mannopyranose ;
β-D-glucopyranosyl-(1→3)-β-D-glucopyranosyl-(1→3)-mannitol ;
β-D-glucopyranosyl-(1→3)-β-D-glucopyranosyl-(1→3)-4-désoxy-mannitol ;
β-D-glucopyranosyl-(1→)-β-D-glucopyranosyl-(1→3)-β-D-glucopyranosyl-(1→3)-β-D-glucopyranosyl-(1→3)-β-D-glucopyranosyl-(1→3)-mannitol ;
β-D-glucopyranosyl-(1→3)-β-D-glucopyranosyl-(1→3)-β-D-glucopyranosyl-(1→3)-β-D-glucopyranosyl-(1→3)-β-D-glucopyranosyl-(1→3)-4-désoxy-(α,β)-D-mannopyranose ; et
β-D-glucopyranosyl-(1→3)-β-D-glucopyranosyl-(1→3)-β-D-glucopyranosyl-(1→3)-β-D-glucopyranosyl-(1→3)-β-D-glucopyranosyl-(1→3)-4-désoxy-mannitol.

3. Procédé de préparation d'un composé selon la revendication 1 ou la revendication 2, comprenant une réaction entre un donneur de glycosyle de formule (D) ci-dessous et un accepteur de glycosyle de formule (A) ci-dessous :

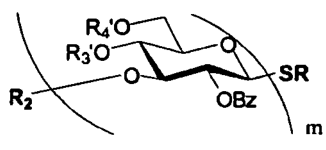

(D)

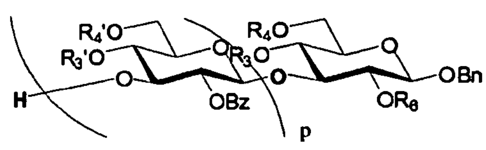

(A)

dans lesquelles

m est un entier de 1 à 9, de préférence de 1 à 6, et plus préférentiellement encore égal à 2, 3 ou 4 ;
p est un entier de 0 à 9, de préférence de 0 à 6, et plus préférentiellement encore égal à 2, 3 ou 4 ;
R représente alkyle tel que éthyle, méthyle, propyle ou butyle, ou aryle tel que phényle ou tolyle ;

$R_2$ représente allyle, méthylnaphtyle, benzyle, paraméthoxybenzyle, halogénoacétyle (chloroacétyle, bromoacétyle, iodoacétyle) ;
$R_3$ et $R_4$ d'une part et $R_3$' et $R_4$' d'autre part forment ensemble un radical éthylidyle, isopropylidyle, hexafluoroisopropylidyle, cyclopentylidyle, cyclohexylidyle, cycloheptylidyle, butylidyle, 1-tertiobutyléthylidyle, benzylidyle, méthoxybenzylidyle, 1-phénylbenzylidyle ou $R_3$, $R_4$, $R_3$' et $R_4$' représentent chacun indépendamment l'un de l'autre un benzyle, chlorobenzyle, nitrobenzyle, allyle, triarylméthyle, trialkylsilyle tel que triéthylsilyle, tri-isopropylsilyle, tertiobutyldiméthylsilyle, ester tel que acétyle, chloroacétyle, benzoyle, pivaloyle ;
$R_5$ représente H, un groupe lévulinoyle, acétyle, chloroacétyle, fluorénylméthyloxycarbonyle, trialkylsilyle tel que triéthylsilyle, tri-isopropylsilyle, tertiobutyldiméthylsilyle, de préférence un groupe lévulinoyle,
à la condition que aucun de $R_3$, $R_4$, $R_3$', $R_4$' ne soit identique à $R_5$.

**4.** Procédé selon la revendication 3, **caractérisé par le fait que** le composé de formule (A) est

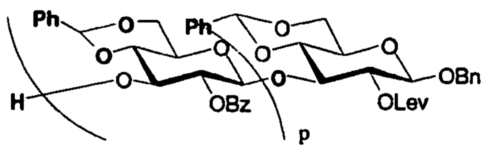

**5.** Procédé selon la revendication 3, **caractérisé par le fait que** le composé de formule (D) est

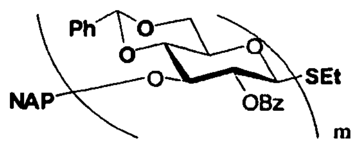

**6.** Procédé de préparation d'un composé selon la revendication 1 ou la revendication 2, comprenant une étape d'épimérisation en position 2 de l'entité terminale.

**7.** Composé choisi parmi les composés de formules (III), (IV), (V) et (VI)

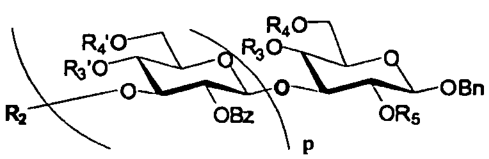

(III)

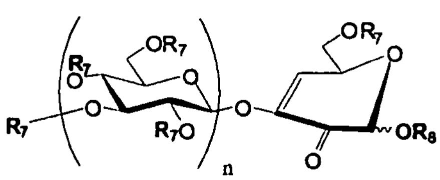

(IV)

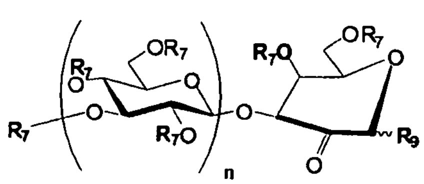

(V)

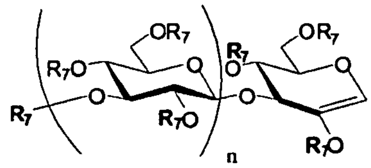

(VI)

dans lesquelles :

p est un entier de 0 à 9, de préférence de 0 à 6, et plus préférentiellement encore égal à 2, 3 ou 4 ;
$R_2$ représente hydrogène, allyle, méthylnaphtyle, benzyle, paraméthoxybenzyle, halogénoacétyle (chloroacétyle, bromoacétyle, iodoacétyle) ;
$R_3$ et $R_4$ d'une part et $R'_3$ et $R'_4$ d'autre part, forment ensemble un radical éthylidyle, isopropylidyle, hexafluoroisopropylidyle, cyclopentylidyle, cyclohexylidyle, cycloheptylidyle, butylidyle, 1-tertiobutyléthylidyle, benzylidyle, méthoxybenzylidyle, 1-phénylbenzylidyle,
ou $R_3$, $R_4$, $R_3$' et $R_4$' représentent chacun indépendamment l'un de l'autre un benzyle, chlorobenzyle, nitrobenzyle, allyle, triarylméthyle, trialkylsilyle tel que triéthylsilyle, tri-isopropylsilyle, tertiobutyldiméthylsilyle, ester tel que acétyle, chloroacétyle, benzoyle, pivaloyle ;
$R_5$ représente H, un groupe lévulinoyle, acétyle, chloroacétyle, fluorénylméthyloxycarbonyle, trialkylsilyle tel que triéthylsilyle, tri-isopropylsilyle, tertiobutyldiméthylsilyle, de préférence un groupe lévulinoyle,
à la condition que aucun de $R_3$, $R_4$, $R_3$', $R_4$' ne soit identique à $R_5$,
$R_7$ représente un ester, notamment acétyle, benzoyle, pivaloyle, de préférence Bz ;
$R_8$, un groupement orthogonal à tous les autres groupements protecteurs, qui peut être choisi parmi Bn, NAP, p-méthoxybenzyle, p-méthoxyphényle, allyle, et de préférence $R_8$ représente Bn, et
$R_9$ représente $OR_8$ ou X.

**8.** Médicament comprenant un composé tel que défini à la revendication 1 ou à la revendication 2.

**9.** Composé tel que défini à la revendication 1 ou à la revendication 2 pour une utilisation dans une méthode de traitement d'une maladie choisie dans le groupe consistant en tumeur, cancer, maladie virale, maladie bactérienne, maladie fongique, maladie du système immunitaire, maladie auto-immune ou maladie liée à une déficience de l'immunostimulation, chez des animaux à sang chaud et des êtres humains.

## Claims

**1.** A compound of general formula (I') or of general formula (II'):

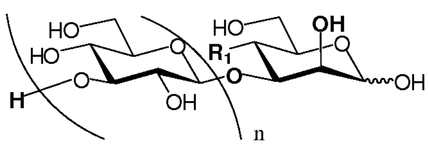

(I')

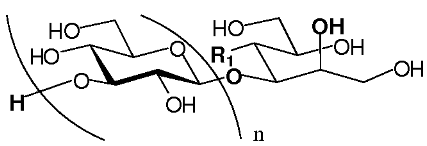

(II')

wherein $R_1$ represents H or OH and n is an integer from 2 to 10, preferably from 2 to 7, and more preferably equal to 3, 4 or 5.

**2.** The compound according to claim 1, wherein said compound is selected from

β-D-glucopyranosyl-(1→3)-β-D-glucopyranosyl-(1→3)-(α,β)-D-mannopyranose;
β-D-glucopyranosyl-(1→3)-β-D-glucopyranosyl-(1→3)-β-D-glucopyranosyl-(1→3)-β-D-glucopyranosyl-(1→3)-β-D-glucopyranosyl-(1→3)-β-D-mannopyranose;
β-D-glucopyranosyl-(1→3)-β-D-glucopyranosyl-(1→3)-4-deoxy-(α,β)-D-deoxy-mannopyranose;
β-D-glucopyranosyl-(1→3)-β-D-glucopyranosyl-(1→3)-mannitol;
β-D-glucopyranosyl-(1→3)-β-D-glucopyranosyl-(1→3)-4-deoxy-mannitol;
β-D-glucopyranosyl-(1→3)-β-D-glucopyranosyl-(1→3)-β-D-glucopyranosyl-(1→3)-β-D-glucopyranosyl-(1→3)-β-D-glucopyranosyl-(1→3)-mannitol;
β-D-glucopyranosyl-(1→3)-β-D-glucopyranosyl-(1→3)-β-D-glucopyranosyl-(1→3)-β-D-glucopyranosyl-(1→3)-β-D-glucopyranosyl-(1→3)-4-deoxy-(α,β)-D-mannopyranose; and
β-D-glucopyranosyl-(1→3)-β-D-glucopyranosyl-(1→3)-β-D-glucopyranosyl-(1→3)-β-D-glucopyranosyl-(1→3)-β-D-glucopyranosyl-(1→3)-4-deoxy-mannitol.

**3.** A method for preparing a compound according to claim 1 or 2 comprising a reaction between a glycosyl donor of formula (D) below and a glycosyl acceptor of formula (A) below:

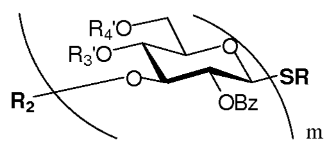

(D)

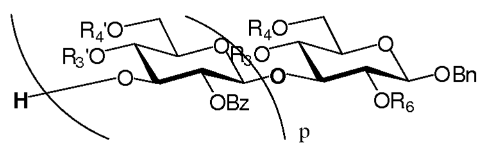

(A)

wherein

m is an integer from 1 to 9, preferably from 1 to 6, and more preferably equal to 2, 3 or 4;
p is an integer from 0 to 9, preferably from 0 to 6, and more preferably equal to 2, 3 or 4;
R represents alkyl such as ethyl, methyl, propyl or butyl, or aryl such as phenyl or tolyl; $R_2$ represents allyl, methylnaphthyl, benzyl, paramethoxybenzyl, halogenoacetyl (chloroacetyl, bromoacetyl, iodoacetyl);
$R_3$ and $R_4$, on the one hand, and $R_3$' and $R_4$', on the other hand, together form an ethylidyl, isopropylidyl, hexafluoroisopropylidyl, cyclopentylidyl, cyclohexylidyl, cycloheptylidyl, butylidyl, 1-tertiobutylethylidyl, benzylidyl, methoxybenzylidyl, 1-phenylbenzylidyl radical or $R_3$, $R_4$, $R_3$' and $R_4$' represent each independently of each other a benzyl, chlorobenzyl, nitrobenzyl, allyl, triarylmethyl, trialkylsilyl such as triethylsilyl, tri-isopropylsilyl, tertiobutyldimethylsilyl, ester such as acetyl, chloroacetyl, benzoyl, pivaloyl;
$R_5$ represents H, a levulinoyl, acetyl, chloroacetyl, fluorenylmethyloxycarbonyl, trialkylsilyl group such as triethylsilyl, tri-isopropylsilyl, tertiobutyldimethylsilyl, preferably a levulinoyl group,
provided that none of $R_3$, $R_4$, $R_3$', $R_4$' is identical to $R_5$.

**4.** The method according to claim 3, wherein the compound of formula (A) is

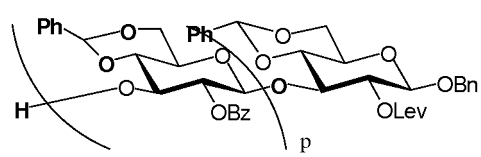

**5.** The method according to claim 3, wherein the compound of formula (D) is

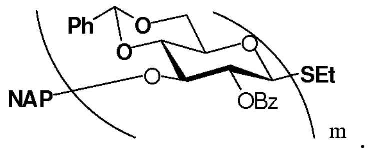

6. A method for preparing a compound according to claim 1 or claim 2, comprising a step of epimerization in position 2 of the terminal entity.

7. A compound selected from the compounds of formulae (III), (IV), (V) and (VI)

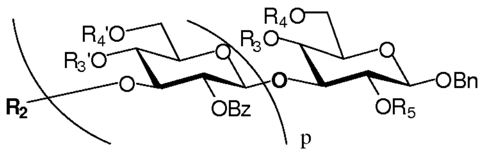

(III)

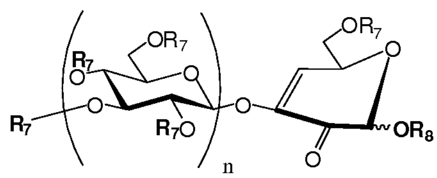

(IV)

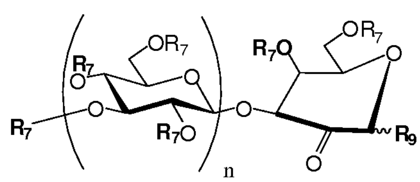

(V)

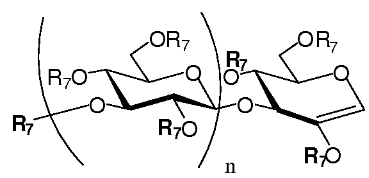

(VI)

wherein

p is an integer from 0 to 9, preferably from 0 to 6, and more preferably equal to 2, 3 or 4;
$R_2$ represents hydrogen, allyl, methylnaphthyl, benzyl, paramethoxybenzyl, halogenoacetyl (chloroacetyl, bromoacetyl, iodoacetyl);
$R_3$ and $R_4$, on the one hand, and $R'_3$ and $R'_4$, on the other hand, together form an ethylidyl, isopropylidyl, hexafluoroisopropylidyl, cyclopentylidyl, cyclohexylidyl, cycloheptylidyl, butylidyl, 1-tertiobutylethylidyl, benzylidyl, methoxybenzylidyl, 1-phenylbenzylidyl radical,
or $R_3$, $R_4$ $R_3$' and $R_4$' represent each independently of each other a benzyl, chlorobenzyl, nitrobenzyl, allyl, triarylmethyl, trialkylsilyl such as triethylsilyl, tri-isopropylsilyl, tertiobutyldimethylsilyl, ester such as acetyl, chloroacetyl, benzoyl, pivaloyl;
$R_5$ represents H, a levulinoyl, acetyl, chloroacetyl, fluorenylmethyloxycarbonyl, trialkylsilyl group such as triethylsilyl, tri-isopropylsilyl, tertiobutyldimethylsilyl, preferably a levulinoyl group,
provided that none of $R_3$, $R_4$, $R_3$', $R_4$' is identical to $R_5$;

$R_7$ represents an ester, in particular acetyl, benzoyl, pivaloyl, preferably Bz;
$R_8$, a group orthogonal to all the other protective groups, which can be chosen from Bn, NAP, p-methoxybenzyl, p-methoxyphenyl, allyl, and preferably $R_8$ represents Bn,
$R_9$ represents $OR_8$ or X.

**8.** A medicine comprising a compound as defined in claim 1 or 2.

**9.** The compound as defined in claim 1 or 2 for use in a method for treatment of a disease selected from the group consisting of tumour, cancer, viral disease, bacterial disease, fungal disease, disease of the immune system, auto-immune disease or disease linked to a deficiency in immunostimulation, in warm-blooded animals and human beings.

**Patentansprüche**

**1.** Verbindung der allgemeinen Formel (I') oder der allgemeinen Formel (II'):

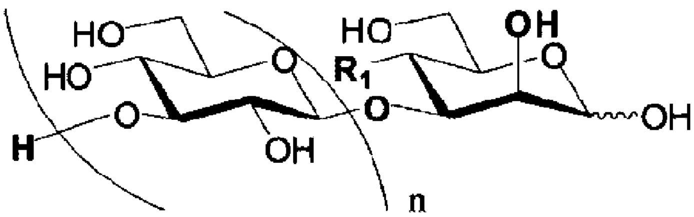

(I')

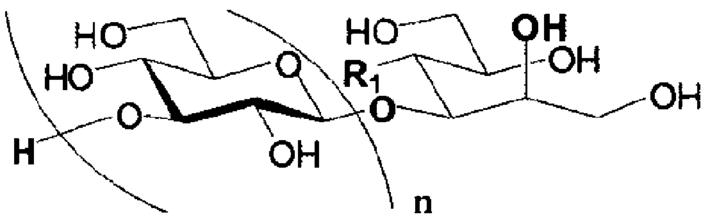

(II')

in denen $R_1$ H oder OH bedeutet und n eine Ganzzahl von 2 bis 10, vorzugsweise von 2 bis 7, und noch bevorzugter gleich 3, 4 oder 5 ist.

**2.** Verbindung gemäß Anspruch 1, **dadurch gekennzeichnet, dass** sie ausgewählt ist aus
β-D-Glucopyranosyl-(1→3)-β-D-glucopyranosyl-(1→3)-(α,β)-D-mannopyranose;
β-D-Glucopyranosyl-(1→3)-β-D-glucopyranosyl-(1→3)-β-D-glucopyranosyl-(1→3)-β-D-glucopyranosyl-(1→3)-β-D-glucopyranosyl-(1→3)-β-D-mannopyranose;
β-D-Glucopyranosyl-(1→3)-β-D-glucopyranosyl-(1→3)-4-desoxy-(α,β)-D-desoxy-mannopyranose;
β-D-Glucopyranosyl-(1→3)-β-D-glucopyranosyl-(1→3)-mannitol;
β-D-Glucopyranosyl-(1→3)-β-D-glucopyranosyl-(1→3)-4-desoxy-mannitol,
β-D-Glucopyranosyl-(1→3)-β-D-glucopyranosyl-(1→3)-β-D-glucopyranosyl-(1→3)-β-D-glucopyranosyl-(1→3)-β-D-glucopyranosyl-(1→3)-mannitol;
β-D-Glucopyranosyl-(1→3)-β-D-glucopyranosyl-(1→3)-β-D-glucopyranosyl-(1→3)-β-D-glucopyranosyl-(1→3)-β-D-glucopyranosyl-(1→3)-4-desoxy-(α,β)-D-mannopyranose; und
β-D-glucopyranosyl-(1→3)-β-D-glucopyranosyl-(1→3)-4-desoxy-(α,β)-D-(1→3)-β-D-glucopyranosyl-(1→3)-β-D-glucopyranosyl-(1→3)-4-desoxy-mannitol.

**3.** Verfahren zur Herstellung einer Verbindung nach Anspruch 1 oder Anspruch 2, umfassend eine Reaktion zwischen einem Glykosyldonor der nachfolgenden Formel (D) und einem Glykosylakzeptor der nachfolgenden Formel (A):

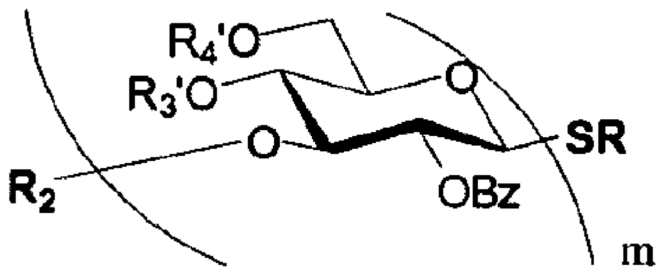

(D)

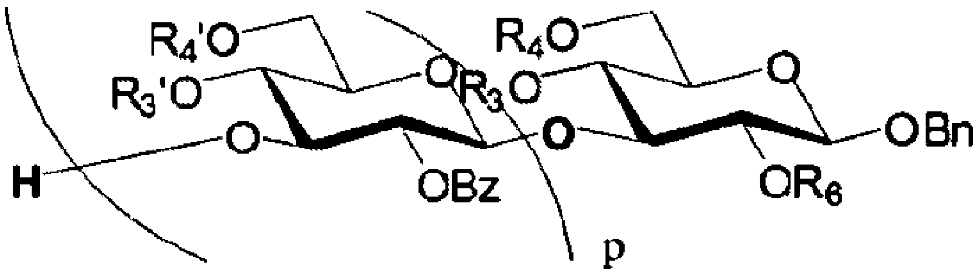

(A)

in denen

m eine Ganzzahl von 1 bis 9, vorzugsweise von 1 bis 6 und noch bevorzugter gleich 2, 3 oder 4 ist;
p eine Ganzzahl von 0 bis 9, vorzugsweise von 0 bis 6 und noch bevorzugter gleich 2, 3 oder 4 ist;
R Alkyl, wie zum Beispiel Ethyl, Methyl, Propyl oder Butyl, oder Aryl, wie zum Beispiel Phenyl oder Toluyl, bedeutet;
$R_2$ Allyl, Methylnaphthyl, Benzyl, Paramethoxybenzyl, Halogenacetyl (Chloracetyl, Bromacetyl, Iodacetyl) bedeutet;
$R_3$ und $R_4$ einerseits und $R_3$' und $R_4$' andererseits zusammen einen Rest des Typs Ethylidyl, Isopropylidyl, Hexyfluoroisopropylidyl, Cyclopentylidyl, Cyclohexylidyl, Cycloheptylidyl, Butylidyl, 1-tert-Butylethylidyl, Benzylidyl, Methoxybenzylidyl, 1-Phenylbenzylidyl bilden oder $R_3$, $R_4$, $R_3$' und $R_4$' jeweils unabhängig voneinander ein Benzyl, Chlorbenzyl, Nitrobenzyl, Allyl, Triarylmethyl, Trialkylsilyl, wie zum Beispiel Triethylsilyl, Triisopropylsilyl, tert-Butyldimethylsilyl, Ester, wie zum Beispiel Acetyl, Chloracetyl, Benzoyl, Pivaloyl bedeuten;
$R_5$ H, Levulinoyl, Acetyl, Chloracetyl, Fluorenylmethyloxycarbonyl, Trialkylsilyl wie zum Beispiel Triethylsilyl, Triisopropylsilyl, tert-Butyldimethylsilyl, vorzugsweise eine Levulinoylgruppe bedeutet,
mit der Bedingung, dass keiner der Reste $R_3$, $R_4$, $R_3$', $R_4$' identisch mit $R_5$ ist.

4. Verfahren gemäß Anspruch 3, **gekennzeichnet durch** die Tatsache, dass die Verbindung der Formel (A)

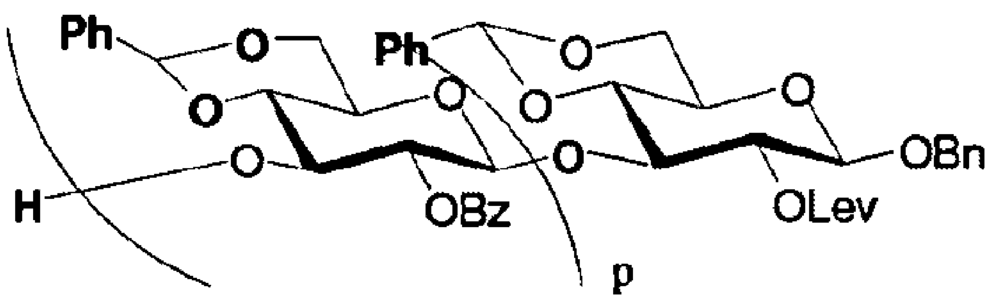

ist.

5. Verfahren gemäß Anspruch 3, **gekennzeichnet durch** die Tatsache, dass die Verbindung der Formel (D)

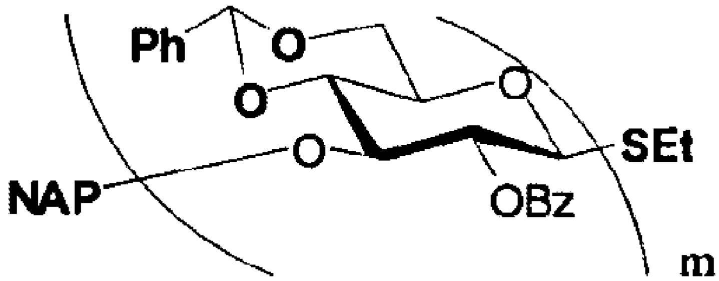

ist.

6. Verfahren zur Herstellung einer Verbindung nach Anspruch 1 oder Anspruch 2, umfassend einen Schritt der Epimerisierung an Position 2 der terminalen Einheit.

7. Verbindung, ausgewählt aus den Verbindungen der Formeln (III), (IV), (V) und (VI)

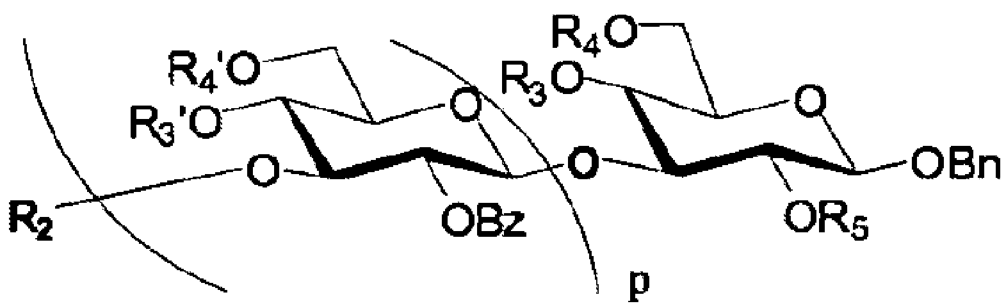

(III)

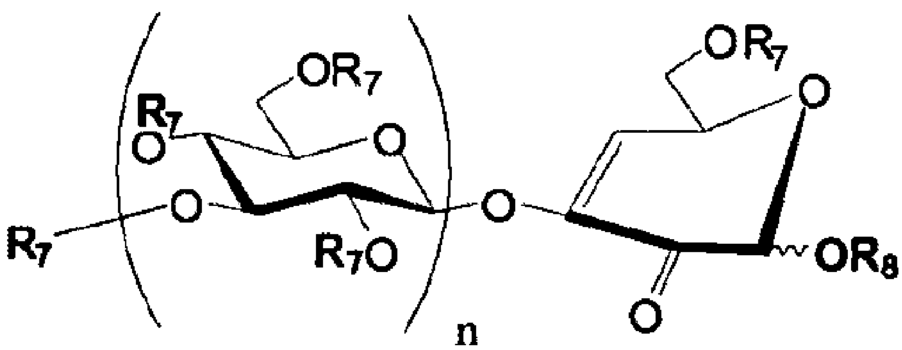

(IV)

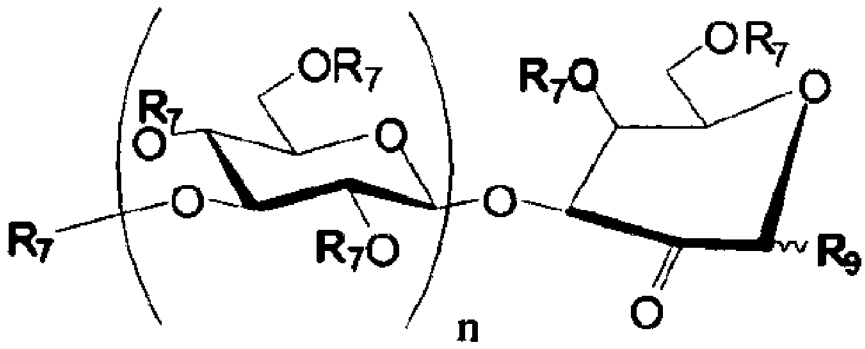

(V)

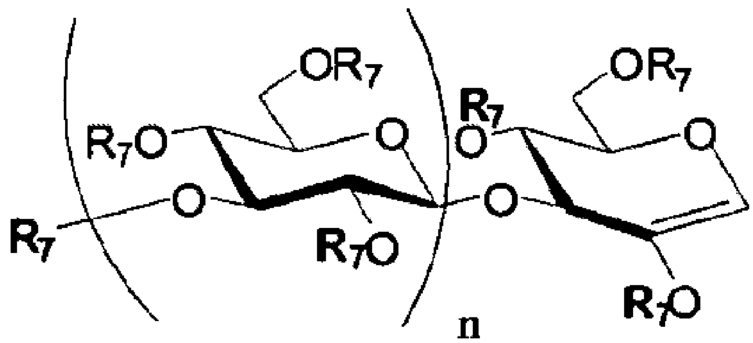

(VI)

in denen:

p eine Ganzzahl von 0 bis 9, vorzugsweise von 0 bis 6 und noch bevorzugter gleich 2, 3 oder 4 ist;
$R_2$ Wasserstoff, Allyl, Methylnaphthyl, Benzyl, Paramethoxybenzyl, Halogenacetyl (Chloracetyl, Bromacetyl, Iodacetyl) bedeutet;
$R_3$ und $R_4$ einerseits und $R_3$' und $R_4$' andererseits zusammen einen Ethylidyl-, Isopropylidyl-, Hexyfluoroisopropylidyl-, Cyclopentylidyl-, Cyclohexylidyl-, Cycloheptylidyl-, Butylidyl-, 1-tert-Butylethylidyl-, Benzylidyl-, Methoxybenzylidyl, 1-Phenylbenzylidylrest bilden,
oder $R_3$, $R_4$, $R_3$' und $R_4$' jeweils unabhängig voneinander Benzyl, Chlorbenzyl, Nitrobenzyl, Allyl, Triarylmethyl, Trialkylsilyl, wie zum Beispiel Triethylsilyl, Triisopropylsilyl, tert-Butyldimethylsilyl, Ester, wie zum Beispiel Acetyl, Chloroacetyl, Benzoyl, Pivaloyl bedeuten;
$R_5$ H, Levulinoyl, Acetyl, Chloracetyl, Fluorenylmethyloxycarbonyl, Trialkylsilyl, wie zum Beispiel Triethylsilyl, Triisopropylsilyl, tert-Butyldimethylsilyl, vorzugsweise eine Levulinoylgruppe bedeutet,
mit der Bedingung, dass keiner der Reste $R_3$, $R_4$, $R_3$', $R_4$' identisch mit $R_5$ ist;
$R_7$ einen Ester, insbesondere Acetyl, Benzoyl, Pivaloyl, vorzugsweise Bz bedeutet;
$R_8$ eine Gruppe ist, die orthogonal zu allen anderen Schutzgruppen ist und die aus Bn, NAP, p-Methoxybenzyl, p-Methoxyphenyl, Allyl ausgewählt ist, und $R_8$ vorzugsweise Bn bedeutet, und
$R_9$ $OR_8$ oder X bedeutet.

**8.** Medikament, umfassend eine Verbindung wie in Anspruch 1 oder Anspruch 2 definiert.

**9.** Verbindung wie in Anspruch 1 oder Anspruch 2 definiert zur Verwendung in einem Verfahren zur Behandlung einer Krankheit, die ausgewählt ist aus der Gruppe bestehend aus Tumor, Krebs, Viruserkrankung, bakterielle Erkrankung, Pilzerkrankung, Erkrankung des Immunsystems, Autoimmunerkrankung oder eine Erkrankung, die mit einer Immunstimulationsdefizienz verbunden ist, bei warmblütigen Tieren und Menschen.

Fig.1

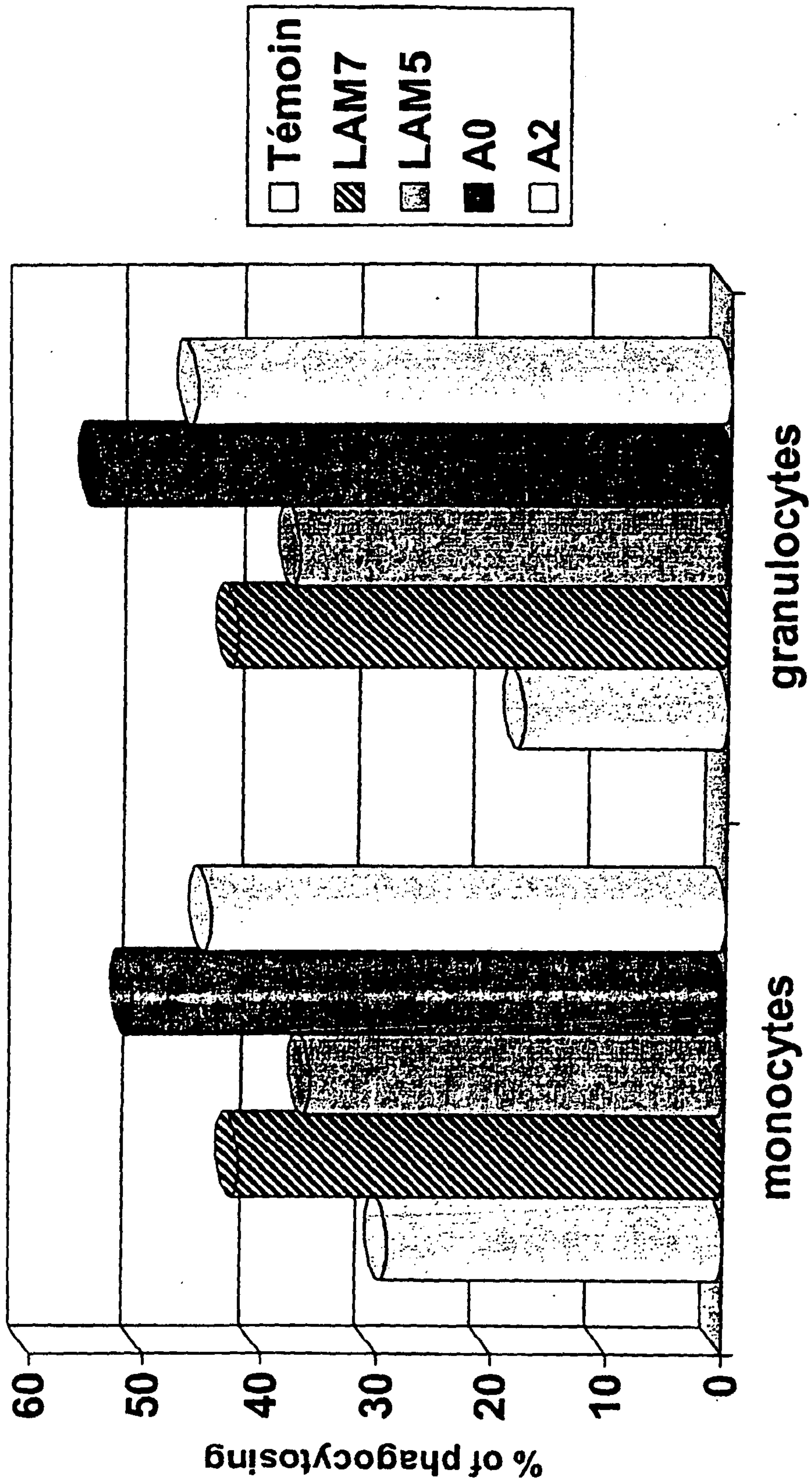
Témoin
LAM7
LAM5
A0
A2
% of phagocytosing
0
10
20
30
40
50
60
monocytes
granulocytes

Fig. 2

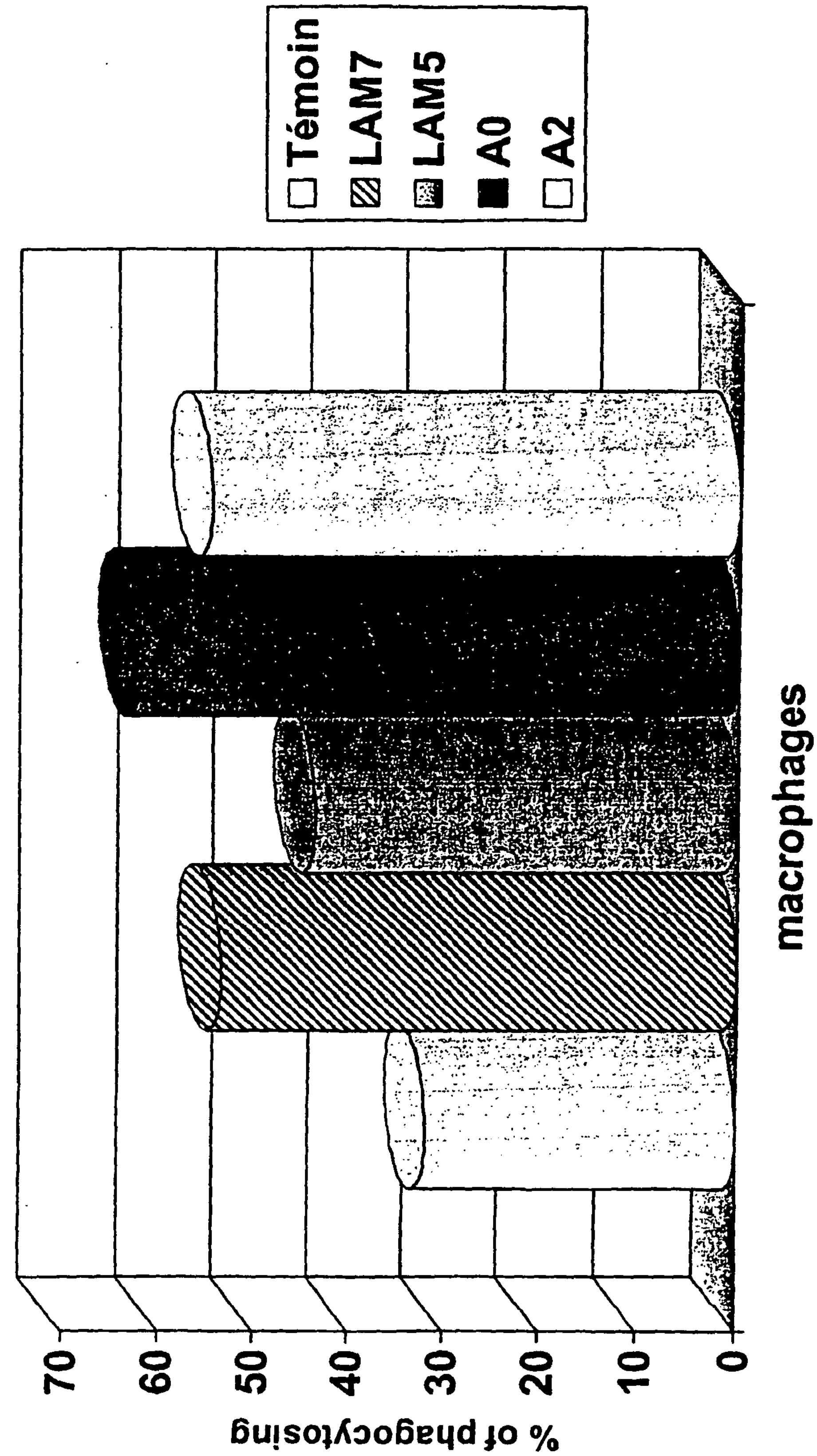
Témoin
LAM7
LAM5
A0
A2
% of phagocytosing macrophages
0
10
20
30
40
50
60
70

Fig.3

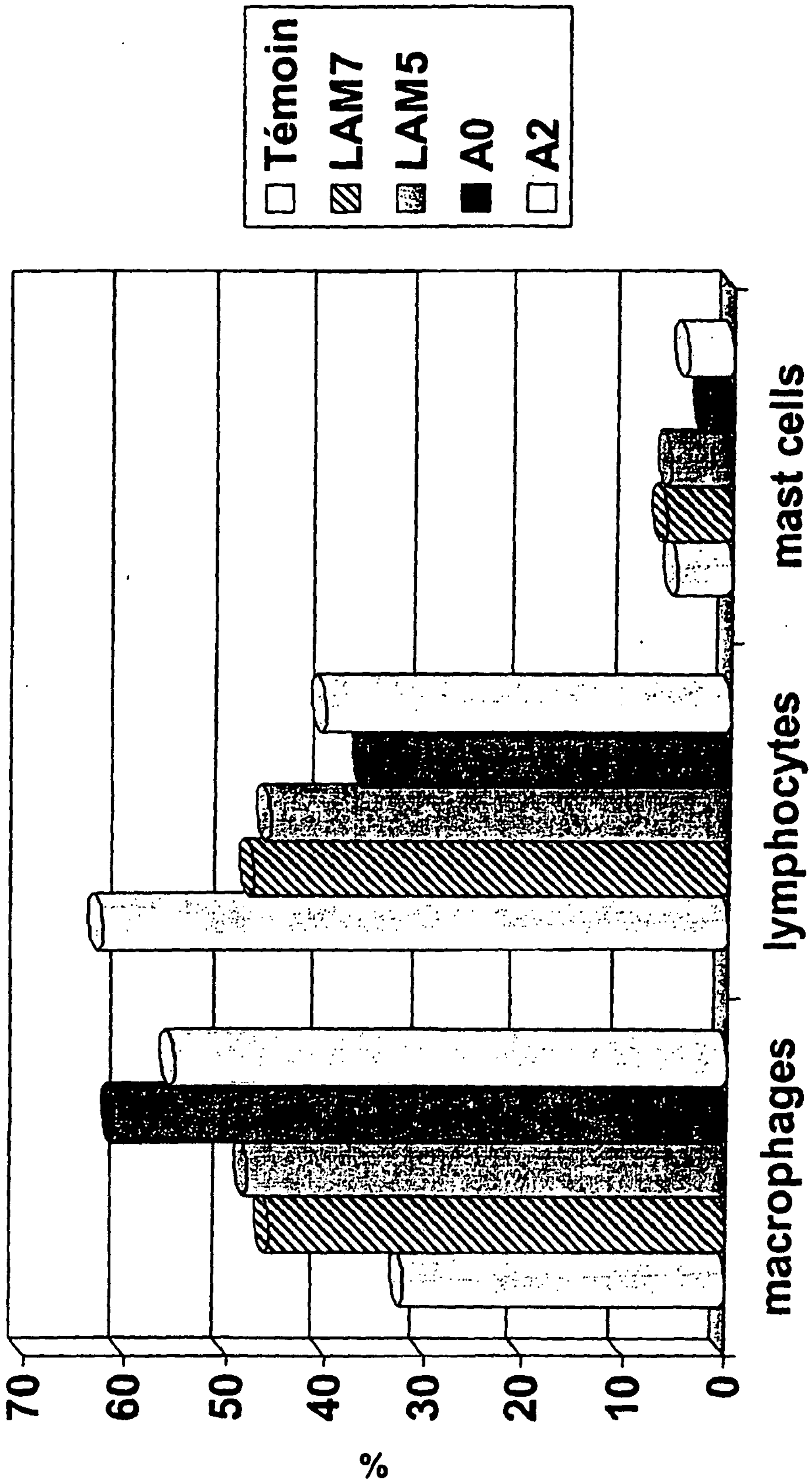
Témoin
LAM7
LAM5
A0
A2
%
70
60
50
40
30
20
10
0
macrophages
lymphocytes
mast cells

Fig.4

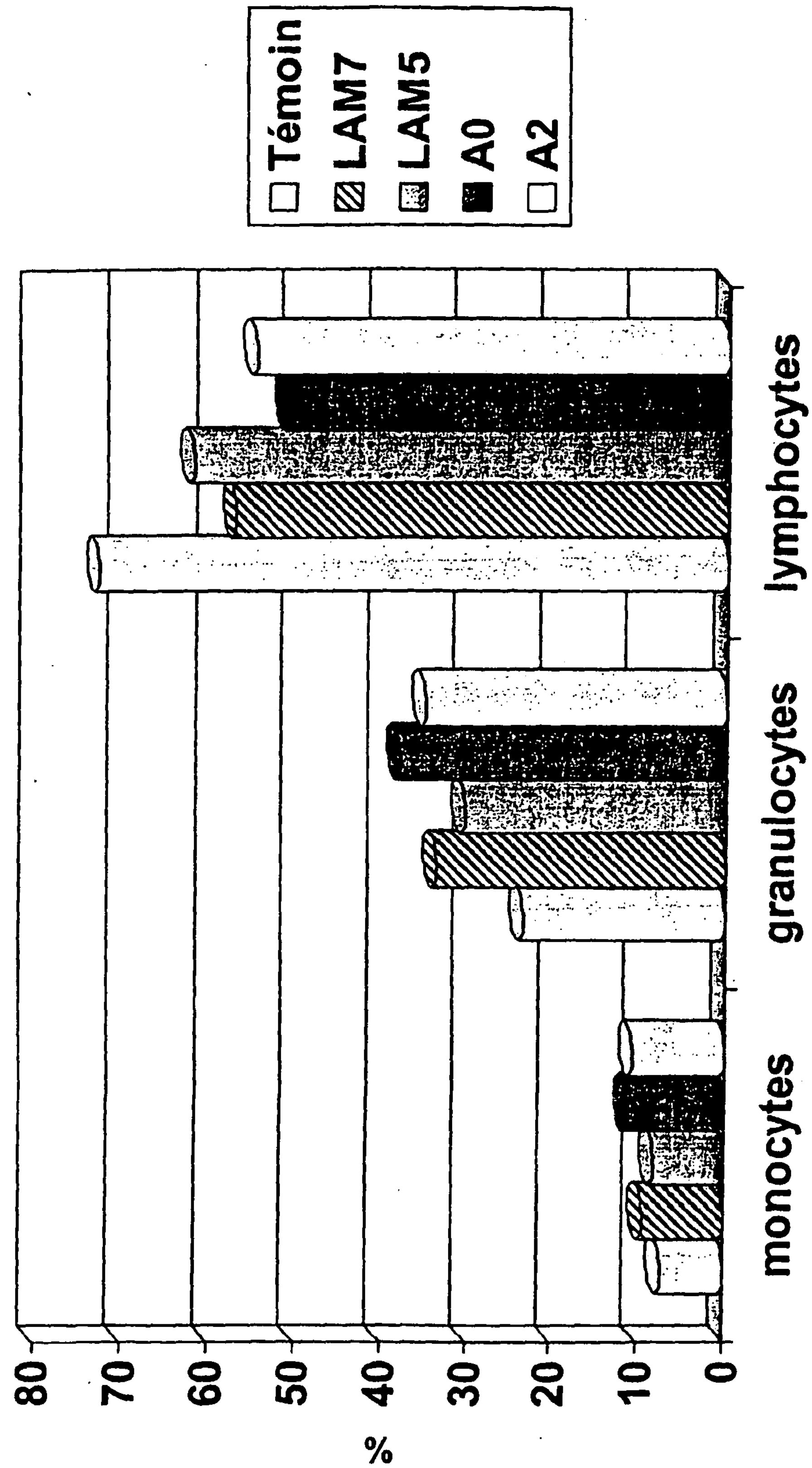
Témoin
LAM7
LAM5
A0
A2
%
80
70
60
50
40
30
20
10
0
monocytes
granulocytes
lymphocytes

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2005049044 A
- JP 59036691 B
- WO 03045414 A
- FR 2804684
- FR 2804694


### Littérature non-brevet citée dans la description


- **Jamois, F. ; Ferrières, V. ; Guégan, J.-P. ; Yvin, J.-C. ; Plusquellec, D. ; Vetvicka, V.** *Glycobiology,* 2005, vol. 15, 393-407
- **Blattner, R. ; Furneaux, R. H. ; Pakulski, Z.** *Carbohydr. Res.,* 2006, vol. 341, 2115-2125